# EUROPEAN PATENT APPLICATION

(11) **EP 4 670 733 A1**
(43) Date of publication of application: **31.12.2025**
(21) Application number: 24315311.1
(22) Date of filing: 27.06.2024
(51) Int. Cl.: A61K 39/12, A61K 39/145, A61P 31/16, C12N 7/00

(54) **METHODS FOR PREPARING VIRAL PROTEINS AND USES THEREOF**

(71) Applicant: Sanofi, 75017 Paris (FR)
(72) Inventor: Bernard, Isabelle, 75017 PARIS (FR); Calvosa, Eric, 75017 PARIS (FR); Charretier, Cedric, 75017 PARIS (FR); Marin, Vincent, 75017 PARIS (FR)
(74) Representative: Ipsilon

(57) **Abstract**

This application relates to methods for preparing viral proteins, such as influenza hemagglutinin proteins and/or influenza neuraminidase proteins, immunogenic compositions comprising the viral proteins thus obtained and vaccines comprising the same, as well as methods of using the same, in particular in the prevention and/or treatment of diseases or conditions caused by viruses, such as influenza viruses.

## Description

### FIELD

This application relates to methods for preparing viral proteins, such as influenza hemagglutinin proteins and/or influenza neuraminidase proteins, immunogenic compositions comprising the viral proteins thus obtained and vaccines comprising the same, as well as methods of using the same, in particular in the prevention and/or treatment of diseases or conditions caused by viruses, such as influenza viruses.

### BACKGROUND

There are at least 6 different types of viral vaccines currently in general use: live-attenuated vaccines, inactivated vaccines, subunit vaccines, toxoid vaccines, viral vector vaccines, and messenger RNA (mRNA) vaccines. Inactivated vaccines can be based on whole virus, "split" virus, or on purified surface antigens. During the manufacturing process of inactivated vaccines, detergents are commonly used reagents to inactivate viruses and/or to purify viral antigens. For instance, Triton X-100, a non-ionic detergent, is applied to influenza vaccines at different stages of the manufacturing process to prevent aggregation and precipitation of biomolecules. It is also used to disintegrate or disrupt the virus particles in split vaccines and to improve homogeneity during production and storage. Similarly, during the manufacturing process of recombinant vaccines, detergents, such as Triton X-100, can be used to solubilize and stabilize the recombinant viral protein.

A degradation product of Triton X-100 is octylphenol, an ecotoxin with demonstrated estrogenic effects that can function as an endocrine disruptor in water with the potential to negatively impact aquatic wildlife. Triton X-100 is currently banned by European regulation REACH (Registration, Evaluation, Authorization and Restriction of Chemicals) although exemptions are available.

Accordingly, there is an urgent need for sustainable alternatives to Triton X-100 for use in the preparation of viral proteins and the manufacturing of viral vaccines.

### SUMMARY

Disclosed herein are methods for preparing viral proteins, such as influenza hemagglutinin proteins and/or influenza neuraminidase proteins, as well as uses thereof. Accordingly, in one aspect, provided herein is a method for preparing at least one viral protein in a manufacturing process of a vaccine, the method comprising subjecting a suspension of virions or a suspension of host cells to a detergent comprising a compound having a CAS registry number of CAS 68131-40-8 or CAS 84133-50-6, wherein each virion comprises a lipid membrane that comprises the at least one viral protein, and wherein the host cells have been modified to express the at least one viral protein. In some embodiments, the detergent does not comprise a compound having a CAS registry number of CAS 9002-93-1 or CAS 9036-19-5. In some embodiments, the detergent consists of the compound having the CAS registry number of CAS 68131-40-8 or CAS 84133-50-6. In some embodiments, the detergent is added to the suspension of virions or the suspension of host cells in an amount of from about 0.01% to about 10% by volume, such as from about 0.1% to about 5% by volume or from about 0.5% to about 1% by volume. In some embodiments, the detergent is added to the suspension of virions or the suspension of host cells in an amount of about 0.5% by volume.

In some embodiments, subjecting the suspension of virions or the suspension of host cells to the detergent releases the at least one viral protein from the virions or host cells, and wherein the method further comprises obtaining a first suspension comprising the at least one viral protein released from the virions or host cells. In some embodiments, the method further comprises removing the detergent from the first suspension to obtain a second suspension comprising the at least one viral protein and a trace amount of the detergent. In some embodiments, removing the detergent comprises diafiltration. In some embodiments, the method further comprises purifying the at least one viral protein from the second suspension.

In some embodiments, the method further comprises formulating the at least one viral protein into a vaccine, wherein the vaccine contains no more than about 250 µg/0.5 mL of the compound having the CAS registry number of CAS 68131-40-8 or CAS 84133-50-6. In some embodiments, formulating comprises combining the at least one viral protein with an adjuvant to produce the vaccine. In some embodiments, the method further comprises filling the vaccine into a syringe or a vial.

In some embodiments, the vaccine prepared by the method of the present disclosure is an influenza vaccine. In some embodiments, the suspension of virions used in the method is obtained by cultivating an influenza virus in an embryonated chicken egg. In some embodiments, the suspension of virions is obtained by cultivating an influenza virus in a viral propagation cell line. In some embodiments, the viral propagation cell line is a Madin-Darby Canine Kidney (MDCK) cell line. In some embodiments, the suspension of host cells used in the method have been modified to recombinantly express an influenza hemagglutinin or neuraminidase polypeptide. In some embodiments, the suspension of host cells comprises SF9 cells of *Spodoptera frugiperda.* In some embodiments, the at least one viral protein is expressed in the SF9 cells using a baculovirus vector. In some embodiments, the suspension of host cells comprises Chinese hamster ovary (CHO) cells.

In some embodiments, the influenza vaccine prepared by the method of the present disclosure is a monovalent influenza vaccine. In some embodiments, the influenza vaccine is a multivalent influenza vaccine, such as a trivalent, quadrivalent, pentavalent, or octavalent influenza vaccine. In some embodiments, the multivalent influenza vaccine comprises at least one viral protein obtained from an influenza A virus and at least one viral protein obtained from an influenza B virus.

In some embodiments, the influenza vaccine prepared by the method of the present disclosure is a trivalent influenza vaccine comprising a first viral protein obtained from an H1N1 influenza virus strain, a second viral protein obtained from an H3N2 influenza virus strain, and a third viral protein obtained from an influenza virus of a B/Victoria lineage. In some embodiments, the method comprises combining and formulating the first, second, and third viral proteins into the trivalent influenza vaccine. In some embodiments, the first, second, and third viral proteins comprise influenza hemagglutinin proteins. In some embodiments, each of the first, second, and third viral proteins is an influenza hemagglutinin protein present in an amount between about 5 µg to about 60 µg per 0.5 mL dose of the vaccine.

In some embodiments, the influenza vaccine prepared by the method of the present disclosure is a quadrivalent influenza vaccine comprising a first viral protein obtained from an H1N1 influenza virus strain, a second viral protein obtained from an H3N2 influenza virus strain, a third viral protein obtained from an influenza virus of a B/Victoria lineage, and a fourth viral protein obtained from an influenza virus of a B/Yamagata lineage. In some embodiments, the method comprises combining and formulating the first, second, third, and fourth viral proteins into the quadrivalent influenza vaccine. In some embodiments, the first, second, third, and fourth viral proteins comprise influenza hemagglutinin proteins. In some embodiments, the amount of influenza hemagglutinin protein in each of the first, second, third, and fourth viral proteins is between about 5 µg to about 60 µg per 0.5 mL dose of the vaccine.

In some embodiments, the influenza vaccine prepared by the method of the present disclosure further comprises sodium phosphate buffered isotonic sodium chloride and optionally a preservative. In some embodiments, the preservative is mercury or a mercury derivative. In some embodiments, the mercury derivative is thimerosal.

In some embodiments, the influenza vaccine prepared by the method of the present disclosure further comprises formaldehyde at a concentration of no more than about 100 µg/0.5 mL.

Also provided herein is a vaccine, such as an influenza vaccine, manufactured by the method of the present disclosure.

In another aspect, provided herein is an immunogenic composition comprising an inactivated virus or at least one viral protein and a trace amount of a detergent comprising a compound having a CAS registry number of CAS 68131-40-8 or CAS 84133-50-6. In some embodiments, the detergent does not comprise a compound having a CAS registry number of CAS 9002-93-1 or CAS 9036-19-5. In some embodiments, the detergent consists of the compound having the CAS registry number of CAS 68131-40-8 or CAS 84133-50-6.

In some embodiments, the immunogenic composition of the present disclosure comprises an inactivated virus and the inactivated virus is an influenza A virus. In some embodiments, the influenza A virus is an H1N1 influenza virus strain. In some embodiments, the influenza A virus is an H3N2 influenza virus strain. In some embodiments, the inactivated virus is an influenza B virus. In some embodiments, the influenza B virus is a B/Victoria lineage. In some embodiments, the influenza B virus is a B/Yamagata lineage.

In some embodiments, the immunogenic composition of the present disclosure comprises at least one viral protein and the at least one viral protein is an influenza hemagglutinin protein and/or an influenza neuraminidase protein. In some embodiments, the at least one viral protein is a recombinant influenza structural protein. In some embodiments, the recombinant influenza structural protein is expressed in SF9 cells of *Spodoptera frugiperda.* In some embodiments, the recombinant influenza structural protein is expressed in the SF9 cells using a baculovirus vector. In some embodiments, the recombinant influenza structural protein is expressed in CHO cells. In some embodiments, the recombinant influenza structural protein is a recombinant influenza hemagglutinin protein and/or a recombinant influenza neuraminidase protein.

In some embodiments, the immunogenic composition of the present disclosure comprises at least one viral protein from an influenza A virus and at least one viral protein from an influenza B virus. In some embodiments, the immunogenic composition comprises a first viral protein from an H1N1 influenza virus strain, a second viral protein from an H3N2 influenza virus strain, and a third viral protein from an influenza virus of a B/Victoria lineage. In some embodiments, the first, second, and third viral proteins are influenza hemagglutinin proteins and/or influenza neuraminidase proteins. In some embodiments, each of the first, second, and third viral proteins is an influenza hemagglutinin protein present in an amount of between about 5 µg to about 60 µg per 0.5 mL dose of the immunogenic composition. In some embodiments, the immunogenic composition comprises a first viral protein from an H1N1 influenza virus strain, a second viral protein from an H3N2 influenza virus strain, a third viral protein from an influenza virus of a B/Victoria lineage, and a fourth viral protein from an influenza virus of a B/Yamagata lineage. In some embodiments, the first, second, third, and fourth viral proteins are influenza hemagglutinin proteins and/or influenza neuraminidase proteins. In some embodiments, each of the first, second, third, and fourth viral proteins is an influenza hemagglutinin protein present in an amount of between about 5 µg to about 60 µg per 0.5 mL dose of the immunogenic composition.

In some embodiments, the immunogenic composition of the present disclosure further comprises an adjuvant.

Further provided herein is a vaccine comprising the immunogenic composition of the present disclosure and a pharmaceutically acceptable carrier. In some embodiments, the vaccine comprises at least one influenza virus hemagglutinin protein. In some embodiments, the vaccine comprises no more than about 250 µg/0.5 mL of the compound having the CAS registry number of CAS 68131-40-8 or CAS 84133-50-6. In some embodiments, the vaccine further comprises sodium phosphate buffered isotonic sodium chloride and optionally a preservative. In some embodiments, the preservative is mercury or a mercury derivative. In some embodiments, the mercury derivative is thimerosal. In some embodiments, the vaccine further comprises formaldehyde at a concentration of no more than about 100 µg/0.5 mL. In some embodiments, the vaccine is a live attenuated virus vaccine, an inactivated whole virus vaccine, a virosome vaccine, a split-virion vaccine, a subunit vaccine, or a recombinant vaccine.

In a further aspect, provided herein are influenza vaccines. In some embodiments, the vaccine comprises about 5-60 µg/0.5 mL of an influenza hemagglutinin antigen from each of an H1N1 influenza virus strain, an H3N2 influenza virus strain, and an influenza virus of a B/Victoria lineage, a sodium phosphate-buffered isotonic sodium chloride solution, no more than about 30-100 µg/0.5 mL of formaldehyde, no more than about 250 µg/0.5 mL of a compound having a CAS registry number of CAS 68131-40-8 or CAS 84133-50-6, and optionally a preservative. In some embodiments, the vaccine does not comprise a compound having a CAS registry number of CAS 9002-93-1 or CAS 9036-19-5. In some embodiments, the preservative comprises thimerosal in an amount of about 2 µg/0.5 mL or mercury in an amount of about 20 µg/0.5 mL. In some embodiments, the vaccine comprises about 15 µg/0.5 mL of the influenza hemagglutinin antigen from each of an H1N1 influenza virus strain, an H3N2 influenza virus strain, and an influenza virus of a B/Victoria lineage, and no more than about 30 µg/0.5 mL of formaldehyde. In some embodiments, the vaccine further comprises thimerosal as the preservative in an amount of about 2 µg/0.5 mL. In some embodiments, the vaccine comprises about 15 µg/0.5 mL of an influenza hemagglutinin antigen from each of an H1N1 influenza virus strain, an H3N2 influenza virus strain, and an influenza virus of a B/Victoria lineage, and no more than about 100 µg/0.5 mL of formaldehyde, and wherein the preservative comprises mercury in an amount of about 25 µg/0.5 mL.

In some embodiments, the vaccine of the present disclosure comprises about 5-60 µg/0.5 mL of an influenza hemagglutinin antigen from each of an H1N1 influenza virus strain, an H3N2 influenza virus strain, an influenza virus of a B/Victoria lineage, and an influenza virus of a B/Yamagata lineage, a sodium phosphate-buffered isotonic sodium chloride solution, no more than about 100 µg/0.5 mL of formaldehyde, no more than about 250 µg/0.5 mL of a compound having a CAS registry number of CAS 68131-40-8 or CAS 84133-50-6, and optionally a preservative. In some embodiments, the vaccine does not comprise a compound having a CAS registry number of CAS 9002-93-1 or CAS 9036-19-5. In some embodiments, the vaccine comprises no more than about 30 µg/0.5 mL of formaldehyde. In some embodiments, the preservative is mercury in an amount of about 25 µg/0.5 mL.

In some embodiments, the vaccine of the present disclosure comprises about 5-60 µg/0.5 mL of an influenza hemagglutinin antigen from each of an H1N1 influenza virus strain, an H3N2 influenza virus strain, an influenza virus of a B/Victoria lineage, and an influenza virus of a B/Yamagata lineage, a buffer solution comprising sodium chloride, potassium chloride, dibasic sodium phosphate dihydrate, and monobasic potassium phosphate, no more than about 30 µg/0.5 mL of formaldehyde, and no more than about 250 µg/0.5 mL of a compound having a CAS registry number of CAS 68131-40-8 or CAS 84133-50-6. In some embodiments, the vaccine does not comprise a compound having a CAS registry number of CAS 9002-93-1 or CAS 9036-19-5. In some embodiments, the vaccine does not comprise an adjuvant or a preservative.

In some embodiments, the vaccine of the present disclosure comprises 45 µg/0.5 mL of a recombinant influenza hemagglutinin antigen from each of an H1N1 influenza virus strain, an H3N2 influenza virus strain, and an influenza virus of a B/Victoria lineage, sodium chloride, monobasic sodium phosphate, dibasic sodium phosphate, polysorbate 20, and no more than about 100 µg/0.5 mL of a compound having a CAS registry number of CAS 68131-40-8 or CAS 84133-50-6. In some embodiments, the vaccine does not comprise a compound having a CAS registry number of CAS 9002-93-1 or CAS 9036-19-5. In some embodiments, the vaccine comprises about 4.4 mg/0.5 mL sodium chloride, about 0.195 mg/0.5 mL monobasic sodium phosphate, about 1.3 mg/0.5 mL dibasic sodium phosphate, and about 27.5 µg/0.5 mL polysorbate 20. In some embodiments, the vaccine comprises about 4.4 mg/0.5 mL sodium chloride, about 0.2 mg/0.5 mL monobasic sodium phosphate, about 0.5 mg/0.5 mL dibasic sodium phosphate, and about 27.5 µg/0.5 mL polysorbate 20. In some embodiments, the vaccine does not comprise a preservative.

In some embodiments, the vaccine of the present disclosure comprises 45 µg/0.5 mL of a recombinant influenza hemagglutinin antigen from each of an H1N1 influenza virus strain, an H3N2 influenza virus strain, an influenza virus of a B/Victoria lineage, and an influenza virus of a B/Yamagata lineage, sodium chloride, monobasic sodium phosphate, dibasic sodium phosphate, polysorbate 20, and no more than about 100 µg/0.5 mL of a compound having a CAS registry number of CAS 68131-40-8 or CAS 84133-50-6. In some embodiments, the vaccine does not comprise a compound having a CAS registry number of CAS 9002-93-1 or CAS 9036-19-5. In some embodiments, the vaccine comprises about 4.4 mg/0.5 mL sodium chloride, about 0.195 mg/0.5 mL monobasic sodium phosphate, about 1.3 mg/0.5 mL dibasic sodium phosphate, and about 27.5 µg/0.5 mL polysorbate 20. In some embodiments, the vaccine comprises about 4.4 mg/0.5 mL sodium chloride, about 0.2 mg/0.5 mL monobasic sodium phosphate, about 0.5 mg/0.5 mL dibasic sodium phosphate, and about 27.5 µg/0.5 mL polysorbate 20. In some embodiments, the vaccine does not comprise a preservative.

In some embodiments, the serum hemagglutinin inhibition (HAI) titer induced by the vaccine of the present disclosure is the same or higher than the serum HAI titer induced by a control vaccine that is identical to the vaccine of the disclosure except that the trace amount of the detergent comprising the compound having the CAS registry number of CAS 68131-40-8 or CAS 84133-50-6 is replaced with a trace amount of a detergent comprising a compound having a CAS registry number of CAS 9002-93-1 or CAS 9036-19-5. In some embodiments, the vaccine induces a serum HAI titer of at least 40, wherein the HAI titer is measured at 20 days following vaccination. In some embodiments, the vaccine induces a serum HAI that increases at least 4-fold as compared to the serum HAI titer prior. to vaccination, wherein the HAI titer is measured at 20 days following vaccination.

In yet another aspect, provided herein is a method of immunizing a subject, the method comprising administering to the subject in need thereof the immunogenic composition or the vaccine disclosed herein. In some embodiments, the method prevents an influenza virus infection in the subject, decreases the subject's likelihood of getting an influenza virus infection, or reduces the subject's likelihood of getting serious illness from an influenza virus infection. In some embodiments, the method raises a protective immune response in the subject. In some embodiments, the subject is a human. In some embodiments, the human is 6 months of age or older, less than 18 years of age, at least 6 months of age and less than 18 years of age, at least 18 years of age and less than 65 years of age, at least 6 months of age and less than 5 years of age, at least 5 years of age and less than 65 years of age, at least 60 years of age, or at least 65 years of age. In some embodiments, the immunogenic composition or the vaccine is administered intramuscularly, intradermally, subcutaneously, intravenously, intranasally, by inhalation, or intraperitoneally.

Also provided herein is a method of reducing one or more symptoms of an influenza virus infection, the method comprising administering to a subject in need thereof the immunogenic composition or the vaccine of disclosed herein. In some embodiments, the method treats or prevents disease caused by either or both a seasonal and a pandemic influenza virus strain.

Further provided herein is a method of reducing one or more symptoms caused by an influenza virus infection, the method comprising administering to a subject in need thereof the immunogenic composition or the vaccine of disclosed herein.

### BRIEF DESCRIPTION OF THE DRAWING

The accompanying drawings, which are incorporated in and constitute a part of this specification, illustrate certain embodiments, and together with the written description, serve to explain certain principles of the methods and compositions disclosed herein.
**FIG. 1** depicts a bar chart showing the splitting efficiency of various detergents based on the single radial immunodiffusion (SRID) titer and the protein content.
**FIG. 2A-2F** show the particle size distribution by intensity (top) and by volume (bottom) as determined by dynamic light scattering (DLS) using different detergents as described in Example 1. **FIG. 2A****:** Ecosurf EH9; **FIG. 2B****:** Tergitol 15S9; **FIG. 2C****:** LDAO; **FIG. 2D****:** TDAO; **FIG. 2E****:** Triton X100; and **FIG. 2F****:** No detergent.
**FIG. 3** shows the protein profiles of the samples collected after the splitting procedure using different detergents analyzed by SDS-PAGE under denaturant/non-reducing conditions. Std: molecular weight markers.
**FIG. 4A** depicts the transmission electron microscopy (TEM) images showing the particles observed in the samples collected after the splitting procedure using different detergents, as well as the TEM images of the virus particles prior to the splitting procedure ("Purified Viral Culture") and the drug substance obtained after the downstream process ("Drug Substance"). **FIG. 4B** depicts a chart summarizing the morphological characteristics of the particles observed in the TEM images of **FIG. 4A****.**
**FIG. 5** depicts a bar chart showing the impact of the detergents on the infectious titer evaluated by cell culture infectious dose 50% (CCID₅₀) assay.
**FIG. 6** depicts the SRID statistical model and the estimate of the associated coefficients for Tergitol 15S9 using the JMP software.
**FIG. 7** depicts the conditions required to obtain an optimal SRID titer for Tergitol 15S9 using the Prediction Profiler of the JMP software.
**FIG. 8** shows the protein profiles of the samples collected after the splitting procedure using different splitting conditions described in Example 2 as analyzed by SDS-PAGE under denaturant/non-reducing conditions. See **Table 4** for a summary of the samples loaded onto the SDS-PAGE gel. Std: molecular weight markers.
**FIG. 9A** shows the protein profiles of the samples collected at different steps of the downstream process described in Example 3 as analyzed by SDS-PAGE under denaturant/nonreducing conditions. Std: molecular weight markers; E5a: Sample E5 collected before addition of formaldehyde; E5i: Sample E5 collected after formaldehyde inactivation. **FIG. 9B** shows the protein profiles of the drug substance obtained from the reference batch using Triton X-100 as the splitting agent.
**FIG. 10** depicts the TEM images showing the particles observed in the samples collected after the splitting and centrifugation ("Step 2") and the samples collected from the drug substance ("Step 7") using Triton X-100 or Tergitol 15S9 as the splitting agent.
**FIG. 11A-11B** show the particle size distribution by intensity (left) and by volume (right) as determined by DLS in the samples collected from the drug substance ("Step 7") using Triton X-100 or Tergitol 15S9 as the splitting agent. **FIG. 11A****:** Tergitol 15S9; **FIG. 11B****:** Triton X-100.
**FIG. 12** shows representative serum HAI titer in mice at day 20 (prime) and day 35 (prime/boost) post-immunization with a monovalent influenza vaccine containing recombinant hemagglutinin (HA) protein (A/Michigan/45/2015 virus strain) expressed in insect cells as described in Example 4.
**FIG. 13** shows representative serum HAI titer in mice at day 20 (prime) and day 35 (prime/boost) post-immunization with a monovalent influenza split vaccine containing HA protein (A/Michigan/45/2015 virus strain) produced in embryonated eggs as described in Examples 3 and 5.

### DETAILED DESCRIPTION

Reference will now be made in detail to various exemplary embodiments, examples of which are illustrated in the accompanying drawings and discussed in the detailed description that follows. It is to be understood that the following detailed description is provided to give the reader a fuller understanding of certain embodiments, features, and details of aspects of the disclosure, and should not be interpreted as limiting the scope of the disclosure.

In order for the present disclosure to be more readily understood, certain terms are first defined below. Additional definitions for the following terms and other terms may be set forth through the specification. If a definition of a term set forth below is inconsistent with a definition in an application or patent that is incorporated by reference, the definition set forth in this application should be used to understand the meaning of the term.

### Definitions

As used in this specification and the appended claims, the singular forms "a," "an," and "the" include plural references unless the context clearly dictates otherwise. Thus, for example, a reference to "a method" includes one or more methods, and/or steps of the type described herein and/or which will become apparent to those persons skilled in the art upon reading this disclosure and so forth.

The term "about," or "approximately," is used herein to mean within the typical ranges of tolerances in the art. For example, "about" can be understood as about 2 standard deviations from the mean. According to certain embodiments, when referring to a measurable value such as an amount and the like, "about" is meant to encompass variations of ±20%, ±10%, ±5%, ±1%, ±0.9%, ±0.8%, ±0.7%, ±0.6%, ±0.5%, ±0.4%, ±0.3%, ±0.2% or ±0.1% from the specified value as such variations are appropriate to perform the disclosed methods and/or to make and use the disclosed compositions. When "about" is present before a series of numbers or a range, it is understood that "about" can modify each of the numbers in the series or range.

The term "and/or," as used herein in the specification and in the claims, should be understood to mean "either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified unless clearly indicated to the contrary. Thus, as a non-limiting example, a reference to "A and/or B," when used in conjunction with open-ended language such as "comprising" can refer, in one embodiment, to A without B (optionally including elements other than B); in another embodiment, to B without A (optionally including elements other than A); in yet another embodiment, to both A and B (optionally including other elements); etc.

As used herein, the term "antigen" refers to an agent that elicits an immune response; and/or (ii) an agent that is bound by a T cell receptor (e.g., when presented by an MHC molecule) or to an antibody (e.g., produced by a B cell) when exposed or administered to an organism. In some embodiments, an antigen elicits a humoral response (e.g., including production of antigen-specific antibodies) in an organism; alternatively or additionally, in some embodiments, an antigen elicits a cellular response (e.g., involving T-cells whose receptors specifically interact with the antigen) in an organism. It will be appreciated by those skilled in the art that a particular antigen may elicit an immune response in one or several members of a target organism (e.g., mice, ferrets, rabbits, primates, humans), but not in all members of the target organism species. In some embodiments, an antigen elicits an immune response in at least about 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99%, including all values and subranges therebetween, of the members of a target organism species. In some embodiments, an antigen binds to an antibody and/or T cell receptor and may or may not induce a particular physiological response in an organism. In some embodiments, for example, an antigen may bind to an antibody and/or to a T cell receptor *in vitro,* whether or not such an interaction occurs *in vivo.* In some embodiments, an antigen reacts with the products of specific humoral or cellular immunity, including those induced by heterologous immunogens.

The term "diafiltration," as used herein, means a combination of "dilution" and "filtration" and generally refers to a dilution process that involves removal or separation of components (e.g., permeable molecules like salts, small proteins, solvents, etc.) of a solution based on their molecular size by using micro-molecule permeable filters in order to obtain a pure or substantially pure solution.

The term "at least," "less than," "more than," or "up to" prior to a number or series of numbers (e.g., "at least two") is understood to include the number adjacent to the term "at least," "less than" or "more than," and all subsequent numbers or integers that could logically be included, as clear from context. When the term "at least," "less than," "more than," or "up to" is present before a series of numbers or a range, it is understood that "at least," "less than," "more than," or "up to" can modify each of the numbers in the series or range.

The term "detergent" refers to an agent that may comprise salts of long-chain aliphatic bases or acids, or hydrophilic moieties, such as sugars, and that possess both hydrophilic and hydrophobic properties. Having both hydrophilic and hydrophobic properties, the detergent can exert particular effects. As used herein, detergents have the ability to disrupt viral envelopes and/or inactivate viruses in some embodiments. Detergents can also have the ability to disrupt cell membranes in some embodiments. In some examples, a detergent may be a composition comprising a surfactant and one or more other agents such as chelating agents and preservatives.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

As used herein, the term "host cell" refers to a cell into which exogenous DNA (recombinant or otherwise) has been introduced. For example, host cells may be used to produce influenza hemagglutinin (HA) and neuraminidase (NA) polypeptides by standard recombinant techniques. Persons of skill upon reading this disclosure will understand that such terms refer not only to the particular subject cell, but, to the progeny of such a cell. Because certain modifications may occur in succeeding generations due to either mutation or environmental influences, such progeny may not, in fact, be identical to the parent cell, but are still included within the scope of the term "host cell" as used herein.

Certain cells may also be used to propagate virions in the manufacturing process of a viral vaccine. In this context, such cells are also referred to herein as a "viral propagation cell" or a "viral propagation cell line."

As used herein, the term "in some embodiments," "in certain embodiments," "in other embodiments," "in some other embodiments," or the like, refers to embodiments of all aspects of the disclosure, unless the context clearly indicates otherwise.

The term "prevent," "preventing," or "prevention," as used herein, refers to prophylaxis, avoidance of disease manifestation, a delay of onset, and/or reduction in frequency and/or severity of one or more symptoms of a particular disease, disorder or condition (e.g., infection with, for example, a virus, such as influenza virus). In some embodiments, prevention is assessed on a population basis such that an agent is considered to "prevent" a particular disease, disorder or condition if a statistically significant decrease in the development, frequency, and/or intensity of one or more symptoms of the disease, disorder or condition is observed in a population susceptible to the disease, disorder, or condition.

As used herein, the term "prophylactically effective amount" means an amount sufficient to avoid disease manifestation, delay onset of and/or reduce in frequency and/or severity one or more symptoms of a particular disease, disorder or condition (e.g., infection with, for example, a virus, such as influenza virus).

As used herein, the term "recombinant" refers to polypeptides (e.g., influenza HA and/or NA polypeptides) that are designed, engineered, prepared, expressed, created or isolated by recombinant means, such as polypeptides expressed using a recombinant expression vector transfected into a host cell, polypeptides isolated from a recombinant, combinatorial polypeptide library or polypeptides prepared, expressed, created or isolated by any other means that involves splicing selected sequence elements to one another. In some embodiments, one or more of such selected sequence elements is found in nature. In some embodiments, one or more of such selected sequence elements and/or combinations thereof is designed *in silico.* In some embodiments, one or more such selected sequence elements results from the combination of multiple (e.g., two or more) known sequence elements that are not naturally present in the same polypeptide (e.g., two epitopes from two separate HA polypeptides).

Each year, based on intensive surveillance efforts, the World Health Organization (WHO) selects influenza strains to be included in the seasonal vaccine preparations. As used herein, the term "standard of care strain" or "SOC strain" refers to an influenza strain that is selected by the World Health Organization (WHO) to be included in the seasonal vaccine preparations. A standard of care strain can include a historical standard of care strain, a current standard of care strain or a future standard of care strain.

As used herein, the term "subject" means any member of the animal kingdom. In some embodiments, "subject" refers to humans. In some embodiments, "subject" refers to non-human animals. In some embodiments, subjects include, but are not limited to, mammals, birds, reptiles, amphibians, fish, insects, and/or worms. In some embodiments, the non-human subject is a mammal (e.g., a rodent, a mouse, a rat, a rabbit, a ferret, a monkey, a dog, a cat, a sheep, cattle, a primate, and/or a pig). In some embodiments, a subject may be a transgenic animal, genetically-engineered animal, and/or a clone. In some embodiments, the subject is an adult, an adolescent or an infant. In some embodiments, the term "individual" or "patient" is used and is intended to be interchangeable with the term "subject."

A "surfactant" or "surface active agent" is a compound, typically (but not necessarily) an organic compound, that contains both hydrophobic and hydrophilic groups, and is thus semi-soluble in both organic and aqueous solvents. Surfactants can be non-ionic, cationic or anionic.

As used herein, the term "trace amount" refers to an extremely small amount that is barely detectable. In some embodiments, the "trace amount" is a detectable amount but the amount detected is extremely small and insignificant. In some embodiments, the "trace amount" is an amount that cannot be detected using any analytic methods known in the art.

The term "virion," as used herein, refers to an extracellular, virus particle comprising a capsid or a lipid membrane that comprises one or more viral proteins and surrounds the viral genome (e.g., DNA or RNA genome).

### Methods for Preparing Viral Proteins

The present disclosure provides methods for preparing viral proteins using detergents other than Triton X-100. Triton X-100, having a CAS registry number of, for example, CAS 9002-93-1 or CAS 9036-19-5, is a detergent that is commonly used, for example, in the manufacturing process or formulation of vaccines and pharmaceutical compositions. However, the use of Triton X-100 is regulated in certain jurisdictions, including Europe, because its degradation product, octylphenol, may negatively impact aquatic wildlife. In searching for alternative detergents to replace Triton X-100, the inventors surprisingly found that detergents of the secondary alcohol ethoxylate type, particularly C₁₁₋₁₅- and C₁₂₋₁₄-secondary alcohol ethoxylates, provide comparable results as Triton X-100. Secondary alcohol ethoxylates are readily biodegradable and low in aquatic toxicity, which makes them good candidates to replace Triton X-100.

Accordingly, one aspect is directed to the use of a secondary alcohol ethoxylate for disrupting virus particles and releasing viral proteins in the manufacture of a split vaccine. In certain embodiments, provided herein is a method for preparing at least one viral protein in a manufacturing process of a vaccine, the method comprising subjecting a suspension of virions, where each virion comprises a lipid membrane that comprises the at least one viral protein, to a secondary alcohol ethoxylate as the detergent in lieu of Triton X-100. The method of the present disclosure can be used to prepare at least one viral protein from any virus, including, but not limited to, influenza virus, respiratory syncytial virus, coronavirus, rotavirus, papillomavirus, flavivirus, and paramyxovirus. In some embodiments, the method is used to prepare at least one viral protein from influenza virus. In some embodiments, the method is used to prepare at least one viral protein from respiratory syncytial virus. In some embodiments, the method is used to prepare at least one viral protein from coronavirus. In some embodiments, the method is used to prepare at least one viral protein from rotavirus. In some embodiments, the method is used to prepare at least one viral protein from papillomavirus. In some embodiments, the method is used to prepare at least one viral protein from flavivirus. In some embodiments, the method is used to prepare at least one viral protein from paramyxovirus.

Another aspect is directed to the use of a secondary alcohol ethoxylate in the manufacture of a recombinant protein vaccine. The methods of the present disclosure can be used to prepare at least one viral protein from host cells that have been modified to express the at least one viral protein. In some embodiments, provided herein is a method for preparing at least one viral protein in a manufacturing process of a vaccine, the method comprising subjecting a suspension of host cells, where the host cells have been modified to express the at least one viral protein, to a secondary alcohol ethoxylate as the detergent in lieu of Triton X-100. The method of the present disclosure can be used to prepare recombinantly expressed viral proteins from any virus, including, but not limited to, influenza virus, respiratory syncytial virus, coronavirus, rotavirus, papillomavirus, flavivirus, and paramyxovirus. For example, in some embodiments, the host cells are modified to express a recombinant influenza hemagglutinin protein. In some embodiments, the host cells are modified to express a recombinant influenza neuraminidase protein. In some embodiments, the host cells are modified to express a viral protein from respiratory syncytial virus. In some embodiments, the host cells are modified to express a viral protein from coronavirus. In some embodiments, the host cells are modified to express a viral protein from rotavirus. In some embodiments, the host cells are modified to express a viral protein from papillomavirus. In some embodiments, the host cells are modified to express a viral protein from flavivirus. In some embodiments, the host cells are modified to express a viral protein from paramyxovirus.

Secondary alcohol ethoxylates useful in the methods of the present disclosure generally have the following chemical formula:

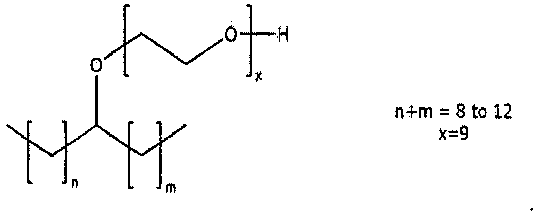

Among these secondary alcohol ethoxylates, C₁₁₋₁₅-secondary alcohol ethoxylates having a CAS registry number of CAS 68131-40-8 or C₁₂₋₁₄-secondary alcohol ethoxylates having a CAS registry number of CAS 84133-50-6 are particularly useful. Depending on the manufacturers, these secondary alcohol ethoxylates are known by different trade names. For example, those manufactured by Dow Chemical Company are known as TERGITOL^{™} 15-S-9, while those manufactured by MilliporeSigma (a Merck group) are known as DEVIRON^{™} 13-S9 (GMP grade). Either TERGITOL^{™} 15-S-9 or DEVIRON^{™} 13-S9 is suitable for the methods disclosed herein.

Accordingly, in some embodiments, provided herein is a method for preparing at least one viral protein in a manufacturing process of a vaccine, the method comprising subjecting a suspension of virions or a suspension of host cells to a detergent comprising a compound having a CAS registry number of CAS 68131-40-8 or CAS 84133-50-6 (e.g., TERGITOL^{™} 15-S-9 or DEVIRON^{™} 13-S9), wherein each virion comprises a lipid membrane that comprises the at least one viral protein, and wherein the host cells have been modified to express the at least one viral protein. In some embodiments, the detergent used in the method of the present disclosure comprises a compound having a CAS registry number of CAS 68131-40-8. In some embodiments, the detergent used in the method of the present disclosure comprises a compound having a CAS registry number of CAS 84133-50-6. In some embodiments, the detergent used in the method of the present disclosure comprises TERGITOL^{™} 15-S-9. In some embodiments, the detergent used in the method of the present disclosure comprises DEVIRON^{™} 13-S9. In some embodiments, the detergent used in the method of the present disclosure does not comprise a compound having a CAS registry number of CAS 9002-93-1 or CAS 9036-19-5. The compound having a CAS registry number of CAS 68131-40-8 or CAS 84133-50-6 (e.g., TERGITOL^{™} 15-S-9 or DEVIRON^{™} 13-S9) can be the only detergent used in the method of the present disclosure. Accordingly, in some embodiments, the detergent used in the method disclosed herein consists of a compound having a CAS registry number of CAS 68131-40-8. In some embodiments, the detergent used in the method disclosed herein consists of a compound having a CAS registry number of CAS 84133-50-6. In some embodiments, the detergent used in the method disclosed herein consists of TERGITOL^{™} 15-S-9. In some embodiments, the detergent used in the method disclosed herein consists of DEVIRON^{™} 13-S9.

The amount of the detergent added to the suspension of virions or the suspension of host cells should be sufficient to disrupt the lipid membrane of the virions or the cell membrane of the host cells. Disrupting the lipid membrane of the virions or the cell membrane of the host cells promotes the release of viral proteins from the lipid membrane of the virions or the cell membrane of the host cells. In some embodiments, the detergent is added to the suspension of virions or the suspension of host cells in an amount of from about 0.1% to about 10% by volume, such as from about 0.25% to about 10% by volume, from about 0.3% to about 8% by volume, from about 0.3% to about 5% by volume, or from about 0.4% to about 1% by volume, including all values and subranges therebetween. In some embodiments, the detergent is added to the suspension of virions or the suspension of host cells in an amount of no more than about 10% by volume, such as about 0.2%, 0.3%, 0.4%, 0.5%, 0.6%, 0.7%, 0.8%, 0.9%, 1%, 2%, 3%, 4%, 5%, 6%, 7%, 8%, 9%, or 10% by volume, including all values and subranges therebetween. In some embodiments, the detergent is added to the suspension of virions or the suspension of host cells in an amount of about 0.1% to 1% by volume. In some embodiments, the detergent is added to the suspension of virions or the suspension of host cells in an amount of about 0.4% to 1% by volume. In some embodiments, the detergent is added to the suspension of virions or the suspension of host cells in an amount of about 0.5% by volume. In some embodiments, the detergent is added to the suspension of virions or the suspension of host cells in an amount of about 1% by volume.

In some embodiments, subjecting the suspension of virions or the suspension of host cells to the detergent disrupts the lipid membrane of the virions or the cell membrane of the host cells and releases the at least one viral protein from the virions or host cells. Thus, in some embodiments, the methods of the present disclosure comprise obtaining a suspension comprising the at least one viral protein released from the virions or host cells. In some embodiments, once the detergent is added to the suspension of virions or the suspension of host cells, the mixture is incubated at a temperature for a period of time sufficient to allow the detergent to disrupt the lipid membrane of the virions or the cell membranes of the host cells, resulting in the release of the at least one viral protein comprised therein. In some embodiments, the mixture is incubated at about 2°C to about 30°C, such as about 2°C to about 8°C, about 8°C to about 28°C, about 10°C to about 26°C, about 15°C to about 24°C, or about 18°C to about 22°C, including all values and subranges therebetween. In some embodiments, the mixture is incubated at a temperature no more than about 30°C, such as 4°C, 5°C, 10°C, 15°C, 20°C, 22°C, 25°C, or 30°C, including all values and subranges therebetween. In some embodiments, the mixture is incubated at a temperature of about 20°C. In some embodiments, the mixture is incubated at a temperature of about 22°C. In some embodiments, the mixture is incubated at a temperature of about 25°C.

The incubation time to allow the detergent to disrupt the lipid membrane of the virions or the cell membranes of host cells can be from about 1 minute to about 48 hours in some embodiments, such as from about 10 minutes to about 24 hours, from about 15 minutes to about 5 hours, from about 30 minutes to about 3 hours, from about 45 minutes to about 2 hours, or from about 30 minutes to about 90 minutes, including all values and subranges therebetween. In some embodiments, the incubation time to allow the detergent to disrupt the lipid membrane of the virions or the cell membranes of host cells is about 15 minutes. In some embodiments, the incubation time is about 30 minutes. In some embodiments, the incubation time is about 45 minutes. In some embodiments, the incubation time is about 60 minutes. In some embodiments, the incubation time is about 90 minutes. In some embodiments, the incubation time is about 2 hours. More than about 2 hours of incubation time can also be used in some embodiments but generally the incubation time does not exceed about 48 hours. In some embodiments, the incubation step includes agitating the suspension, for example, using a stir bar or other agitation instrument.

In some embodiments, during the incubation, the mixture is stirred using, for instance, a magnetic bar at a speed from about 50 rpm to about 500 rpm, such as from about 75 rpm to about 275 rpm, from about 100 rpm to about 250 rpm, from about 150 rpm to about 225 rpm, from about 300 rpm to about 475 rpm, from about 350 rpm to about 450 rpm, or from about 400 rpm to about 440 rpm, including all values and subranges therebetween. In some embodiments, the mixture is stirred at a speed of about 150 rpm during the incubation. In some embodiments, the mixture is stirred at a speed of about 175 rpm during the incubation. In some embodiments, the mixture is stirred at a speed of about 200 rpm during the incubation. In some embodiments, the mixture is stirred at a speed of about 225 rpm during the incubation. In some embodiments, the mixture is stirred at a speed of about 275 rpm during the incubation. In some embodiments, the mixture is stirred at a speed of about 300 rpm during the incubation. In some embodiments, the mixture is stirred at a speed of about 350 rpm during the incubation. In some embodiments, the mixture is stirred at a speed of about 400 rpm during the incubation. In some embodiments, the mixture is stirred at a speed of about 405 rpm during the incubation. In some embodiments, the mixture is stirred at a speed of about 410 rpm during the incubation. In some embodiments, the mixture is stirred at a speed of about 415 rpm during the incubation. In some embodiments, the mixture is stirred at a speed of about 420 rpm during the incubation. In some embodiments, the mixture is stirred at a speed of about 425 rpm during the incubation. In some embodiments, the mixture is stirred at a speed of about 430 rpm during the incubation. In some embodiments, the mixture is stirred at a speed of about 435 rpm during the incubation. In some embodiments, the mixture is stirred at a speed of about 440 rpm during the incubation.

In some embodiments, the methods of the present disclosure comprise a step of centrifugation, such as ultracentrifugation. Any centrifugation or ultracentrifugation conditions sufficient to fulfill the purpose of this step can be used. For example, in some embodiments, a centrifugation at about 1,200-1,500 rpm for about 10-30 minutes can be used. In other embodiments, an ultracentrifugation at about 30,000-32,000 rpm for about 10-30 minutes can be used. Variations of these exemplified centrifugation or ultracentrifugation condition can be used according to the knowledge of the skilled person.

In some embodiments, the methods of the present disclosure comprise a step of removing the detergent from the suspension comprising the at least one viral protein released from the virions or host cells. The detergent can be removed by any appropriate methods known to one skilled in the art, such as gel filtration, size exclusion, dialysis, diafiltration, and ion-exchange chromatography. In some embodiments, the detergent is removed by gel filtration. In some embodiments, the detergent is removed by size exclusion. In some embodiments, the detergent is removed by dialysis. In some embodiments, the detergent is removed by diafiltration. In some embodiments, the detergent is removed by ion-exchange chromatography.

It may not be possible to completely remove the detergent from a solution. Thus, in some embodiments, the methods of the present disclosure further comprise obtaining a suspension comprising the at least one viral protein and a trace amount of the detergent. In some embodiments, the trace amount is an extremely small amount that is barely detectable. In some embodiments, the trace amount is a detectable amount but the amount detected is extremely small and insignificant. In some embodiments, the trace amount of the detergent remains in the suspension is not more than about 5%, such as 4.5%, 4%, 3.5%, 3%, 2.5%, 2%, 1.5%, 1%, 0.9%, 0.8%, 0.7%, 0.6%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, or 0.001%, by volume, including all values and subranges therebetween. In some embodiments, the trace amount is an amount that cannot be detected using any analytical methods known in the art.

In some embodiments, the methods of the present disclosure comprise a step of diafiltration. Diafiltration utilizes permeable membrane filters to separate the components of solutions and suspensions based on their molecular size. Small components can pass through the membrane to form a filtrate, whereas larger components (e.g., virions) cannot, and so are retained. Any suitable membrane (e.g., PES, celluloses, etc.) in any suitable form (e.g., hollow fire, cassette, spirals, etc.) can be used. Diafiltration can be used to reduce the concentration of salts, solvents, or other low molecular weight species in a liquid medium, and optionally to introduce new species (e.g., to exchange buffers). Diafiltration can be either continuous or discontinuous. Continuous diafiltration involves washing out original low molecular weight species by adding water or buffer to the sample at substantially the same rate as filtrate is being generated. If a buffer is used for diafiltration then the concentration of the new buffer salt in the sample increases at a rate inversely proportional to that of the species being removed. Using continuous diafiltration, greater than 99.5% of a 100% permeable solute can typically be removed by washing through six equivalent sample volumes with the buffer of choice. Discontinuous diafiltration dilutes a sample with a volume of water or buffer and then concentrates back to the original volume. The diafiltration can be performed with a tangential flow filtration (TFF) system. In some embodiments, the diafiltration is continuous diafiltration. In some embodiments, the diafiltration is discontinuous diafiltration. In some embodiments, the diafiltration is performed with a TFF system (i.e., TFF-diafiltration).

In some embodiments, the methods of the present disclosure comprise purifying the at least one viral protein from the suspension. Any methods known in the art for purifying proteins from solutions, such as filtration (e.g., diafiltration and ultrafiltration) and chromatography (e.g., size exclusion chromatography, ion exchange column chromatography, and affinity chromatography) can be used. In some embodiments, the at least one viral protein is purified from the suspension by diafiltration. In some embodiments, the at least one viral protein is purified from the suspension by ultrafiltration. In some embodiments, the at least one viral protein is purified from the suspension by size exclusion chromatography. In some embodiments, the at least one viral protein is purified from the suspension by ion exchange column chromatography. In some embodiments, the at least one viral protein is purified from the suspension by affinity chromatography.

In some embodiments, the methods of the present disclosure comprise inactivating any virus that may be present in the suspension, such as residual virions from the starting materials. Any methods known in the art for inactivating viruses can be used. For example, chemical means, such as treatment with an effective amount of one or more of detergents, formaldehyde (e.g., as formalin), β-propiolactone, methylene blue, psoralen, carboxyfullerene (C60), or a combination of any thereof, can be used to inactivate viruses. Other methods of viral inactivation, such as, for example, binary ethylamine, acetyl ethyleneimine, or gamma irradiation, or UV light, can also be used. In some embodiments, the method of the present disclosure comprises inactivating viruses with, for example, 5% (v/v) formaldehyde.

In some embodiments, the method of the present disclosure further comprises formulating the at least one viral protein thus prepared into a vaccine. In some embodiments, such a formulating step comprises combining the at least one viral protein with an adjuvant to produce the vaccine. Any adjuvant known in the art, such as those described herein elsewhere, can be used. In some embodiments, the vaccine thus formulated comprises no more than about 500 µg/0.5 mL, such as about 5 µg/0.5 mL, about 25 µg/0.5 mL, about 50 µg/0.5 mL, about 75 µg/0.5mL, about 100 µg/0.5 mL, about 125 µg/0.5 mL, about 150 µg/0.5 mL, about 175 µg/0.5 mL, about 200 µg/0.5 mL, about 225 µg/0.5 mL, about 250 µg/0.5 mL, about 275 µg/0.5 mL, about 300 µg/0.5 mL, about 325 µg/0.5 mL, about 350 µg/0.5 mL, about 375 µg/0.5 mL, about 400 µg/0.5 mL, about 425 µg/0.5 mL, about 450 µg/0.5 mL, about 475 µg/0.5 mL, or about 500 µg/0.5 mL, including all values and subranges therebetween, of the compound having the CAS registry number of CAS 68131-40-8. In some embodiments, the vaccine thus formulated comprises no more than about 250 µg/0.5 mL, such as about 5 µg/0.5 mL, about 25 µg/0.5 mL, about 50 µg/0.5 mL, about 75 µg/0.5mL, about 100 µg/0.5 mL, about 125 µg/0.5 mL, about 150 µg/0.5 mL, about 175 µg/0.5 mL, about 200 µg/0.5 mL, about 225 µg/0.5 mL, about 250 µg/0.5 mL, about 275 µg/0.5 mL, about 300 µg/0.5 mL, about 325 µg/0.5 mL, about 350 µg/0.5 mL, about 375 µg/0.5 mL, about 400 µg/0.5 mL, about 425 µg/0.5 mL, about 450 µg/0.5 mL, about 475 µg/0.5 mL, or about 500 µg/0.5 mL, including all values and subranges therebetween, of the compound having the CAS registry number of CAS 84133-50-6.

The vaccine prepared according to the method of the disclosure can be formulated for administration by any suitable route, such as those described herein elsewhere. To facilitate administration and/or storage, the vaccine prepared according to the method of the disclosure can be filled in a syringe or a vial. Accordingly, in some embodiments, the method of the present disclosure further comprises filling the vaccine into a syringe. In some embodiments, the method of the present disclosure further comprises filling the vaccine into a vial.

In some embodiments, the vaccine prepared according to the methods of the present disclosure is an influenza vaccine. Traditionally, influenza vaccine is produced by propagating influenza virions in embryonated chicken eggs. Influenza virions can also be propagated in cultured viral propagation cells. *See e.g.,* Jae-Mon Song, Journal of Microbiology (2016) 54(6):403-412). Such viral propagation cells are typically of mammalian origin. Suitable cells of mammalian origin include, but are not limited to, hamster, cattle, primate (including humans and monkeys) and dog cells. In some embodiments, the viral propagation cells are avian cells, including, but not limited to, duck cells or chick embryo fibroblasts. Various cell types may be used to propagate virions, including but not limited to, kidney cells, fibroblasts, retinal cells, and lung cells. Examples of suitable hamster cells are the cell lines having the names BHK21 (baby hamster kidney fibroblast) or HKCC (Human kidney cancer stem cells). Suitable monkey cells include but are not limited to, African green monkey cells, such as kidney cells, including, for example, the Vero cell line. Suitable dog cells include, but are not limited to, kidney cells, including, for example, the cutter laboratory dog kidney (CLDK) and Madin-Darby canine kidney (MDCK) cell lines. MDCK cells are commonly used for the propagation of human influenza viruses. Suitable viral propagation cell lines include, but are not limited to, MDCK, CHO, CLDK, HKCC, 293T, BHK, Vero, MRC-5, PER.C6, FRhL2, and WT-38 and their derivatives. Suitable cell lines are widely available from, for instance, the American Type Cell Culture (ATCC) collection, from the Coriell Cell Repositories, or from the European Collection of Cell Cultures (ECACC). For example, the ATCC supplies various different Vero cells under catalog numbers CCL-81. CCL-81.2, CRL-1586 and CRL-1587, and it supplies MDCK cells under catalog number CCL-34. PER.C6 is available from the ECACC under deposit number 96022940. Any of these cell types can be used for growth, reassortment and/or passaging according to the present disclosure. In some embodiments, the viral propagation cells are MDCK cells.

Accordingly, in some embodiments, the suspension of virions used in the method of the present disclosure is obtained by cultivating an influenza virus in an embryonated chicken egg. In some embodiments, the suspension of virions used in the method of the present disclosure is obtained by cultivating and propagating an influenza virus in a viral propagation cell line, such as a Vero cell line or a MDCK cell line. In some embodiments, the suspension of virions used in the method of the present disclosure is obtained by cultivating and propagating an influenza virus in a MDCK cell line.

Methods for propagating influenza virus in viral propagation cells (e.g. for growing influenza virus in cultured MDCK cells) generally include inoculating a culture of the viral propagation cells with an inoculum of the viral strain to be grown, cultivating the infected cells for a desired time period for virus propagation and collecting the propagated virus. The cultivation time can be determined using, for example, virus titer or antigen expression (e.g. between 24 and 168 hours after inoculation).

The influenza virus vaccine can also be produced using recombinant means. For instance, influenza hemagglutinin (HA) and/or neuraminidase (NA) polypeptides, or antigenic fragments thereof, can be produced in a host cell by standard recombinant techniques. Such host cells include any prokaryotic and eukaryotic cells suitable for expressing an exogenous DNA (e.g., a recombinant nucleic acid sequence). Exemplary cells include those of prokaryotes and eukaryotes (single-cell or multiple-cell), bacterial cells (e.g., strains of *E. coli, Bacillus* spp., *Streptomyces* spp., etc.), mycobacteria cells, fungal cells, yeast cells (e.g., *S*. *cerevisiae*, *S. pombe*, *P. pastoris*, *P. methanolica*, etc.), plant cells, microalgae (including both eukaryotic algae, such as *Chlamydomonas*, *Chlorella*, *Nannochloropsis*, Thraustochytriales (e.g., *Schizochytrium* sp.), diatoms (e.g., *Phaeodactylum*), and prokaryotic cyanobacteria, also known as blue-green algae such as *Arthrospira*), insect cells (e.g., SF-9, SF-21, baculovirus-infected insect cells, *Trichoplusia ni,* etc.), non-human animal cells, human cells, or cell fusions such as, for example, hybridomas or quadromas. In some embodiments, the cell is a human, monkey, ape, hamster, rat, or mouse cell. In some embodiments, the cell is eukaryotic and is selected from the following cells: CHO (e.g., CHO K1, DXB-11 CHO, Veggie-CHO), COS (e.g., COS-7), retinal cell, Vero, CV1, kidney (e.g., HEK293, 293 EBNA, MSR 293, HaK, BHK), HeLa, HepG2, WI38, MRC 5, Colo205, HB 8065, HL-60, (e.g., BHK21), Jurkat, Daudi, A431 (epidermal), CV-1, U937, 3T3, L cell, C127 cell, SP2/0, NS-0, MMT 060562, Sertoli cell, BRL 3A cell, HT1080 cell, myeloma cell, tumor cell, and a cell line derived from an aforementioned cell. In some embodiments, the cell comprises one or more viral genes, e.g., a retinal cell that expresses a viral gene (e.g., a PER.C6^{™} cell). In some embodiments, the host cell is a SF9 cell of *Spodoptera frugiperda. See* U.S. Patent No. 6,103,526, which is hereby incorporated by reference in its entirety. In some embodiments, the host cell is a SF9 cell of *Spodoptera frugiperda* which has been infected with a baculovirus vector (e.g., *Autographa californica* nuclear polyhedrosis virus). In some embodiments, the host cell is a CHO cell. In some embodiments, encompassed herein is an influenza HA polypeptide expressed in a SF9 cell of *Spodoptera frugiperda* which has been infected with a baculovirus vector (e.g., *Autographa californica* nuclear polyhedrosis virus). In some embodiments, encompassed herein is an influenza NA polypeptide expressed in a SF9 cell of *Spodoptera frugiperda* which has been infected with a baculovirus vector (e.g., *Autographa californica* nuclear polyhedrosis virus). In some embodiments, encompassed herein is an influenza HA polypeptide expressed in a CHO cell. In some embodiments, encompassed herein is an influenza NA polypeptide expressed in a CHO cell.

The influenza vaccine according to the present disclosure can be monovalent or multivalent. In some embodiments, the vaccine is a monovalent influenza vaccine. In some embodiments, the vaccine is a multivalent influenza vaccine, such as a trivalent, quadrivalent, pentavalent, or octavalent influenza vaccine. In some embodiments, the vaccine is a trivalent influenza vaccine. In some embodiments, the vaccine is a quadrivalent influenza vaccine. In some embodiments, the vaccine is a pentavalent influenza vaccine. In some embodiments, the vaccine is a hexavalent influenza vaccine. In some embodiments, the vaccine is a heptavalent influenza vaccine. In some embodiments, the vaccine is an octavalent influenza vaccine.

In some embodiments, the influenza vaccine according to the present disclosure comprises one or more, such as 1-8, hemagglutinins from one or more, such as 1-8, influenza strains. In some embodiments, the influenza vaccine according to the present disclosure comprises one or more, such as 1-8, neuraminidases from one or more, such as 1-8, influenza strains. In some embodiments, the influenza vaccine according to the present disclosure comprises one or more hemagglutinins from one or more influenza strains and one or more neuraminidases from one or more influenza strains. In some embodiments, the influenza vaccine according to the present disclosure comprises 1-8 hemagglutinins from one or more influenza strains and/or 1-8 neuraminidases from one or more influenza strains.

In some embodiments, the vaccine prepared according to the method of the present disclosure is a multivalent influenza vaccine comprising at least one viral protein obtained from an influenza A virus and at least one viral protein obtained from an influenza B virus. In some embodiments, the vaccine is a trivalent influenza vaccine comprising a first viral protein obtained from an H1N1 influenza virus strain, a second viral protein obtained from an H3N2 influenza virus strain, and a third viral protein obtained from an influenza virus of a B/Victoria lineage. In such embodiments, the method comprises preparing at least one viral protein from each influenza virus strain according to the present disclosure, and then combining and formulating the first, second, and third viral proteins into the trivalent influenza vaccine. In some embodiments, the first, second, and third viral proteins comprise influenza hemagglutinin proteins. In some embodiments, the amount of influenza hemagglutinin protein in each of the first, second, and third viral proteins is between about 5 µg to about 120 µg, such as from about 10 µg to about 80 µg, from about 15 µg to about 60 µg, or from about 20 µg to about 45 µg, including all values and subranges therebetween, per 0.5 mL dose of the vaccine. In some embodiments, the amount of influenza hemagglutinin protein in each of the first, second, and third viral proteins is between about 15 µg to about 60 µg per 0.5 mL dose of the vaccine. In some embodiments, the amount of influenza hemagglutinin protein in each of the first, second, and third viral proteins is about 15 µg per 0.5 mL dose of the vaccine. In some embodiments, the amount of influenza hemagglutinin protein in each of the first, second, and third viral proteins is about 45 µg per 0.5 mL dose of the vaccine.

In some embodiments, the vaccine prepared according to the method of the present disclosure is a quadrivalent influenza vaccine comprising a first viral protein obtained from an H1N1 influenza virus strain, a second viral protein obtained from an H3N2 influenza virus strain, a third viral protein obtained from an influenza virus of a B/Victoria lineage, and a fourth viral protein obtained from an influenza virus of a B/Yamagata lineage. In such embodiments, the methods comprise preparing at least one viral protein from each influenza virus strain according to the present disclosure, and then combining and formulating the first, second, third, and fourth viral proteins into the quadrivalent influenza vaccines. In some embodiments, the first, second, third, and fourth viral proteins comprise influenza hemagglutinin proteins. In some embodiments, the amount of influenza hemagglutinin protein in each of the first, second, third, and fourth viral proteins is between about 5 µg to about 120 µg, such as from about 10 µg to about 80 µg, from about 15 µg to about 60 µg, or from about 20 µg to about 45 µg, including all values and subranges therebetween, per 0.5 mL dose of the vaccine. In some embodiments, the amount of influenza hemagglutinin protein in each of the first, second, third, and fourth viral proteins is between about 15 µg to about 60 µg per 0.5 mL dose of the vaccine. In some embodiments, the amount of influenza hemagglutinin protein in each of the first, second, third, and fourth viral proteins is about 15 µg per 0.5 mL dose of the vaccine. In some embodiments, the amount of influenza hemagglutinin protein in each of the first, second, third, and fourth viral proteins is about 45 µg per 0.5 mL dose of the vaccine.

In some embodiments, the vaccine prepared according to the method of the present disclosure further comprises a pharmaceutically acceptable carrier as described herein elsewhere, such as sodium phosphate buffered isotonic sodium chloride. In some embodiments, the vaccine prepared according to the method of the present disclosure further comprises a preservative as described herein elsewhere, such as mercury or a mercury derivative. Any mercury derivatives known in the art, such as thimerosal, which is a mercuric derivative of thiosalicylic acid, can be used. In some embodiments, the vaccine prepared according to the method of the present disclosure comprises mercury as the preservative. In some embodiments, the vaccine prepared according to the method of the present disclosure comprises thimerosal as preservative.

In the embodiments where formaldehyde is used to inactivate viruses during the preparation of the at least one viral protein, a trace amount of formaldehyde may remain in the final vaccine product. Accordingly, in some embodiments, the vaccine prepared according to the method of the present disclosure may comprise formaldehyde at a concentration of no more than about 100 µg/0.5 mL, such as about 1 µg/0.5 mL, about 5 µg/0.5 mL, about 10 µg/0.5 mL, about 20 µg/0.5 mL, about 30 µg/0.5 mL, about 40 µg/0.5 mL, about 50 µg/0.5 mL, about 60 µg/0.5 mL, about 70 µg/0.5 mL, about 80 µg/0.5 mL, about 90 µg/0.5 mL, or about 100 µg/0.5 mL, including all values and subranges therebetween. In some embodiments, the vaccine prepared according to the method of the present disclosure comprises formaldehyde at a concentration of no more than about 100 µg/0.5 mL.

It has been found that, the trace amount of the secondary alcohol ethoxylates as described herein, such as TERGITOL^{™} 15-S-9 or DEVIRON^{™} 13-S9, can prevent protein aggregation, and thus serves as a stabilizing agent, during the manufacturing process. Thus, in addition to the splitting function, the secondary alcohol ethoxylates as described herein, such as TERGITOL^{™} 15-S-9 or DEVIRON^{™} 13-S9, can also have a stabilization function. In some embodiments, therefore, provided herein is the use of the secondary alcohol ethoxylates as described herein, such as TERGITOL^{™} 15-S-9 or DEVIRON^{™} 13-S9, as a stabilizing agent in a manufacturing process of a vaccine. The amount of the secondary alcohol ethoxylates as described herein, such as TERGITOL^{™} 15-S-9 or DEVIRON^{™} 13-S9, to be used as a stabilizing agent can be determined by those skilled in the art but typically is not more than about 10%, such as 9%, 8%, 7%, 6%, 5%, 4.5%, 4%, 3.5%, 3%, 2.5%, 2%, 1.5%, 1%, 0.9%, 0.8%, 0.7%, 0.6%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, or 0.001%, by volume, including all values and subranges therebetween.

It has also been found that the trace amount of the secondary alcohol ethoxylates as described herein, such as TERGITOL^{™} 15-S-9 or DEVIRON^{™} 13-S9, can facilitate inactivation and/or removal of adventitious agents during the manufacturing process. Adventitious agents include, but are not limited to, microorganisms that have been unintentionally introduced into the manufacturing process, such as bacteria, viruses, fungi, mycoplasma, mycobacteria, and parasites. For instance, during the manufacturing process of an influenza split virion vaccine, the presence of the secondary alcohol ethoxylates as described herein, such as TERGITOL^{™} 15-S-9 or DEVIRON^{™} 13-S9, optionally in combination with a second viral inactivating agent, such as formaldehyde, can help to inactivate any influenza virus remaining in the vaccine preparation. In other embodiments, therefore, provided herein is the use of the secondary alcohol ethoxylates as described herein, such as TERGITOL^{™} 15-S-9 or DEVIRON^{™} 13-S9, to inactivate and/or remove an adventitious agent from any immunogenic compositions or vaccines. The amount of the secondary alcohol ethoxylates as described herein, such as TERGITOL^{™} 15-S-9 or DEVIRON^{™} 13-S9, to be used can be determined by those skilled in the art but typically not more than about 10%, such as 9%, 8%, 7%, 6%, 5%, 4.5%, 4%, 3.5%, 3%, 2.5%, 2%, 1.5%, 1%, 0.9%, 0.8%, 0.7%, 0.6%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, or 0.001%, by volume, including all values and subranges therebetween.

It has further been found that the trace amount of the secondary alcohol ethoxylates as described herein, such as TERGITOL^{™} 15-S-9 or DEVIRON^{™} 13-S9, can be used as an excipient to preserve vaccine potency (e.g., stability). In further embodiments, accordingly, provided herein is the use of the secondary alcohol ethoxylates as described herein, such as TERGITOL^{™} 15-S-9 or DEVIRON^{™} 13-S9, as an excipient for any immunogenic compositions and vaccines. The amount of the secondary alcohol ethoxylates as described herein, such as TERGITOL^{™} 15-S-9 or DEVIRON^{™} 13-S9, to be used as an excipient can be determined by those skilled in the art but typically not more than about 10%, such as 9%, 8%, 7%, 6%, 5%, 4.5%, 4%, 3.5%, 3%, 2.5%, 2%, 1.5%, 1%, 0.9%, 0.8%, 0.7%, 0.6%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, or 0.001%, by volume, including all values and subranges therebetween. In some embodiments, the secondary alcohol ethoxylates as described herein, such as TERGITOL^{™} 15-S-9 or DEVIRON^{™} 13-S9, is added as an excipient to the immunogenic composition or vaccine. In some embodiments, the secondary alcohol ethoxylates as described herein, such as TERGITOL^{™} 15-S-9 or DEVIRON^{™} 13-S9, is present in the immunogenic composition or vaccine because it is not fully removed during the manufacturing process of the immunogenic composition or vaccine. In some embodiments, the secondary alcohol ethoxylates as described herein, such as TERGITOL^{™} 15-S-9 or DEVIRON^{™} 13-S9, is present in a first amount in the immunogenic composition or vaccine because it is not fully removed during the manufacturing process of the immunogenic composition or vaccine and is added to the immunogenic composition or vaccine in a second amount as an excipient.

### Immunogenic Compositions and Vaccines

The present disclosure also relates to immunogenic compositions that comprise an inactivated virus or at least one viral protein prepared using the secondary alcohol ethoxylate disclosed herein as the detergent in lieu of Triton X-100. As used herein, the term "immunogenic composition" refers to a composition that generates an immune response that may or may not be a protective immune response or protective immunity. The term "immune response" refers to a response of a cell of the immune system, such as a B cell, T cell, dendritic cell, macrophage or polymorphonucleocyte, to a stimulus such as an antigen, immunogen, or vaccine. An immune response can include any cell of the body involved in a host defense response, including for example, an epithelial cell that secretes an interferon or a cytokine. An immune response includes, but is not limited to, an innate and/or adaptive immune response. Methods of measuring immune responses are well known in the art and include, for example, measuring proliferation and/or activity of lymphocytes (such as B or T cells), secretion of cytokines or chemokines, inflammation, antibody production and the like. An antibody response or humoral response is an immune response in which antibodies are produced. A "cellular immune response" is one mediated by T cells and/or other white blood cells.

Also provided herein is a vaccine manufactured by any of the methods disclosed herein or a vaccine comprising the immunogenic composition of the present disclosure and a pharmaceutically acceptable carrier. As used herein, the term "vaccine" refers to a composition that generates a protective immune response or protective immunity in a subject. A "protective immune response" or "protective immunity" refers to an immune response that protects a subject from infection (prevents infection or prevents the development of disease associated with infection) or reduces the symptoms of infection (for instance, an infection by an influenza virus). Vaccines may elicit both prophylactic (preventative) and therapeutic responses. Methods of administration vary according to the vaccine, but may include inoculation, ingestion, inhalation or other forms of administration. Inoculations can be delivered by any of a number of routes, including parenteral, such as intravenous, subcutaneous, intraperitoneal, intradermal, intranasal, by inhalation, or intramuscular.

The term "pharmaceutically acceptable" means that the carrier, at the dosages and concentrations employed, will not cause unwanted or harmful effects in the subjects to which they are administered. Such pharmaceutically acceptable carriers and excipients are well known in the art (see e.g., Remington's Pharmaceutical Sciences, 19th ed., Mack Publishing Co., Easton, PA, 1995; Pharmaceutical Formulation Development of Peptides and Proteins, S. Frokjaer and L. Hovgaard, Eds., Taylor & Francis, 2000; and Handbook of Pharmaceutical Excipients, 3rd ed., A. Kibbe, Ed., Pharmaceutical Press, 2000). The term "carrier" refers to a diluent, adjuvant, excipient, or vehicle with which the composition is administered. Saline solutions and aqueous dextrose and glycerol solutions can, e.g., be employed as liquid carriers, particularly for injectable solutions. The exact formulation should suit the mode of administration. The immunogenic composition or vaccine of the present disclosure preferably are formulated and administered as a sterile solution. Sterile solutions are prepared by sterile filtration or by other methods known in the art. The solutions can then be lyophilized or filled into pharmaceutical dosage containers. The pH of the solution generally is in the range of pH 3.0 to 9.5, such as pH 5.0 to 7.5.

Accordingly, in some embodiments, provided herein is an immunogenic composition or vaccine comprising an inactivated virus or at least one viral protein and a trace amount of a detergent comprising a compound having a CAS registry number of CAS 68131-40-8 or CAS 84133-50-6. In some embodiments, the at least one viral protein is obtained from the inactivated virus. In some embodiments, the at least one viral protein is a recombinant protein. In some embodiments, the detergent comprised in the immunogenic composition or vaccine of the present disclosure does not comprise a compound having a CAS registry number of CAS 9002-93-1 or CAS 9036-19-5. The compound having a CAS registry number of CAS 68131-40-8 or CAS 84133-50-6 can be the only detergent in the immunogenic composition or vaccine of the present disclosure. Accordingly, in some embodiments, the detergent in the immunogenic composition or vaccine of the present disclosure consists of the compound having the CAS registry number of CAS 68131-40-8. In some embodiments, the detergent in the immunogenic composition or vaccine of the present disclosure consists of the compound having the CAS registry number of CAS 84133-50-6. In some embodiments, the detergent comprising a compound having a CAS registry number of CAS 68131-40-8 or CAS 84133-50-6 is present in the immunogenic composition or vaccine because the detergent is used in processing steps during the manufacture of the immunogenic composition or vaccine. Such processing steps can include, for example, inactivating viruses, purifying viral antigens, and/or disintegrating virus particles.

The trace amount of the detergent in the immunogenic composition or vaccine of the present disclosure is an extremely small amount that is barely detectable. In some embodiments, the trace amount is a detectable amount but the amount detected is extremely small and insignificant. In some embodiments, the trace amount of the detergent in the immunogenic composition or vaccine of the present disclosure is not more than about 5%, such as 4.5%, 4%, 3.5%, 3%, 2.5%, 2%, 1.5%, 1%, 0.9%, 0.8%, 0.7%, 0.6%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, or 0.001%, by volume, including all values and subranges therebetween. In some embodiments, the trace amount of the detergent in the immunogenic composition or vaccine of the present disclosure is an amount that cannot be detected using any analytical methods known in the art.

In some embodiments, the immunogenic composition or vaccine of the present disclosure contains no more than about 500 µg/0.5 mL, such as about 5 µg/0.5 mL, about 25 µg/0.5 mL, about 50 µg/0.5 mL, about 75 µg/0.5mL, about 100 µg/0.5 mL, about 125 µg/0.5 mL, about 150 µg/0.5 mL, about 175 µg/0.5 mL, about 200 µg/0.5 mL, about 225 µg/0.5 mL, about 250 µg/0.5 mL, about 275 µg/0.5 mL, about 300 µg/0.5 mL, about 325 µg/0.5 mL, about 350 µg/0.5 mL, about 375 µg/0.5 mL, about 400 µg/0.5 mL, about 425 µg/0.5 mL, about 450 µg/0.5 mL, about 475 µg/0.5 mL, or about 500 µg/0.5 mL, including all values and subranges therebetween, of the compound having the CAS registry number of CAS 68131-40-8. In some embodiments, the immunogenic composition or vaccine of the present disclosure contains no more than about 500 µg/0.5 mL, such as about 5 µg/0.5 mL, about 25 µg/0.5 mL, about 50 µg/0.5 mL, about 75 µg/0.5mL, about 100 µg/0.5 mL, about 125 µg/0.5 mL, about 150 µg/0.5 mL, about 175 µg/0.5 mL, about 200 µg/0.5 mL, about 225 µg/0.5 mL, about 250 µg/0.5 mL, about 275 µg/0.5 mL, about 300 µg/0.5 mL, about 325 µg/0.5 mL, about 350 µg/0.5 mL, about 375 µg/0.5 mL, about 400 µg/0.5 mL, about 425 µg/0.5 mL, about 450 µg/0.5 mL, about 475 µg/0.5mL, or about 500 µg/0.5 mL, including all values and subranges therebetween, of the compound having the CAS registry number of CAS 84133-50-6.

In some embodiments, the immunogenic composition or vaccine of the present disclosure comprises an inactivated virus, or at least one viral protein obtained from the inactivated virus, including, but not limited to, an influenza virus, a respiratory syncytial virus, a coronavirus, a rotavirus, a papillomavirus, a flavivirus, or a paramyxovirus. In some embodiments, the immunogenic composition or vaccine of the present disclosure comprises at least one viral protein obtained from an inactivated virus including, but not limited to, an influenza virus, a respiratory syncytial virus, a coronavirus, a rotavirus, a papillomavirus, a flavivirus, or a paramyxovirus. In some embodiments, the immunogenic composition or vaccine of the present disclosure comprises at least one recombinant viral protein from a virus including, but not limited to, an influenza virus, a respiratory syncytial virus, a coronavirus, a rotavirus, a papillomavirus, a flavivirus, or a paramyxovirus.

In some embodiments, the inactivated virus in the immunogenic composition or vaccine of the present disclosure comprises an inactivated influenza A virus, or the at least one viral protein in the immunogenic composition or vaccine of the present disclosure is from an influenza A virus. In some embodiments, the at least one viral protein is obtained from an inactivated influenza A virus. In some embodiments, the at least one viral protein is a recombinant influenza A virus protein. In some embodiments, the influenza A virus is an H1N1 influenza virus strain. In some embodiments, the influenza A virus is an H3N2 influenza virus strain.

In some embodiments, the inactivated virus in the immunogenic composition or vaccine of the present disclosure comprises an inactivated influenza B virus, or the at least one viral protein in the immunogenic composition or vaccine of the present disclosure is from an influenza B virus. In some embodiments, the at least one viral protein is obtained from an inactivated influenza B virus. In some embodiments, the at least one viral protein is a recombinant influenza B virus protein. In some embodiments, the influenza B virus is a B/Victoria lineage. In some embodiments, the influenza B virus is a B/Yamagata lineage.

In some embodiments, the at least one viral protein in the immunogenic composition or vaccine of the present disclosure comprises an influenza hemagglutinin protein. In some embodiments, the at least one viral protein in the immunogenic composition or vaccine of the present disclosure comprises an influenza neuraminidase protein. In some embodiments, the at least one viral protein in the immunogenic composition or vaccine of the present disclosure comprises an influenza hemagglutinin protein and an influenza neuraminidase protein. In some embodiments, the influenza hemagglutinin protein and/or neuraminidase protein is obtained from an inactivated influenza virus. In some embodiments, the influenza hemagglutinin protein and/or neuraminidase protein is a recombinant protein.

In some embodiments, the at least one viral protein in the immunogenic composition or vaccine of the present disclosure comprises a recombinant influenza structural protein, such as a recombinant influenza hemagglutinin protein or a recombinant influenza neuraminidase protein. Accordingly, in some embodiments, the at least one viral protein in the immunogenic composition or vaccine of the present disclosure comprises a recombinant influenza hemagglutinin protein. In some embodiments, the at least one viral protein in the immunogenic composition or vaccine of the present disclosure comprises a recombinant influenza neuraminidase protein. In some embodiments, the at least one viral protein in the immunogenic composition or vaccine of the present disclosure comprises a recombinant influenza hemagglutinin protein and a recombinant influenza neuraminidase protein.

In some embodiments, the immunogenic composition or vaccine of the present disclosure comprises at least one viral protein from an influenza A virus and at least one viral protein from an influenza B virus. In some embodiments, the immunogenic composition or vaccine of the present disclosure comprises a first viral protein from an H1N1 influenza virus strain, a second viral protein from an H3N2 influenza virus strain, and a third viral protein from an influenza virus of a B/Victoria lineage. In some embodiments, the first, second, and third viral proteins are influenza hemagglutinin proteins. In some embodiments, the first, second, and third influenza hemagglutinin proteins are obtained from an inactivated influenza virus. In some embodiments, the first, second, and third influenza hemagglutinin proteins are recombinant proteins. In some embodiments, the first, second, and third viral proteins are influenza neuraminidase proteins. In some embodiments, the first, second, and third influenza neuraminidase proteins are obtained from an inactivated influenza virus. In some embodiments, the first, second, and third influenza neuraminidase proteins are recombinant proteins. In some embodiments, the immunogenic composition or vaccine of the present disclosure comprises a first influenza hemagglutinin protein and a first influenza neuraminidase protein from an H1N1 influenza virus strain, a second influenza hemagglutinin protein and a second influenza neuraminidase protein from an H3N2 influenza virus strain, and a third influenza hemagglutinin protein and a third influenza neuraminidase protein from an influenza virus of a B/Victoria lineage. In some embodiments, the first, second, and third influenza hemagglutinin proteins and the first, second, and third influenza neuraminidase proteins are obtained from an inactivated influenza virus. In some embodiments, the first, second, and third influenza hemagglutinin proteins and the first, second, and third influenza neuraminidase proteins are recombinant proteins.

In some embodiments, the immunogenic composition or vaccine of the present disclosure comprises a first viral protein from an H1N1 influenza virus strain, a second viral protein from an H3N2 influenza virus strain, a third viral protein from an influenza virus of a B/Victoria lineage, and a fourth viral protein from an influenza virus of a B/Yamagata lineage. In some embodiments, the first, second, third, and fourth viral proteins are influenza hemagglutinin proteins. In some embodiments, the first, second, third, and fourth influenza hemagglutinin proteins are obtained from an inactivated influenza virus. In some embodiments, the first, second, third, and fourth influenza hemagglutinin proteins are recombinant proteins. In some embodiments, the first, second, third, and fourth viral proteins are influenza neuraminidase proteins. In some embodiments, the first, second, third, and fourth influenza neuraminidase proteins are obtained from an inactivated influenza virus. In some embodiments, the first, second, third, and fourth influenza neuraminidase proteins are recombinant proteins. In some embodiments, the immunogenic composition or vaccine of the present disclosure comprises a first influenza hemagglutinin protein and a first influenza neuraminidase protein from an H1N1 influenza virus strain, a second influenza hemagglutinin protein and a second influenza neuraminidase protein from an H3N2 influenza virus strain, a third influenza hemagglutinin protein and a third influenza neuraminidase protein from an influenza virus of a B/Victoria lineage, and a fourth influenza hemagglutinin protein and a fourth influenza neuraminidase protein from an influenza virus of a B/Yamagata lineage. In some embodiments, the first, second, third, and fourth influenza hemagglutinin proteins and the first, second, third, and fourth influenza neuraminidase proteins are obtained from an inactivated influenza virus. In some embodiments, the first, second, third, and fourth influenza hemagglutinin proteins and the first, second, third, and fourth influenza neuraminidase proteins are recombinant proteins.

Each viral protein may be present in the immunogenic composition or vaccine of the present disclosure in an amount effective to induce an immune response in a subject to which the immunogenic composition or vaccine is administered. In some embodiments, each viral protein may be present in the immunogenic composition or vaccine of the present disclosure in an amount ranging, for example, from about 5 µg to about 120 µg, such as from about 10 µg to about 80 µg, from about 15 µg to about 60 µg, or from about 20 µg to about 45 µg, including all values and subranges therebetween per 0.5 mL dose of the immunogenic composition or vaccine. In some embodiments, each viral protein in the immunogenic composition or vaccine of the present disclosure is an influenza hemagglutinin protein present in an amount between about 5 µg to about 60 µg per 0.5 mL dose of the immunogenic composition or vaccine. In some embodiments, each viral protein in the immunogenic composition or vaccine of the present disclosure is an influenza hemagglutinin protein present in an amount of about 5 µg per 0.5 mL dose of the immunogenic composition or vaccine. In some embodiments, each viral protein in the immunogenic composition or vaccine of the present disclosure is an influenza hemagglutinin protein present in an amount of about 15 µg per 0.5 mL dose of the immunogenic composition or vaccine. In some embodiments, each viral protein in the immunogenic composition or vaccine of the present disclosure is an influenza hemagglutinin protein present in an amount of about 45 µg per 0.5 mL dose of the immunogenic composition or vaccine. In some embodiments, each viral protein in the immunogenic composition or vaccine of the present disclosure is an influenza neuraminidase protein present in an amount between about 5 µg to about 60 µg per 0.5 mL dose of the immunogenic composition or vaccine. In some embodiments, each viral protein in the immunogenic composition or vaccine of the present disclosure is an influenza neuraminidase protein present in an amount of about 5 µg per 0.5 mL dose of the immunogenic composition or vaccine. In some embodiments, each viral protein in the immunogenic composition or vaccine of the present disclosure is an influenza neuraminidase protein present in an amount of about 15 µg per 0.5 mL dose of the immunogenic composition or vaccine. In some embodiments, each viral protein in the immunogenic composition or vaccine of the present disclosure is an influenza neuraminidase protein present in an amount of about 45 µg per 0.5 mL dose of the immunogenic composition or vaccine.

To prolong the shelf-life of the immunogenic composition or vaccine of the present disclosure, and/or to facilitate administration of the immunogenic composition or vaccine, the immunogenic composition or vaccine can be formulated in combination with one or more carriers, targeting ligands, stabilizing reagents (e.g., preservatives and antioxidants), and/or other pharmaceutically acceptable excipients. Examples of such excipients are parabens, thimerosal, thiomersal, chlorobutanol, bezalkonium chloride, and chelators (e.g., EDTA). In some embodiments, the immunogenic composition or vaccine of the present disclosure comprises sodium phosphate buffered isotonic sodium chloride. In some embodiments, the immunogenic composition or vaccine of the present disclosure comprises a preservative, such as mercury or a mercury derivative. In some embodiments, the immunogenic composition or vaccine of the present disclosure comprises mercury as the preservative. In some embodiments, the immunogenic composition or vaccine of the present disclosure comprises thimerosal as preservative.

In some embodiments, the immunogenic composition or vaccine of the present disclosure comprises a trace amount of formaldehyde at a concentration of no more than about 100 µg/0.5 mL, such as about 1 µg/0.5 mL, about 5 µg/0.5 mL, about 10 µg/0.5 mL, about 20 µg/0.5 mL, about 30 µg/0.5 mL, about 40 µg/0.5 mL, about 50 µg/0.5 mL, about 60 µg/0.5 mL, about 70 µg/0.5 mL, about 80 µg/0.5 mL, about 90 µg/0.5 mL, or about 100 µg/0.5 mL, including all values and subranges therebetween.

The immunogenic composition or vaccine of the present disclosure may be provided as a frozen liquid form or a lyophilized form. A variety of cryoprotectants may be used, including, without limitations, sucrose, trehalose, glucose, mannitol, mannose, dextrose, and the like. The cryoprotectant may constitute 5-30% (w/v) of the immunogenic composition or vaccine. In some embodiments, the immunogenic composition or vaccine comprises trehalose, e.g., at 5-30% (e.g., 10%) (w/v). Once formulated with the cryoprotectant, the immunogenic composition or vaccine may be frozen (or lyophilized and cryopreserved) at -20°C to -80°C. The immunogenic composition or vaccine may be provided to a subject in an aqueous buffered solution - thawed if previously frozen, or if previously lyophilized, reconstituted in an aqueous buffered solution prior to administration. The buffered solution preferably is isotonic and suitable for e.g., intramuscular or intradermal injection. In some embodiments, the buffered solution is a phosphate-buffered saline (PBS).

The vaccine of the present disclosure can be any type of vaccine known in the art. In some embodiments, the vaccine of the present disclosure is a live attenuated virus vaccine. In some embodiments, the vaccine of the present disclosure is an inactivated whole virus vaccine. In some embodiments, the vaccine of the present disclosure is a virosome vaccine. In some embodiments, the vaccine of the present disclosure is a split-virion vaccine. In some embodiments, the vaccine of the present disclosure is a subunit vaccine. In some embodiments, the vaccine of the present disclosure is a recombinant vaccine.

In some embodiments, provided herein is a vaccine comprising 5-60 µg/0.5 mL of an influenza hemagglutinin antigen from each of an H1N1 influenza virus strain, an H3N2 influenza virus strain, and an influenza virus of a B/Victoria lineage, a sodium phosphate-buffered isotonic sodium chloride solution, no more than about 100 µg/0.5 mL of formaldehyde, no more than about 400 µg/0.5 mL of a compound having a CAS registry number of CAS 68131-40-8 or CAS 84133-50-6, and optionally a preservative. In some embodiments, the vaccine comprises no more than about 250 µg/0.5 mL of a compound having a CAS registry number of CAS 68131-40-8 or CAS 84133-50-6. In some embodiments, the vaccine comprises no more than about 250 µg/0.5 mL of a compound having a CAS registry number of CAS 68131-40-8. In some embodiments, the vaccine comprises no more than about 250 µg/0.5 mL of a compound having a CAS registry number of CAS 84133-50-6. In some embodiments, the vaccine does not comprise a compound having a CAS registry number of CAS 9002-93-1 or CAS 9036-19-5. In some embodiments, the vaccine comprises no more than about 30 µg/0.5 mL of formaldehyde. In some embodiments, the vaccine comprises mercury or a mercury derivative, such as thimerosal, as a preservative in an amount of about 2-25 µg/0.5 mL. In some embodiments, the vaccine comprises about 15 µg/0.5 mL of an influenza hemagglutinin antigen from each of an H1N1 influenza virus strain, an H3N2 influenza virus strain, and an influenza virus of a B/Victoria lineage, up to about 0.5 mL of sodium phosphate-buffered isotonic sodium chloride solution, no more than about 30 µg/0.5 mL of formaldehyde, and no more than about 250 µg/0.5 mL of a compound having a CAS registry number of CAS 68131-40-8 or CAS 84133-50-6, and optionally about 2 µg/0.5 mL of thimerosal or about 20 µg/0.5 mL of mercury as a preservative. In some embodiments, the vaccine comprises about 15 µg/0.5 mL of an influenza hemagglutinin antigen from each of an H1N1 influenza virus strain, an H3N2 influenza virus strain, and an influenza virus of a B/Victoria lineage, up to about 0.5 mL of sodium phosphate-buffered isotonic sodium chloride solution, no more than about 100 µg/0.5 mL of formaldehyde, and no more than about 250 µg/0.5 mL of a compound having a CAS registry number of CAS 68131-40-8 or CAS 84133-50-6, and optionally about 20 µg/0.5 mL of mercury as a preservative.

Also provided herein is a vaccine comprising 5-60 µg/0.5 mL of an influenza hemagglutinin antigen from each of an H1N1 influenza virus strain, an H3N2 influenza virus strain, an influenza virus of a B/Victoria lineage, and an influenza virus of a B/Yamagata lineage, a sodium phosphate-buffered isotonic sodium chloride solution, no more than about 100 µg/0.5 mL of formaldehyde, no more than about 400 µg/0.5 mL of a compound having a CAS registry number of CAS 68131-40-8 or CAS 84133-50-6, and optionally a preservative. In some embodiments, the vaccine comprises no more than about 250 µg/0.5 mL of a compound having a CAS registry number of CAS 68131-40-8 or CAS 84133-50-6. In some embodiments, the vaccine comprises no more than about 250 µg/0.5 mL of a compound having a CAS registry number of CAS 68131-40-8. In some embodiments, the vaccine comprises no more than about 250 µg/0.5 mL of a compound having a CAS registry number of CAS 84133-50-6. In some embodiments, the vaccine does not comprise a compound having a CAS registry number of CAS 9002-93-1 or CAS 9036-19-5. In some embodiments, the vaccine comprises no more than about 30 µg/0.5 mL of formaldehyde. In some embodiments, the vaccine comprises mercury or a mercury derivative, such as thimerosal, as a preservative in an amount of about 2-25 µg/0.5 mL. In some embodiments, the vaccine comprises about 15 µg/0.5 mL of an influenza hemagglutinin antigen from each of an H1N1 influenza virus strain, an H3N2 influenza virus strain, an influenza virus of a B/Victoria lineage, and an influenza virus of a B/Yamagata lineage, a sodium phosphate-buffered isotonic sodium chloride solution, no more than about 30 µg/0.5 mL of formaldehyde, no more than about 250 µg/0.5 mL of a compound having a CAS registry number of CAS 68131-40-8 or CAS 84133-50-6, and optionally about 2 µg/0.5 mL of thimerosal as a preservative. In some embodiments, the vaccine comprises about 15 µg/0.5 mL of an influenza hemagglutinin antigen from each of an H1N1 influenza virus strain, an H3N2 influenza virus strain, an influenza virus of a B/Victoria lineage, and an influenza virus of a B/Yamagata lineage, a sodium phosphate-buffered isotonic sodium chloride solution, no more than about 100 µg/0.5 mL of formaldehyde, no more than about 250 µg/0.5 mL of a compound having a CAS registry number of CAS 68131-40-8 or CAS 84133-50-6, and optionally about 25 µg/0.5 mL of mercury as a preservative.

In some embodiments, provided herein is a vaccine comprising about 5-60 µg/0.5 mL of an influenza hemagglutinin antigen from each of an H1N1 influenza virus strain, an H3N2 influenza virus strain, an influenza virus of a B/Victoria lineage, and an influenza virus of a B/Yamagata lineage, a buffer solution comprising sodium chloride, potassium chloride, dibasic sodium phosphate dihydrate, and monobasic potassium phosphate, no more than about 100 µg/0.5 mL of formaldehyde, and no more than about 400 µg/0.5 mL of a compound having a CAS registry number of CAS 68131-40-8 or CAS 84133-50-6. In some embodiments, the vaccine comprises no more than about 250 µg/0.5 mL of a compound having a CAS registry number of CAS 68131-40-8 or CAS 84133-50-6. In some embodiments, the vaccine comprises no more than about 250 µg/0.5 mL of a compound having a CAS registry number of CAS 68131-40-8. In some embodiments, the vaccine comprises no more than about 250 µg/0.5 mL of a compound having a CAS registry number of CAS 84133-50-6. In some embodiments, the vaccine does not comprise a compound having a CAS registry number of CAS 9002-93-1 or CAS 9036-19-5. In some embodiments, the vaccine comprises no more than about 30 µg/0.5 mL of formaldehyde. In some embodiments, the vaccine comprises neither an adjuvant nor a preservative. In some embodiments, the vaccine comprises about 15 µg/0.5 mL of an influenza hemagglutinin antigen from each of an H1N1 influenza virus strain, an H3N2 influenza virus strain, an influenza virus of a B/Victoria lineage, and an influenza virus of a B/Yamagata lineage, a buffer solution comprising sodium chloride, potassium chloride, dibasic sodium phosphate dihydrate, and monobasic potassium phosphate, no more than about 30 µg/0.5 mL of formaldehyde, and no more than about 222.5 µg/0.5 mL of a compound having a CAS registry number of CAS 68131-40-8 or CAS 84133-50-6.

In some embodiments, provided herein is a vaccine comprising about 5-60 µg/0.5 mL of a recombinant influenza hemagglutinin antigen from each of an H1N1 influenza virus strain, an H3N2 influenza virus strain, and an influenza virus of a B/Victoria lineage, sodium chloride, monobasic sodium phosphate, dibasic sodium phosphate, polysorbate 20, and no more than about 400 µg/0.5 mL of a compound having a CAS registry number of CAS 68131-40-8 or CAS 84133-50-6. In some embodiments, the vaccine comprises no more than about 200 µg/0.5 mL of a compound having a CAS registry number of CAS 68131-40-8 or CAS 84133-50-6. In some embodiments, the vaccine comprises no more than about 100 µg/0.5 mL of a compound having a CAS registry number of CAS 68131-40-8 or CAS 84133-50-6. In some embodiments, the vaccine comprises no more than about 100 µg/0.5 mL of a compound having a CAS registry number of CAS 68131-40-8. In some embodiments, the vaccine comprises no more than about 100 µg/0.5 mL of a compound having a CAS registry number of CAS 84133-50-6. In some embodiments, the vaccine does not comprise a compound having a CAS registry number of CAS 9002-93-1 or CAS 9036-19-5. In some embodiments, the vaccine comprises about 4-6 mg/0.5 mL sodium chloride, about 0.150-0.250 mg/0.5 mL monobasic sodium phosphate, about 0.25-2 mg/0.5 mL dibasic sodium phosphate, and about 20-35 µg/0.5 mL polysorbate 20. In some embodiments, the vaccine comprises about 15-60 µg/0.5 mL of a recombinant influenza hemagglutinin antigen from each of an H1N1 influenza virus strain, an H3N2 influenza virus strain, and an influenza virus of a B/Victoria lineage and an optional adjuvant. In some embodiments, the vaccine comprises about 5-15 µg/0.5 mL of a recombinant influenza hemagglutinin antigen from each of an H1N1 influenza virus strain, an H3N2 influenza virus strain, and an influenza virus of a B/Victoria lineage and an adjuvant.

In some embodiments, the vaccine comprises about 5-45 µg/0.5 mL of a recombinant influenza hemagglutinin antigen from each of an H1N1 influenza virus strain, an H3N2 influenza virus strain, and an influenza virus of a B/Victoria lineage, about 4.4 mg/0.5 mL sodium chloride, about 0.2 mg/0.5 mL monobasic sodium phosphate, about 0.5 mg/0.5 mL dibasic sodium phosphate, about 27.5 µg/0.5 mL polysorbate 20, and no more than about 100 µg/0.5 mL of a compound having a CAS registry number of CAS 68131-40-8 or CAS 84133-50-6. In some embodiments, the vaccine comprises 45 µg/0.5 mL of a recombinant influenza hemagglutinin antigen from each of an H1N1 influenza virus strain, an H3N2 influenza virus strain, and an influenza virus of a B/Victoria lineage, optionally without an adjuvant. In some embodiments, the vaccine comprises 5-15 µg/0.5 mL of a recombinant influenza hemagglutinin antigen from each of an H1N1 influenza virus strain, an H3N2 influenza virus strain, and an influenza virus of a B/Victoria lineage and further comprises an adjuvant. In some embodiments, the vaccine comprises about 5-45 µg/0.5 mL of a recombinant influenza hemagglutinin antigen from each of an H1N1 influenza virus strain, an H3N2 influenza virus strain, and an influenza virus of a B/Victoria lineage, about 4.4 mg/0.5 mL sodium chloride, about 0.195 mg/0.5 mL monobasic sodium phosphate, about 1.3 mg /0.5 mL dibasic sodium phosphate, about 27.5 µg/0.5 mL polysorbate 20, and no more than about 100 µg/0.5 mL of a compound having a CAS registry number of CAS 68131-40-8 or CAS 84133-50-6. In some embodiments, the vaccine does not comprise a preservative. In some embodiments, the vaccine comprises 45 µg/0.5 mL of a recombinant influenza hemagglutinin antigen from each of an H1N1 influenza virus strain, an H3N2 influenza virus strain, and an influenza virus of a B/Victoria lineage, optionally without an adjuvant. In some embodiments, the vaccine comprises 5-15 µg/0.5 mL of a recombinant influenza hemagglutinin antigen from each of an H1N1 influenza virus strain, an H3N2 influenza virus strain, and an influenza virus of a B/Victoria lineage and an adjuvant.

Further provided herein is a vaccine comprising about 5-60 µg/0.5 mL of a recombinant influenza hemagglutinin antigen from each of an H1N1 influenza virus strain, an H3N2 influenza virus strain, an influenza virus of a B/Victoria lineage, and an influenza virus of a B/Yamagata lineage, sodium chloride, monobasic sodium phosphate, dibasic sodium phosphate, polysorbate 20, and no more than about 400 µg/0.5 mL of a compound having a CAS registry number of CAS 68131-40-8 or CAS 84133-50-6. In some embodiments, the vaccine comprises no more than about 200 µg/0.5 mL of a compound having a CAS registry number of CAS 68131-40-8 or CAS 84133-50-6. In some embodiments, the vaccine comprises no more than about 100 µg/0.5 mL of a compound having a CAS registry number of CAS 68131-40-8 or CAS 84133-50-6. In some embodiments, the vaccine comprises no more than about 100 µg/0.5 mL of a compound having a CAS registry number of CAS 68131-40-8. In some embodiments, the vaccine comprises no more than about 100 µg/0.5 mL of a compound having a CAS registry number of CAS 84133-50-6. In some embodiments, the vaccine does not comprise a compound having a CAS registry number of CAS 9002-93-1 or CAS 9036-19-5. In some embodiments, the vaccine comprises about 4-6 mg/0.5 mL sodium chloride, about 0.150-0.250 mg/0.5 mL monobasic sodium phosphate, about 0.25-2 mg/0.5 mL dibasic sodium phosphate, and about 20-35 µg/0.5 mL polysorbate 20. In some embodiments, the vaccine comprises about 15-60 µg/0.5 mL of a recombinant influenza hemagglutinin antigen from each of an H1N1 influenza virus strain, an H3N2 influenza virus strain, an influenza virus of a B/Victoria lineage, and an influenza virus of a B/Yamagata lineage and an optional adjuvant. In some embodiments, the vaccine comprises about 5-15 µg/0.5 mL of a recombinant influenza hemagglutinin antigen from each of an H1N1 influenza virus strain, an H3N2 influenza virus strain, an influenza virus of a B/Victoria lineage, and an influenza virus of a B/Yamagata lineage and an adjuvant.

In some embodiments, the vaccine comprises about 5-45 µg/0.5 mL of an influenza hemagglutinin antigen from each of an H1N1 influenza virus strain, an H3N2 influenza virus strain, an influenza virus of a B/Victoria lineage, and an influenza virus of a B/Yamagata lineage, about 4.4 mg/0.5 mL sodium chloride, about 0.2 mg/0.5 mL monobasic sodium phosphate, about 0.5 mg /0.5 mL dibasic sodium phosphate, about 27.5 µg/0.5 mL polysorbate 20, and no more than about 100 µg/0.5 mL of a compound having a CAS registry number of CAS 68131-40-8 or CAS 84133-50-6. In some embodiments, the vaccine comprises about 45 µg/0.5 mL of an influenza hemagglutinin antigen from each of an H1N1 influenza virus strain, an H3N2 influenza virus strain, an influenza virus of a B/Victoria lineage, and an influenza virus of a B/Yamagata lineage, about 4.4 mg/0.5 mL sodium chloride, about 0.195 mg/0.5 mL monobasic sodium phosphate, about 1.3 mg /0.5 mL dibasic sodium phosphate, about 27.5 µg/0.5 mL polysorbate 20, and no more than about 100 µg/0.5 mL of a compound having a CAS registry number of CAS 68131-40-8 or CAS 84133-50-6. In some embodiments, the vaccine does not comprise a preservative. In some embodiments, the vaccine comprises 45 µg/0.5 mL of a recombinant influenza hemagglutinin antigen from each of an H1N1 influenza virus strain, an H3N2 influenza virus • strain, an influenza virus of a B/Victoria lineage, and an influenza virus of a B/Yamagata lineage, optionally without an adjuvant. In some embodiments, the vaccine comprises 5-15 µg/0.5 mL of a recombinant influenza hemagglutinin antigen from each of an H1N1 influenza virus strain, an H3N2 influenza virus strain, an influenza virus of a B/Victoria lineage, and an influenza virus of a B/Yamagata lineage and further comprises an adjuvant.

The vaccine of the present disclosure generally induces a serum hemagglutinin inhibition (HAI) titer that is comparable to a control vaccine that comprises the same components except that the trace amount of a compound having the CAS registry number of CAS 68131-40-8 or CAS 84133-50-6 is replaced with the same trace amount of a compound having a CAS registry number of CAS 9002-93-1 or CAS 9036-19-5. In some embodiments, the HAI titer induced by the vaccine of the present disclosure is about the same as the serum HAI titer induced by the control vaccine. In some embodiments, the HAI titer induced by the vaccine of the present disclosure is higher than the serum HAI titer induced by the control vaccine. The serum HAI titer can be measured using any methods known in the art.

In some embodiments, the vaccine of the present disclosure induces a serum HAI titer of at least 40, 80, or 160 as measured at 20 days following vaccination. In some embodiments, the vaccine of the present disclosure induces a serum HAI that increases at least about 4-fold, 8-fold, or 16-fold as measured at 20 days following vaccination, as compared to the serum HAI titer prior to vaccination. In some embodiments, the vaccine of the present disclosure induces a serum HAI titer of at least 640, 1280, or 2560 as measured at 35 days following a priming vaccination at day 0 and a boost vaccination at day 21. In some embodiments, the vaccine of the present disclosure induces a serum HAI that increases at least about 4-fold, 8-fold, or 16-fold as measured at 35 days following vaccination at day 0 and a boost vaccination at day 21, as compared to the serum HAI titer at day 20.

### Adjuvants

In some embodiments, the immunogenic composition or vaccine of the present disclosure comprises an adjuvant. In other embodiments, the immunogenic composition or vaccine of the present disclosure does not contain an adjuvant. Similarly, in some embodiments, the vaccine of the present disclosure can be administered with an adjuvant to boost the immune response. In other embodiments, the vaccines can be administered without an adjuvant. As used herein, the term "adjuvant" refers to a substance or combination of substances that may be used to enhance an immune response to an antigen component of a vaccine or immunogenic composition. Adjuvants can include a suspension of minerals (alum, aluminum salts, including, for example, aluminum hydroxide/oxyhydroxide (AlOOH), aluminum phosphate (AlPO₄), aluminum hydroxyphosphate sulfate (AAHS) and/or potassium aluminum sulfate) on which antigen is adsorbed; or water-in-oil emulsion in which antigen solution is emulsified in mineral oil (for example, Freund's incomplete adjuvant), sometimes with the inclusion of killed mycobacteria (Freund's complete adjuvant) to further enhance antigenicity. Immunostimulatory oligonucleotides (such as those including a CpG motif) can also be used as adjuvants (for example, see U.S. Patent Nos. 6,194,388; 6,207,646; 6,214,806; 6,218,371; 6,239,116; 6,339,068; 6,406,705; and 6,429,199). Adjuvants also include biological molecules, such as lipids and costimulatory molecules. Exemplary biological adjuvants include, but are not limited to, AS04 (Didierlaurent et al., J. Immunol., 2009, 183:6186-6197), IL-2, RANTES, GM-CSF, TNF-α, IFN-γ, G-CSF, LFA-3, CD72, B7-1, B7-2, OX-40L and 41 BBL.

In certain embodiments, the adjuvant is a squalene-based adjuvant. In certain embodiments, the squalene based adjuvant comprises an oil-in-water adjuvant emulsion comprising at least: squalene, an aqueous solvent, a polyoxyethylene alkyl ether hydrophilic nonionic surfactant, and a hydrophobic nonionic surfactant. In certain embodiments, the emulsion is thermoreversible, optionally wherein about 90% of the population by volume of the oil drops has a size less than about 200 nm.

In certain embodiments, the polyoxyethylene alkyl ether is of formula CH₃-(CH₂)ₓ-(O-CH₂-CH₂)ₙ-OH, in which n is an integer from 10 to 60, and x is an integer from 11 to 17. In certain embodiments, the polyoxyethylene alkyl ether surfactant is polyoxyethylene(12) cetostearyl ether.

In certain embodiments, about 90% of the population by volume of the oil drops has a size less than about 160 nm. In certain embodiments, about 90% of the population by volume of the oil drops has a size less than about 150 nm. In certain embodiments, about 50% of the population by volume of the oil drops has a size less than about 100 nm. In certain embodiments, about 50% of the population by volume of the oil drops has a size less than about 90 nm.

In certain embodiments, the adjuvant further comprises at least one alditol, including, but not limited to, glycerol, erythritol, xylitol, sorbitol and mannitol.

In some embodiments the hydrophilic/lipophilic balance (HLB) of the hydrophilic nonionic surfactant is greater than or equal to about 10. In certain embodiments, the HLB of the hydrophobic nonionic surfactant is less than about 9. In certain embodiments, the HLB of the hydrophilic nonionic surfactant is greater than or equal to about 10 and the HLB of the hydrophobic nonionic surfactant is less than about 9.

In certain embodiments, the hydrophobic nonionic surfactant is a sorbitan ester, such as sorbitan monooleate, or a mannide ester surfactant. In certain embodiments, the amount of squalene is between about 5% and about 45%. In certain embodiments, the amount of polyoxyethylene alkyl ether surfactant is between about 0.9% and about 9%. In certain embodiments, the amount of hydrophobic nonionic surfactant is between about 0.7% and about 7%. In certain embodiments, the adjuvant comprises: i) about 32.5% of squalene, ii) about 6.18% of polyoxyethylene(12) cetostearyl ether, iii) about 4.82% of sorbitan monooleate, and iv) about 6% of mannitol.

In certain embodiments, the adjuvant further comprises an alkylpolyglycoside and/or a cryoprotective agent, such as a sugar, in particular dodecylmaltoside and/or sucrose. In certain embodiments, the adjuvant further comprises an antioxidant, such as alpha-tocopherol.

In certain embodiments, the adjuvant comprises AF03, as described in Klucker et al., J. Pharm. Sci., 2012, 101(12):4490-4500, which is hereby incorporated by reference in its entirety. In certain embodiments, the adjuvant comprises squalene, alpha-tocopherol, and polysorbate 80. In certain embodiments, the adjuvant comprises AS03, as described in Garcon et al., Expert Rev Vaccines (2012) 11:349-66; Cohet et al., Vaccine (2019) 37(23):3006-21; and WO2006/100109, which is hereby incorporated by reference in its entirety. In certain embodiments, the adjuvant comprises a liposome-based adjuvant, such as SPA14. SPA14 is a liposome-based adjuvant (AS01-like) containing a toll-like receptor 4 (TLR4) agonist (E6020) and saponin (QS21). In certain embodiments, the adjuvant comprises MF59, as described in Podda and Del Giudice (2003) Expert Rev Vaccines 2:197-203; Podda (2001 ) Vaccine 19: 2673-2680; and WO90/14837, which is hereby incorporated by reference in its entirety.

In some embodiments, the vaccine composition does not comprise an adjuvant. In other embodiments, the vaccine composition further comprises an adjuvant.

### Administration

The immunogenic composition or vaccine of the present disclosure can be formulated for administration in any way known in the art of drug delivery, for example, orally, parenterally, intravenously, intramuscularly, subcutaneously, intradermally, transdermally, intrathecally, submucosally, sublingually, rectally, vaginally, etc. In some embodiments, the immunogenic composition or vaccine of the present disclosure is formulated for sublingual administration, intramuscular administration, intradermal administration, subcutaneous administration, intravenous administration, intranasal administration, administration by inhalation, or intraperitoneal administration.

In some embodiments, the immunogenic composition or vaccine of the present disclosure is formulated for parenteral administration, such as intravenous, subcutaneous, intraperitoneal, intradermal, or intramuscular. In some embodiments, the immunogenic composition or vaccine of the present disclosure is formulated for sublingual administration. In some embodiments, the immunogenic composition or vaccine is formulated for intramuscular injection. The immunogenic composition or vaccine of the present disclosure may also be formulated for intranasal or inhalation administration. The immunogenic composition or vaccine of the present disclosure can also be formulated for any other intended route of administration.

In some embodiments, the immunogenic composition or vaccine of the present disclosure is formulated for intradermal injection, intranasal administration or intramuscular injection. General considerations in the formulation and manufacture of pharmaceutical agents for administration by these routes may be found, for example, in Remington's Pharmaceutical Sciences, 19th ed., Mack Publishing Co., Easton, PA, 1995; incorporated herein by reference. At present the oral or nasal spray or aerosol route (e.g., by inhalation) are most commonly used to deliver therapeutic agents directly to the lungs and respiratory system. In some embodiments, the immunogenic composition or vaccine of the present disclosure is administered using a device that delivers a metered dosage of the vaccine composition. Suitable devices for use in delivering intradermal pharmaceutical compositions described herein include short needle devices such as those described in U.S. Patent No. 4,886,499, U.S. Patent No. 5,190,521, U.S. Patent No. 5,328,483, U.S. Patent No. 5,527,288, U.S. Patent No. 4,270,537, U.S. Patent No. 5,015,235, U.S. Patent No. 5,141,496, U.S. Patent No. 5,417,662, all of which are incorporated herein by reference. Intradermal compositions may also be administered by devices which limit the effective penetration length of a needle into the skin, such as those described in WO1999/34850, incorporated herein by reference, and functional equivalents thereof.

In certain embodiments, the immunogenic composition or vaccine of the present disclosure is provided for use in skin injection, e.g., in the epidermis, the dermis or the hypodermis of the skin. In some embodiments, the immunogenic composition or vaccine of the present disclosure is provided in a device suitable for skin injection, such as a needle (e.g., an epidermic, dermic or hypodermic needle), a needle free device, a microneedle device or a microprojection array device. Examples of microneedle or microprojection array devices suitable for the skin injection according to the present disclosure are described in US 2023/0270842A1, US 2022/0339416A1, US 2021/0085598A1, US 2020/0246450A1, US 2022/0143376A1, US 2018/0264244A1, US 2018/0263641A1, and US 2011/0245776A1.

Also suitable are jet injection devices which deliver liquid vaccines to the dermis via a liquid jet injector or via a needle which pierces the stratum corneum and produces a jet which reaches the dermis. Jet injection devices are described for example in U.S. Patent No. 5,480,381, U.S. Patent No. 5,599,302, U.S. Patent No. 5,334,144, U.S. Patent No. 5,993,412, U.S. Patent No. 5,649,912, U.S. Patent No. 5,569,189, U.S. Patent No. 5,704,911, U.S. Patent No. 5,383,851, U.S. Patent No. 5,893,397, U.S. Patent No. 5,466,220, U.S. Patent No. 5,339,163, U.S. Pat. No. 5,312,335, U.S. Pat. No. 5,503,627, U.S. Pat. No. 5,064,413, U.S. Patent No. 5,520,639, U.S. Patent No. 4,596,556, U.S. Patent No. 4,790,824, U.S. Patent No. 4,941,880, U.S. Patent No. 4,940,460, WO1997/37705, and WO1997/13537, all of which are incorporated herein by reference. Additionally, conventional syringes may be used in the classical Mantoux method of intradermal administration.

Preparations for parenteral administration typically include sterile aqueous or nonaqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, antioxidants, chelating agents, and inert gases and the like.

The immunogenic composition or vaccine of the present disclosure may be packaged in a container, such as a prefilled syringe, a vial, or an autoinjector. In some embodiments, the immunogenic composition or vaccine of the present disclosure is packaged in a prefilled syringe. In some embodiments, the immunogenic composition or vaccine of the present disclosure is packaged in a vial. In some embodiments, the immunogenic composition or vaccine of the present disclosure is packaged in an autoinjector. In other embodiments, the immunogenic composition or vaccine of the present disclosure is packaged cartridges for patient-friendly autoinjector and infusion pump devices.

Prefilled syringes provide several advantages over other types of packages, such as convenience, affordability, accuracy, sterility, and safety. Accordingly, in some embodiments, provided herein is a pre-filled syringe comprising about 0.1, 0.15, 0.2, 0.25, 0.3, 0.35, 0.4, 0.45, or 0.5 mL volume of any of the immunogenic compositions or vaccines disclosed herein.

### Methods of Use

Also provided herein are methods of administering the vaccines described herein to a subject. The methods may be used to vaccinate a subject to prevent a virus infection in the subject, to decrease the subject's likelihood of getting a virus infection, or to reduce the subject's likelihood of getting serious illness from a virus infection, including, but not limited to, a virus infection caused by an influenza virus, a respiratory syncytial virus, a coronavirus, a rotavirus, a papillomavirus, a flavivirus, or a paramyxovirus. Likewise, the present disclosure provides any of the vaccine compositions described herein for use in vaccinating a subject against a virus infection. Also disclosed is use of any of the immunogenic compositions as described herein for the manufacture of a vaccine for use in vaccinating a subject against a virus infection. In some embodiments, the vaccination method or use comprises administering to a subject in need thereof an immunologically effective amount of any of the vaccines described herein.

In some embodiments, the methods may be used to vaccinate a subject to prevent an influenza virus infection in the subject, to decrease the subject's likelihood of getting an influenza virus infection, or to reduce the subject's likelihood of getting serious illness from an influenza virus infection. Likewise, the present disclosure provides any of the vaccine compositions described herein for use in vaccinating a subject against an influenza virus infection. Also disclosed is use of any of the immunogenic compositions as described herein for the manufacture of a vaccine for use in vaccinating a subject against an influenza virus infection. In some embodiments, the vaccination method or use comprises administering to a subject in need thereof an immunologically effective amount of any of the vaccines described herein.

As used herein, the term "immunologically effective amount" or "therapeutically effective amount" means an amount sufficient to immunize a subject. In some embodiments, the immunologically effective amount or therapeutically effective amount is capable of eliciting protective immunity against an infectious disease, which include, but are not limited to, an increase of antibody titers and/or T cell immunity against an infectious disease. In some embodiments, an immunologically effective amount or therapeutically effective amount of the vaccine or composition as disclosed herein increases protective immunity in a subject by about 0.1%, about 0.5%, about 1%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 99%, about 100%, including all values and subranges therebetween, when compared with a subject who is not administered with the vaccine or composition as disclosed herein.

Accordingly, in some embodiments, the disclosure provides a method of immunizing a subject comprising administering to the subject in need thereof any of the vaccines described herein. In some embodiments, the disclosure provides a method of immunizing a subject comprising administering to the subject in need thereof an immunologically effective amount any of the vaccines described herein. As used herein, "immunize" or "immunizing" means to induce in a subject a protective immune response against a virus infection, including, but not limited to an influenza virus, a respiratory syncytial virus, a coronavirus, a rotavirus, a papillomavirus, a flavivirus, or a paramyxovirus infection. Likewise, the present disclosure provides any of the vaccine compositions described herein for use in immunizing a subject against a virus infection, including, but not limited to an influenza virus, a respiratory syncytial virus, a coronavirus, a rotavirus, a papillomavirus, a flavivirus, or a paramyxovirus infection. Also disclosed is use of any of the immunogenic compositions as described herein, for the manufacture of a vaccine for use in immunizing a subject against a virus infection, including, but not limited to an influenza virus, a respiratory syncytial virus, a coronavirus, a rotavirus, a papillomavirus, a flavivirus, or a paramyxovirus infection.

In some embodiments, the method or use prevents influenza virus infection or disease caused by the influenza virus infection in the subject. In some embodiments, the method or use decreases the subject's likelihood of getting an influenza virus infection by about 0.1%, about 0.5%, about 1%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 99%, about 100%, including all values and subranges therebetween, when compared with a subject who is not administered with the vaccine or immunogenic composition as disclosed herein. In some embodiments, the method or use reduces the subject's likelihood of getting serious illness from the influenza virus infection by about 0.1%, about 0.5%, about 1%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 99%, about 100%, including all values and subranges therebetween, when compared with a subject who is not administered with the vaccine or immunogenic composition as disclosed herein. In some embodiments, the method or use raises a protective immune response in the subject. In some embodiments, the protective immune response is an antibody response.

Also provided is a method of reducing one or more symptoms of a virus infection comprising administering to a subject in need thereof any of the vaccines described herein, including, but not limited to an influenza virus, a respiratory syncytial virus, a coronavirus, a rotavirus, a papillomavirus, a flavivirus, or a paramyxovirus infection. In some embodiments, provided herein is a method of reducing one or more symptoms of a virus infection comprising administering to a subject in need thereof a prophylactically effective amount of any of the vaccines described herein, including, but not limited to an influenza virus, a respiratory syncytial virus, a coronavirus, a rotavirus, a papillomavirus, a flavivirus, or a paramyxovirus infection.

The present disclosure provides any of the vaccine compositions described herein for use in reducing one or more symptoms of a virus infection, including, but not limited to an influenza virus, a respiratory syncytial virus, a coronavirus, a rotavirus, a papillomavirus, a flavivirus, or a paramyxovirus infection. Also disclosed is any of the immunogenic compositions as described herein, for the manufacture of a vaccine for use in reducing one or more symptoms of a virus infection in a subject, including, but not limited to an influenza virus, a respiratory syncytial virus, a coronavirus, a rotavirus, a papillomavirus, a flavivirus, or a paramyxovirus infection.

Also provided, in some embodiments, is a method of reducing one or more symptoms of an influenza virus infection comprising administering to a subject in need thereof any of the vaccines described herein. In some embodiments, provided herein is a method of reducing one or more symptoms of an influenza virus infection comprising administering to a subject in need thereof a prophylactically effective amount of any of the vaccines described herein.

The present disclosure provides any of the vaccine compositions described herein for use in reducing one or more symptoms of an influenza virus infection. Also disclosed is any of the immunogenic compositions as described herein, for the manufacture of a vaccine for use in reducing one or more symptoms of an influenza virus infection in a subject.

In some embodiments, the method or use of the present disclosure reduces one or more symptoms of an influenza virus infection by about 0.1%, about 0.5%, about 1%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 99%, about 100%, including all values and subranges therebetween, when compared with a subject who is not administered with the vaccine or immunogenic composition as disclosed herein.

In some embodiments, the vaccine, and an optional adjuvant, may be administered prior to or after development of one or more symptoms of the influenza virus infection. That is, in some embodiments, the vaccines described herein may be administered prophylactically to prevent the influenza virus infection or ameliorate the symptoms of a potential influenza virus infection.

In some embodiments, the subject is at risk of infection if the subject will be in contact with other individuals or other animals known or suspected to have been infected with an influenza virus infection and/or if the subject will be present in a location in which influenza virus infection is known or thought to be prevalent or endemic. In some embodiments, the vaccine is administered to a subject suffering from an influenza virus infection, or the subject is displaying one or more symptoms commonly associated with an influenza virus infection. In some embodiments, the subject is known or believed to have been exposed to an influenza virus infection.

Vaccines in accordance with the present disclosure may be administered in any amount or dose appropriate to achieve a desired outcome. In some embodiments, the desired outcome is induction of a lasting adaptive immune response against the influenza virus. In some embodiments, the desired outcome is reduction in intensity, severity, and/or frequency, and/or delay of onset of one or more symptoms associated with influenza virus infection. The dose required may vary from subject to subject depending on the species, age, weight and general condition of the subject, the severity of the infection being treated, the particular composition being used and its mode of administration.

In some embodiments, the vaccines described herein are administered to subjects, wherein the subjects can be any member of the animal kingdom. In some embodiments, the subject is a non-human animal. In some embodiments, the non-human subject is an avian (e.g., a chicken or a bird), a reptile, an amphibian, a fish, an insect, and/or a worm. In some embodiments, the non-human subject is a mammal (e.g., a ferret, a rodent, a mouse, a rat, a rabbit, a monkey, a dog, a cat, a sheep, cattle, a primate, and/or a pig).

In some embodiments, the vaccines described herein are administered to a human subject. In some embodiments, a human subject is 6 months of age or older, 6 months through 35 months of age, at least two years of age, at least 3 years of age, 36 months through 8 years of age, 9 years of age or older, at least 6 months of age and less than 5 years of age, at least 6 months of age and less than 18 years of age, or at least 3 years of age and less than 18 years of age. In some embodiments, the human subject is an infant (less than 36 months). In some embodiments, the human subject is a child or adolescent (less than 18 years of age). In some embodiments, the human subject is a child of at least 6 months of age and less than 5 years of age. In some embodiments, the human subject is at least 5 years of age and less than 60 years of age. In some embodiments, the human subject is at least 5 years of age and less than 65 years of age. In some embodiments, the human subject is elderly (at least 60 years of age or at least 65 years of age). In some embodiments, the human subject is a non-elderly adult (at least 18 years of age and less than 65 years of age or at least 18 years of age and less than 60 years of age).

The vaccine can be administered using any suitable route of administration, including, for example, parenteral delivery, as discussed above. In some embodiments, the vaccine is administered intramuscularly, intradermally, subcutaneously, intravenously, intranasally, by inhalation, or intraperitoneally.

### Representative Embodiments of the Present Disclosure

Embodiment 1: A method for preparing at least one viral protein in a manufacturing process of a vaccine, the method comprising subjecting a suspension of virions or a suspension of host cells to a detergent comprising a compound having a CAS registry number of CAS 68131-40-8 or CAS 84133-50-6, wherein each virion comprises a lipid membrane that comprises the at least one viral protein, and wherein the host cells have been modified to express the at least one viral protein.

Embodiment 2: The method of Embodiment 1, wherein the detergent does not comprise a compound having a CAS registry number of CAS 9002-93-1 or CAS 9036-19-5.

Embodiment 3: The method of Embodiment 1 or 2, wherein the detergent consists of the compound having the CAS registry number of CAS 68131-40-8 or CAS 84133-50-6.

Embodiment 4: The method of any one of Embodiments 1-3, wherein the detergent is added to the suspension of virions or the suspension of host cells in an amount of from about 0.01 % to about 10% by volume, such as from about 0.1% to about 5% by volume or from about 0.5% to about 1% by volume.

Embodiment 5: The method of Embodiment 4, wherein the detergent is added to the suspension of virions or the suspension of host cells in an amount of about 0.5% by volume.

Embodiment 6: The method of any one of Embodiments 1-5, wherein subjecting the suspension of virions or the suspension of host cells to the detergent releases the at least one viral protein from the virions or host cells, and wherein the method further comprises obtaining a first suspension comprising the at least one viral protein released from the virions or host cells.

Embodiment 7: The method of Embodiment 6, further comprising removing the detergent from the first suspension to obtain a second suspension comprising the at least one viral protein and a trace amount of the detergent.

Embodiment 8: The method of Embodiment 7, wherein removing the detergent comprises diafiltration.

Embodiment 9: The method of Embodiment 7 or 8, further comprising purifying the at least one viral protein from the second suspension.

Embodiment 10: The method of any one of Embodiments 1-9, further comprising formulating the at least one viral protein into a vaccine, wherein the vaccine contains no more than about 250 µg/0.5 mL of the compound having the CAS registry number of CAS 68131-40-8 or CAS 84133-50-6.

Embodiment 11: The method of Embodiment 10, wherein formulating comprises combining the at least one viral protein with an adjuvant to produce the vaccine.

Embodiment 12: The method of Embodiment 10 or 11, further comprising filling the vaccine into a syringe or a vial.

Embodiment 13: The method of any one of Embodiments 10-12, wherein the vaccine is an influenza vaccine.

Embodiment 14: The method of Embodiment 13, wherein the suspension of virions is obtained by cultivating an influenza virus in an embryonated chicken egg.

Embodiment 15: The method of Embodiment 13, wherein the suspension of virions is obtained by cultivating an influenza virus in a viral propagation cell line.

Embodiment 16: The method of Embodiment 15, wherein the viral propagation cell line is a Madin-Darby Canine Kidney (MDCK) cell line.

Embodiment 17: The method of Embodiment 13, wherein the suspension of host cells have been modified to recombinantly express an influenza hemagglutinin or neuraminidase polypeptide.

Embodiment 18: The method of Embodiment 17, wherein the suspension of host cells comprises SF9 cells of *Spodoptera frugiperda* or Chinese hamster ovary (CHO) cells.

Embodiment 19: The method of Embodiment 18, wherein the at least one viral protein is expressed in the SF9 cells using a baculovirus vector.

Embodiment 20: The method of any one of Embodiments 13-19, wherein the influenza vaccine is a monovalent influenza vaccine.

Embodiment 21: The method of any one of Embodiments 13-19, wherein the influenza vaccine is a multivalent influenza vaccine, such as a trivalent, quadrivalent, pentavalent, or octavalent influenza vaccine.

Embodiment 22: The method of Embodiment 21, wherein the multivalent influenza vaccine comprises at least one viral protein obtained from an influenza A virus and at least one viral protein obtained from an influenza B virus.

Embodiment 23: The method of Embodiment 21 or 22, wherein the influenza vaccine is a trivalent influenza vaccine comprising a first viral protein obtained from an H1N1 influenza virus strain, a second viral protein obtained from an H3N2 influenza virus strain, and a third viral protein obtained from an influenza virus of a B/Victoria lineage.

Embodiment 24: The method of Embodiment 23, comprising combining and formulating the first, second, and third viral proteins into the trivalent influenza vaccine.

Embodiment 25: The method of Embodiment 23 or 24, wherein the first, second, and third viral proteins comprise influenza hemagglutinin proteins.

Embodiment 26: The method of any one of Embodiments 23-25, wherein each of the first, second, and third viral proteins is an influenza hemagglutinin protein present in an amount between about 5 µg to about 60 µg per 0.5 mL dose of the vaccine.

Embodiment 27: The method of Embodiment 21 or 22, wherein the influenza vaccine is a quadrivalent influenza vaccine comprising a first viral protein obtained from an H1N1 influenza virus strain, a second viral protein obtained from an H3N2 influenza virus strain, a third viral protein obtained from an influenza virus of a B/Victoria lineage, and a fourth viral protein obtained from an influenza virus of a B/Yamagata lineage.

Embodiment 28: The method of Embodiment 27, comprising combining and formulating the first, second, third, and fourth viral proteins into the quadrivalent influenza vaccine.

Embodiment 29: The method of Embodiment 27 or 28, wherein the first, second, third, and fourth viral proteins comprise influenza hemagglutinin proteins.

Embodiment 30: The method of any one of Embodiments 27-29, wherein the amount of influenza hemagglutinin protein in each of the first, second, third, and fourth viral proteins is between about 5 µg to about 60 µg per 0.5 mL dose of the vaccine.

Embodiment 31: The method of any one of Embodiments 10-30, wherein the vaccine further comprises sodium phosphate buffered isotonic sodium chloride and optionally a preservative.

Embodiment 32: The method of Embodiment 31, wherein the preservative is mercury or a mercury derivative.

Embodiment 33: The method of Embodiment 32, wherein the mercury derivative is thimerosal.

Embodiment 34: The method of any one of Embodiments 10-33, wherein the vaccine further comprises formaldehyde at a concentration of no more than about 100 µg/0.5 mL.

Embodiment 35: A vaccine manufactured by the method of any one of Embodiments 10-34.

Embodiment 36: An immunogenic composition comprising an inactivated virus or at least one viral protein and a trace amount of a detergent comprising a compound having a CAS registry number of CAS 68131-40-8 or CAS 84133-50-6.

Embodiment 37: The immunogenic composition of Embodiment 36, wherein the detergent does not comprise a compound having a CAS registry number of CAS 9002-93-1 or CAS 9036-19-5.

Embodiment 38: The immunogenic composition of Embodiment 36 or 37, wherein the detergent consists of the compound having the CAS registry number of CAS 68131-40-8 or CAS 84133-50-6.

Embodiment 39: The immunogenic composition of any one of Embodiments 36-38, wherein the inactivated virus is an influenza A virus.

Embodiment 40: The immunogenic composition of Embodiment 39, wherein the influenza A virus is an H1N1 influenza virus strain.

Embodiment 41: The immunogenic composition of Embodiment 39, wherein the influenza A virus is an H3N2 influenza virus strain.

Embodiment 42: The immunogenic composition of any one of Embodiments 36-41, wherein the inactivated virus is an influenza B virus.

Embodiment 43: The immunogenic composition of Embodiment 42, wherein the influenza B virus is a B/Victoria lineage.

Embodiment 44: The immunogenic composition of Embodiment 42, wherein the influenza B virus is a B/Yamagata lineage.

Embodiment 45: The immunogenic composition of any one of Embodiments 36-44, wherein the at least one viral protein is an influenza hemagglutinin protein and/or an influenza neuraminidase protein.

Embodiment 46: The immunogenic composition of any one of Embodiments 36-45, wherein the at least one viral protein is a recombinant influenza structural protein, optionally wherein the recombinant influenza structural protein is expressed in SF9 cells of *Spodoptera frugiperda* or CHO cells, and optionally wherein the recombinant influenza structural protein is expressed in the SF9 cells using a baculovirus vector.

Embodiment 47: The immunogenic composition of Embodiment 46, wherein the recombinant influenza structural protein is a recombinant influenza hemagglutinin protein and/or a recombinant influenza neuraminidase protein.

Embodiment 48: The immunogenic composition of any one of Embodiments 36-47, comprising at least one viral protein from an influenza A virus and at least one viral protein from an influenza B virus.

Embodiment 49: The immunogenic composition of Embodiment 48, comprising a first viral protein from an HIN1 influenza virus strain, a second viral protein from an H3N2 influenza virus strain, and a third viral protein from an influenza virus of a B/Victoria lineage.

Embodiment 50: The immunogenic composition of Embodiment 49, wherein the first, second, and third viral proteins are influenza hemagglutinin proteins and/or influenza neuraminidase proteins.

Embodiment 51: The immunogenic composition of Embodiment 50, wherein each of the first, second, and third viral proteins is an influenza hemagglutinin protein present in an amount of between about 5 µg to about 60 µg per 0.5 mL dose of the immunogenic composition.

Embodiment 52: The immunogenic composition of Embodiment 48, comprising a first viral protein from an H1N1 influenza virus strain, a second viral protein from an H3N2 influenza virus strain, a third viral protein from an influenza virus of a B/Victoria lineage, and a fourth viral protein from an influenza virus of a B/Yamagata lineage.

Embodiment 53: The immunogenic composition of Embodiment 52, wherein the first, second, third, and fourth viral proteins are influenza hemagglutinin proteins and/or influenza neuraminidase proteins.

Embodiment 54: The immunogenic composition of Embodiment 53, wherein each of the first, second, third, and fourth viral proteins is an influenza hemagglutinin protein present in an amount of between about 5 µg to about 60 µg per 0.5 mL dose of the immunogenic composition.

Embodiment 55: The immunogenic composition of any one of Embodiments 36-54, further comprising an adjuvant.

Embodiment 56: A vaccine comprising the immunogenic composition of any one of Embodiments 36-52 and a pharmaceutically acceptable carrier.

Embodiment 57: The vaccine of Embodiment 56, wherein the vaccine comprises at least one influenza virus hemagglutinin protein.

Embodiment 58: The vaccine of Embodiment 56 or 57, wherein the vaccine comprises no more than about 250 µg/0.5 mL of the compound having the CAS registry number of CAS 68131-40-8 or CAS 84133-50-6.

Embodiment 59: The vaccine of any one of Embodiments 56-58, further comprising sodium phosphate buffered isotonic sodium chloride and optionally a preservative.

Embodiment 60: The vaccine of Embodiment 59, wherein the preservative is mercury or a mercury derivative.

Embodiment 61: The vaccine of Embodiment 60, wherein the mercury derivative is thimerosal.

Embodiment 62: The vaccine of any one of Embodiments 56-61, further comprising formaldehyde at a concentration of no more than about 100 µg/0.5 mL.

Embodiment 63: The vaccine of any one of Embodiments 56-62, wherein the vaccine is a live attenuated virus vaccine, an inactivated whole virus vaccine, a virosome vaccine, a split-virion vaccine, a subunit vaccine, or a recombinant vaccine.

Embodiment 64: A vaccine comprising: about 5-60 µg/0.5 mL of an influenza hemagglutinin antigen from each of an H1N1 influenza virus strain, an H3N2 influenza virus strain, and an influenza virus of a B/Victoria lineage; a sodium phosphate-buffered isotonic sodium chloride solution; no more than about 30-100 µg/0.5 mL of formaldehyde; no more than about 250 µg/0.5 mL of a compound having a CAS registry number of CAS 68131-40-8 or CAS 84133-50-6; and optionally a preservative.

Embodiment 65: The vaccine of Embodiment 64, wherein the vaccine does not comprise a compound having a CAS registry number of CAS 9002-93-1 or CAS 9036-19-5.

Embodiment 66: The vaccine of Embodiment 64 or 65, wherein the preservative comprises thimerosal in an amount of about 2 µg/0.5 mL or mercury in an amount of about 20 µg/0.5 mL.

Embodiment 67: The vaccine of any one of Embodiments 64-66, comprising about 15 µg/0.5 mL of the influenza hemagglutinin antigen from each of an H1N1 influenza virus strain, an H3N2 influenza virus strain, and an influenza virus of a B/Victoria lineage, and no more than about 30 µg/0.5 mL of formaldehyde.

Embodiment 68: The vaccine of Embodiment 67, further comprising thimerosal as the preservative in an amount of about 2 µg/0.5 mL.

Embodiment 69: The vaccine of any one of Embodiments 64-66, comprising about 15 µg/0.5 mL of an influenza hemagglutinin antigen from each of an H1N1 influenza virus strain, an H3N2 influenza virus strain, and an influenza virus of a B/Victoria lineage, and no more than about 100 µg/0.5 mL of formaldehyde, and wherein the preservative comprises mercury in an amount of about 25 µg/0.5 mL.

Embodiment 70: A vaccine comprising: about 5-60 µg/0.5 mL of an influenza hemagglutinin antigen from each of an H1N1 influenza virus strain, an H3N2 influenza virus strain, an influenza virus of a B/Victoria lineage, and an influenza virus of a B/Yamagata lineage;

a sodium phosphate-buffered isotonic sodium chloride solution; no more than about 100 µg/0.5 mL of formaldehyde; no more than about 250 µg/0.5 mL of a compound having a CAS registry number of CAS 68131-40-8 or CAS 84133-50-6; and optionally a preservative.

Embodiment 71: The vaccine of Embodiment 70, wherein the vaccine does not comprise a compound having a CAS registry number of CAS 9002-93-1 or CAS 9036-19-5.

Embodiment 72: The vaccine of Embodiment 70 or 71, wherein the vaccine comprises no more than about 30 µg/0.5 mL of formaldehyde.

Embodiment 73: The vaccine of any one of Embodiments 70-72, wherein the preservative is mercury in an amount of about 25 µg/0.5 mL.

Embodiment 74: A vaccine comprising: about 5-60 µg/0.5 mL of an influenza hemagglutinin antigen from each of an H1N1 influenza virus strain, an H3N2 influenza virus strain, an influenza virus of a B/Victoria lineage, and an influenza virus of a B/Yamagata lineage;

a buffer solution comprising sodium chloride, potassium chloride, dibasic sodium phosphate dihydrate, and monobasic potassium phosphate; no more than about 30 µg/0.5 mL of formaldehyde; and no more than about 250 µg/0.5 mL of a compound having a CAS registry number of CAS 68131-40-8 or CAS 84133-50-6.

Embodiment 75: The vaccine of Embodiment 74, wherein the vaccine does not comprise a compound having a CAS registry number of CAS 9002-93-1 or CAS 9036-19-5.

Embodiment 76: The vaccine of Embodiment 74 or 75, wherein the vaccine does not comprise an adjuvant or a preservative.

Embodiment 77: A vaccine comprising: 45 µg/0.5 mL of a recombinant influenza hemagglutinin antigen from each of an H1N1 influenza virus strain, an H3N2 influenza virus strain, and an influenza virus of a B/Victoria lineage; sodium chloride; monobasic sodium phosphate; dibasic sodium phosphate; polysorbate 20; and no more than about 100 µg/0.5 mL of a compound having a CAS registry number of CAS 68131-40-8 or CAS 84133-50-6.

Embodiment 78: The vaccine of Embodiment 77, wherein the vaccine does not comprise a compound having a CAS registry number of CAS 9002-93-1 or CAS 9036-19-5.

Embodiment 79: The vaccine of Embodiment 77 or 78, comprising: about 4.4 mg/0.5 mL sodium chloride; about 0.195 mg/0.5 mL monobasic sodium phosphate; about 1.3 mg/0.5 mL dibasic sodium phosphate; and about 27.5 µg/0.5 mL polysorbate 20.

Embodiment 80: The vaccine of Embodiment 77 or 78, comprising: about 4.4 mg/0.5 mL sodium chloride; about 0.2 mg/0.5 mL monobasic sodium phosphate; about 0.5 mg/0.5 mL dibasic sodium phosphate; and about 27.5 µg/0.5 mL polysorbate 20.

Embodiment 81: The vaccine of any one of Embodiments 77-80, wherein the vaccine does not comprise a preservative.

Embodiment 82: A vaccine comprising: 45 µg/0.5 mL of a recombinant influenza hemagglutinin antigen from each of an H1N1 influenza virus strain, an H3N2 influenza virus strain, an influenza virus of a B/Victoria lineage, and an influenza virus of a B/Yamagata lineage; sodium chloride; monobasic sodium phosphate; dibasic sodium phosphate; polysorbate 20; and no more than about 100 µg/0.5 mL of a compound having a CAS registry number of CAS 68131-40-8 or CAS 84133-50-6.

Embodiment 83: The vaccine of Embodiment 82, wherein the vaccine does not comprise a compound having a CAS registry number of CAS 9002-93-1 or CAS 9036-19-5.

Embodiment 84: The vaccine of Embodiment 82 or 83, comprising: about 4.4 mg/0.5 mL sodium chloride; about 0.195 mg/0.5 mL monobasic sodium phosphate; about 1.3 mg/0.5 mL dibasic sodium phosphate; and about 27.5 µg/0.5 mL polysorbate 20.

Embodiment 85: The vaccine of Embodiment 82 or 83, comprising: about 4.4 mg/0.5 mL sodium chloride; about 0.2 mg/0.5 mL monobasic sodium phosphate; about 0.5 mg/0.5 mL dibasic sodium phosphate; and about 27.5 µg/0.5 mL polysorbate 20.

Embodiment 86: The vaccine of any one of Embodiments 82-85, wherein the vaccine does not comprise a preservative.

Embodiment 87: The vaccine of any one of Embodiments 56-86, wherein the serum hemagglutinin inhibition (HAI) titer induced by the vaccine is the same or higher than the serum HAI titer induced by a control vaccine that is identical to the vaccine of any one of Embodiments 56-86 except that the trace amount of the detergent comprising the compound having the CAS registry number of CAS 68131-40-8 or CAS 84133-50-6 is replaced with a trace amount of a detergent comprising a compound having a CAS registry number of CAS 9002-93-1 or CAS 9036-19-5.

Embodiment 88: The vaccine of any one of Embodiments 56-86, wherein: the vaccine induces a serum hemagglutinin inhibition (HAI) titer of at least 40, wherein the HAI titer is measured at 20 days following vaccination; and/or the vaccine induces a serum HAI that increases at least 4-fold as compared to the serum HAI titer prior to vaccination, wherein the HAI titer is measured at 20 days following vaccination.

Embodiment 89: A method of immunizing a subject, the method comprising administering to the subject in need thereof the vaccine of Embodiment 35, the immunogenic composition of any one of Embodiments 36-55, or the vaccine of any one of Embodiments 56-88.

Embodiment 90: The method of Embodiment 89, wherein the method prevents an influenza virus infection in the subject, decreases the subject's likelihood of getting an influenza virus infection, or reduces the subject's likelihood of getting serious illness from an influenza virus infection.

Embodiment 91: The method of Embodiment 89 or 90, wherein the method raises a protective immune response in the subject.

Embodiment 92: The method of any one of Embodiments 89-91, wherein the subject is a human.

Embodiment 93: The method of Embodiment 92, wherein the human is 6 months of age or older, less than 18 years of age, at least 6 months of age and less than 18 years of age, at least 18 years of age and less than 65 years of age, at least 6 months of age and less than 5 years of age, at least 5 years of age and less than 65 years of age, at least 60 years of age, or at least 65 years of age.

Embodiment 94: The method of any one of Embodiments 89-93, wherein the viral vaccine, the immunogenic composition or the vaccine is administered intramuscularly, intradermally, subcutaneously, intravenously, intranasally, by inhalation, or intraperitoneally.

Embodiment 95: A method of reducing one or more symptoms of an influenza virus infection, the method comprising administering to a subject in need thereof the vaccine of Embodiment 35, the immunogenic composition of any one of Embodiments 36-55, or the vaccine of any one of Embodiments 56-88.

Embodiment 96: The method of any one of Embodiments 89-95, wherein the method treats or prevents disease caused by either or both a seasonal and a pandemic influenza virus strain.

Embodiment 97: A method of reducing one or more symptoms caused by an influenza virus infection, the method comprising administering to a subject in need thereof the viral vaccine of Embodiment 35, the immunogenic composition of any one of Embodiments 36-55, or the vaccine of any one of Embodiments 56-88.

### EXAMPLES

The following examples are to be considered illustrative and not limiting on the scope of the present disclosure described above.

### Example 1. Evaluation of Detergents as Alternatives to Triton X-100

This example describes the evaluation of various detergents for their potential of being an alternative to Triton X-100 in influenza virus splitting process. The aim is to identify alternative detergents with influenza virus splitting efficiency comparable to that of Triton X-100.

Triton X-100 is a non-ionic surfactant and commonly used with viral vaccines at different stages of the manufacturing process. For example, Triton X-100 is used in the current influenza split virion vaccine VAXIGRIP^{®} manufactured by Sanofi as the splitting agent to extract and purify the influenza hemagglutinin (HA) antigen from the virus particle. It also contributes to the maintenance of suspended antigens in the final formulation by reducing the risk of protein aggregation. Triton X-100 also contributes to the inactivation and/or removal of adventitious agents, such as bacteria, viruses (including influenza virus), fungi, and mycoplasma, in the manufacturing process. Thus, Triton X-100 is considered to be an important reagent in the viral vaccine production process.

However, octylphenol, a degradation product of Triton X-100, can function as an endocrine disruptor with estrogen-mimetic activity in water with the potential to negatively impact aquatic wildlife. Triton X-100 is currently banned by European regulation REACH (Registration, Evaluation, Authorization and Restriction of Chemicals).

To this end, nine detergents listed in **Table 1** were used for screening with Triton X-100 being used as a positive control and detergent-free as a negative control.

**Table 1. List of detergents evaluated.**

| **Detergent** | **Type** | **CAS No.** | **Target Concentration (v/v)** |
|---|---|---|---|
| CALX133ACE | Calixaren-based | NA | 0.5% |
| Sulfobetan 3-14 | Zwitterionic | CAS 14933-09-6 | 0.1% |
| Ecosurf EH-9¹ | Nonionic | CAS 64366-70-7 | 0.5% |
| CHAPs | Zwitterionic | CAS 331717-45-4 | 0.5% |
| LDAO | Zwitterionic | CAS 1643-20-5 | 0.5% |
| OBDG² | Nonionic | CAS 29836-26-8 | 0.5% |
| Tergitol 15S9³ | Nonionic | CAS 68131-40-8 | 0.5% |
| TDAO | Zwitterionic | CAS 3332-27-2 | 1.0% |
| Triton X-100⁴ | Nonionic | CAS 9002-93-1 | 0.5% |

| | | | |
|---|---|---|---|
| 1: Ecosurf EH-9 and Ecosurf EH9 are used interchangeably herein. 2: OBDG = Octyl beta-D-glycopyranoside. 3: Tergitol 15S9, Tergitol 15-S9, and Tergitol 15-S-9 are used interchangeably herein. 4: Triton X-100 and Triton X100 are used interchangeably herein. | | | |

The detergents provided in **Table 1** were evaluated in a splitting procedure involving the A/H1N1/Michigan/45/15 X-275 (H1N1) virus strain grown in embryonated eggs. Specifically, detergents were added to viral samples to reach the target concentration (v/v) provided in **Table 1** and incubated at 22°C for 1 hour under magnetic stirring (225 rpm). Triton X-100 was added as a positive control, while one sample was kept detergent-free and used as a negative control. Various samples were taken before, during, and after the splitting procedure and were analyzed for their respective HA content, protein content, protein profile, particle size, morphological characteristics, and infectious titer. More specifically, Sample E0 was obtained before splitting, Sample E1 was obtained following detergent incubation, and Sample E2 was obtained following removal of "viral cores" not split by pelleting.

### HA and Protein Contents

The HA content was analyzed by the single radial immunodiffusion (SRID) assay, which measures the concentration of HA by virtue of its reaction with a specific antibody to produce precipitin rings in an agarose gel. The SRID assay was used to monitor the HA levels in the samples and any alternative detergent having a higher SRID titer than that of Triton X-100 was considered to be a potential alternative to Triton X-100.

The protein content was used for assessing purity. Two analytical assays were used to measure the protein content. The first one is a chemiluminescence assay for determining the protein content in samples obtained from the fractions treated with detergents containing no nitrogen in the chemical structure. A colorimetric assay was used to determine the protein content in samples obtained from the fractions treated with nitrogen-containing detergents. A preliminary testing using Triton X-100 as the splitting agent showed that these two analytical assays produced comparable results in measuring the protein content (data not shown). The ratio of SRID titer to protein content was then used as a selection criterion and any alternative detergent having the SRID/protein ratio similar to that of Triton X-100 was considered to be a potential alternative to Triton X-100.

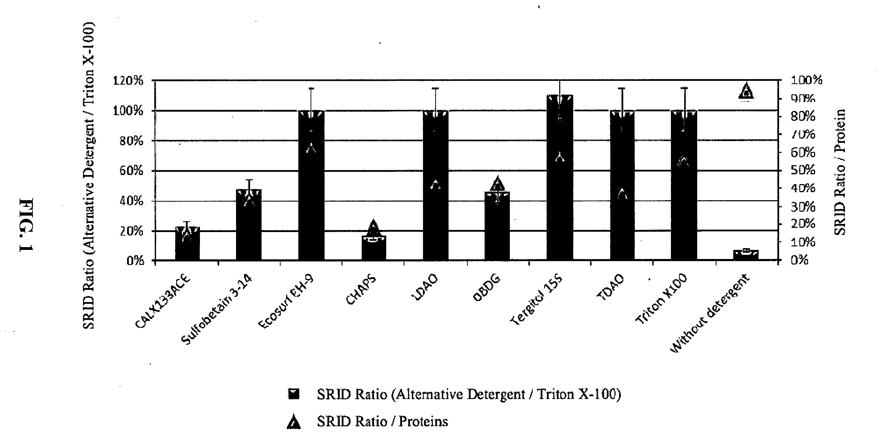

**FIG. 1** shows the splitting efficiency of the detergents based on these two criteria. The SRID titer of each alternative detergent was normalized to the SRID titer obtained with Triton X-100 and is represented in **FIG. 1** as "SRID Ratio (Alternative Detergent / Triton X-100)." The ratio of SRID titer to protein content of each alternative detergent is represented in **FIG. 1** as "SRID Ratio / Protein." The low SRID titer obtained from the negative control ("Without detergent" in **FIG. 1****),** which is only about 6% of the value of the "Triton XI00" control, demonstrates that the HA quantifications of the alternative detergents tested are related to the splitting action of the detergents.

As shown in **FIG. 1****,** four alternative detergents have their respective SRID titer equivalent to that of the positive control "Triton X100": Ecosurf EH-9, Tergitol 15S9 (marked as "Tergitol 15S" in **FIG. 1****),** LDAO, and TDAO. Ecosurf EH-9 and Tergitol 15S9 also have their respective SRID/Protein ratio that is closest to the value of 55% obtained from the positive control "Triton X100" (63% and 58%, respectively).

### Particle Size

The particle size in the samples collected was determined using dynamic light scattering (DLS). The DLS analysis was used to determine the distribution of particle size and any alternative detergent that reduces the particle size distribution after splitting was considered to be a potential alternative to Triton X-100.

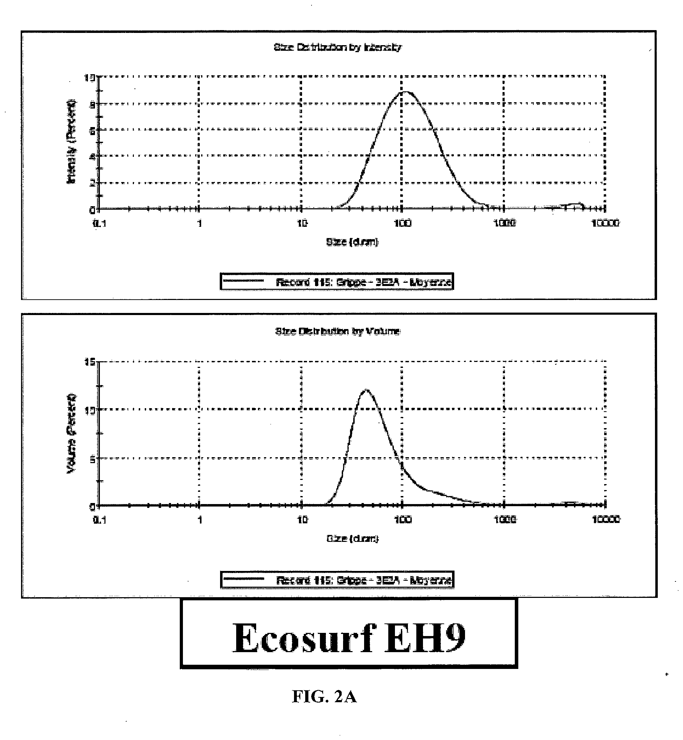

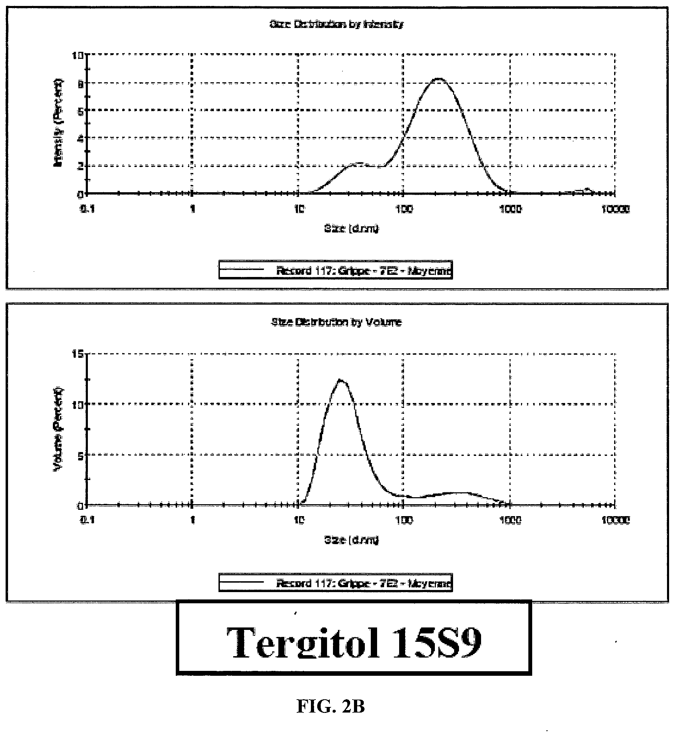

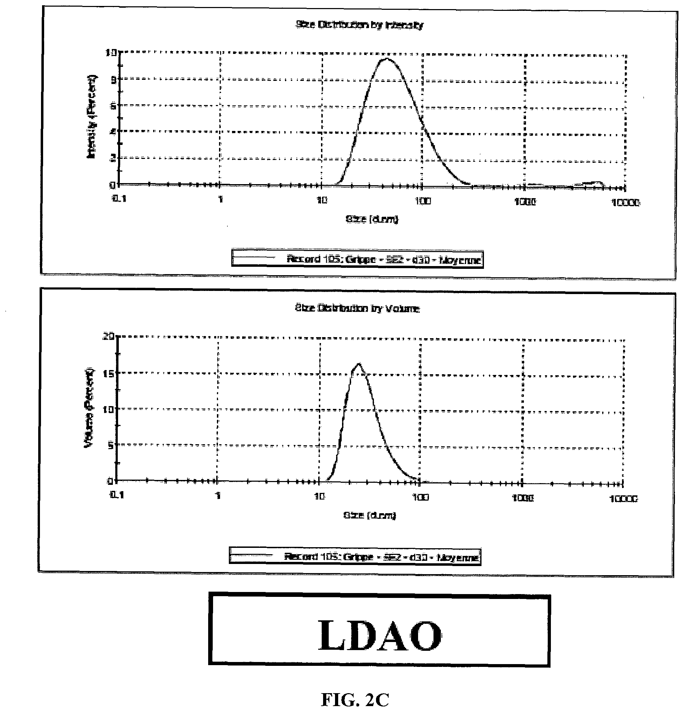

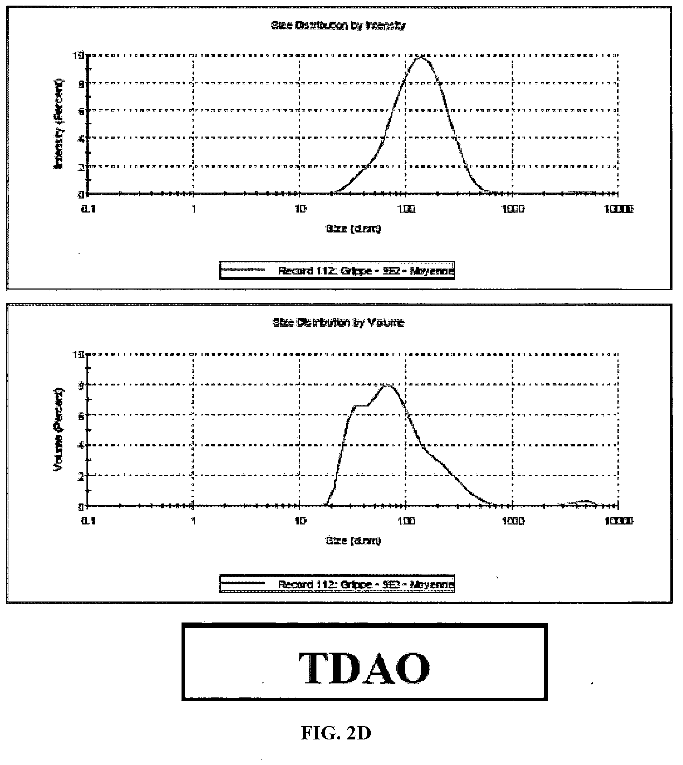

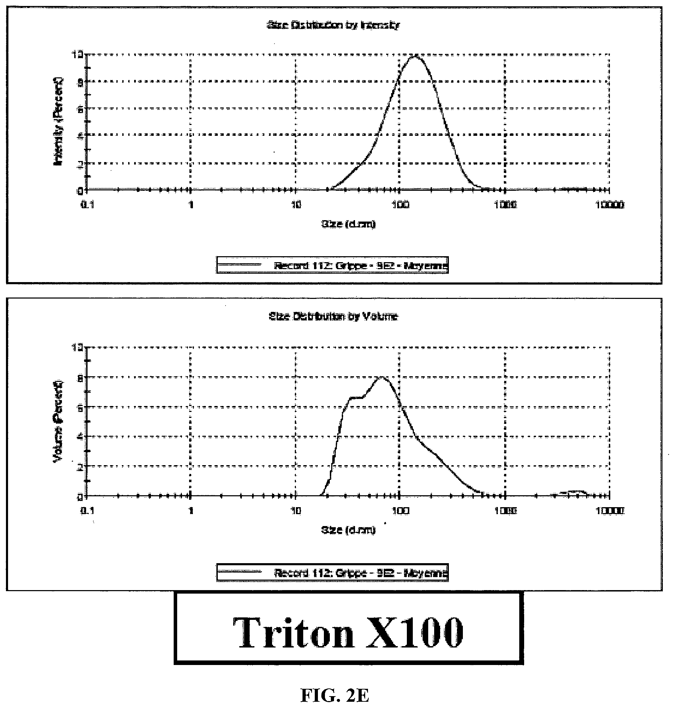

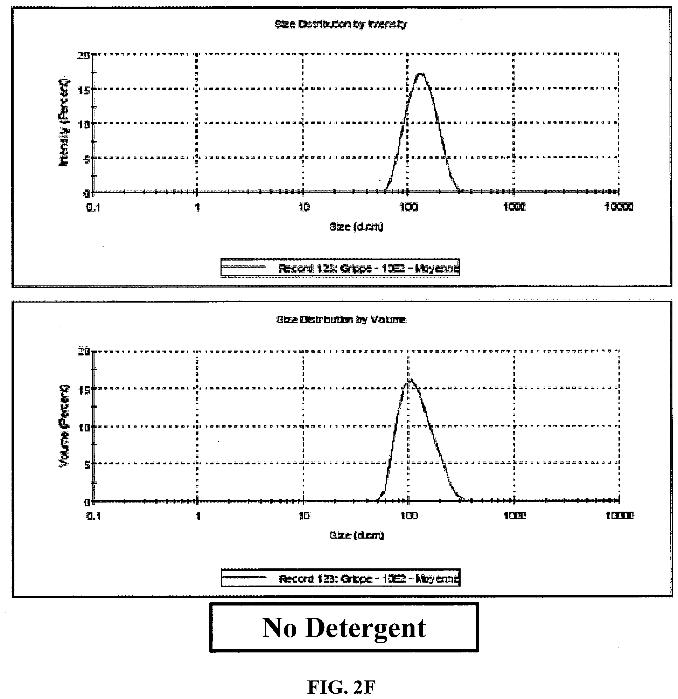

The particle size distribution in Sample E2 obtained from the four alternative detergents identified as having a SRID titer equivalent to that of the positive control (i.e., Ecosurf EH-9, Tergitol 15S9, LDAO, and TDAO) was determined using DLS and their respective DLS profiles (intensity and volume) are shown in **FIG. 2A-2D****.** The DLS profiles (intensity and volume) of the two controls ("Triton X100" and "No Detergent") are shown in **FIG. 2E-2F****.** As shown in **FIG. 2A-2D****,** each of these alternative detergents reduced the particle size distribution after splitting as compared to the "No Detergent" control **(****FIG. 2F****).**

### Protein Profile

The protein profile of the samples collected was analyzed using SDS-PAGE under denatured/non-reducing conditions and any alternative detergent produces the same protein bands (both size and intensity) as the "Triton X100" control was considered to be a potential alternative to Triton X-100.

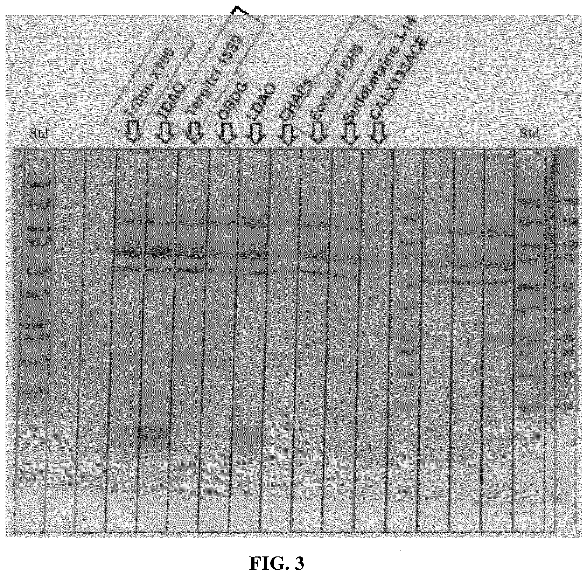

The SDS-PAGE analysis of Sample E2 obtained from all the alternative detergents tested is shown in **FIG. 3****.** Based on the size and intensity of the observed bands on the SDS-PAGE, Tergitol 15S9 and Ecosurf EH9 were identified as comparable alternatives to Triton X-100. The majority of the proteins visible on the SDS-PAGE are expected to be HA (70 kDa, 140 kDa and > 210 kDa) and NA (about 55kDa).

### Morphological Characteristics

The morphological characteristics of the particles in the samples collected were analyzed using transmission electron microscopy (TEM). This additional analytical method makes it possible to visualize the different structures in the post-splitting samples. Any alternative detergent that produces the same visible population type as the "Triton X100" control is considered to be a potential alternative to Triton X-100.

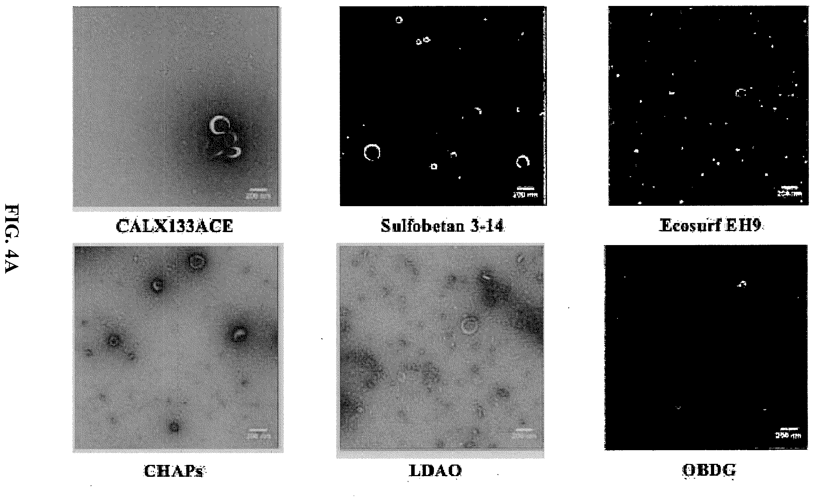

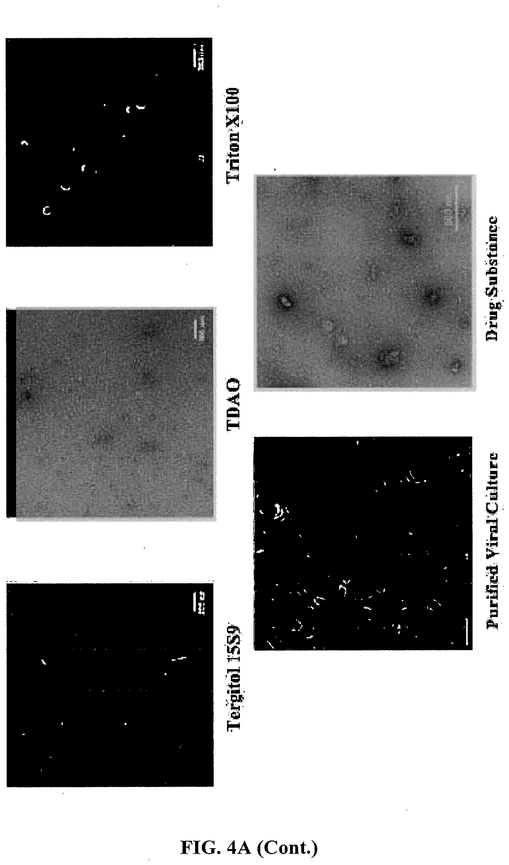

The TEM images obtained from Sample E2, as well as the TEM images of the purified virus culture prior to splitting ("Purified Virus Culture") and the subsequent purified drug substance ("Drug Substance"), are shown in **FIG. 4A****.** As shown in **FIG. 4A****,** all the alternative detergents tested were able to reduce the amount of the whole viruses in the post-splitting sample in favor of proteins and wrap/flat particles as compared to the sample obtained prior to the splitting procedure ("Purified Virus Culture").

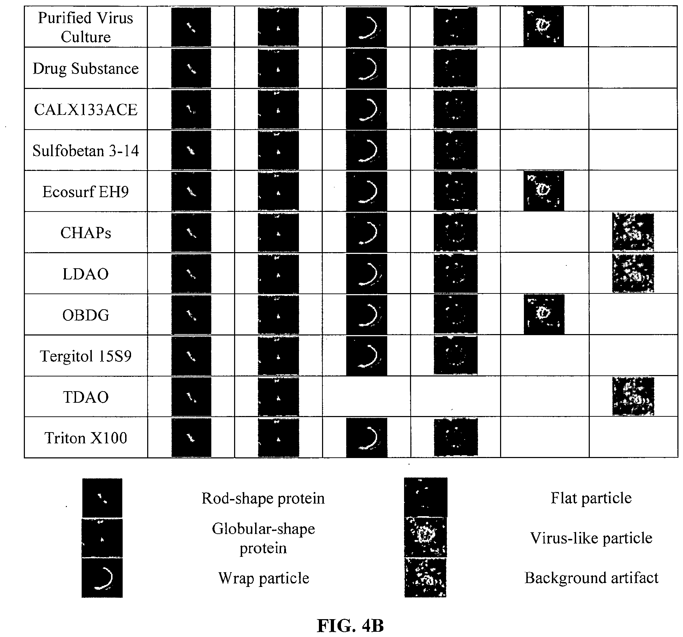

The morphological characteristics of the particles observed in these TEM images are summarized in **FIG. 4B****.** Among all the alternative detergents tested, several detergents, including Tergitol 15S9, produced the same morphological characteristic patterns as the "Triton X100" control and the subsequent purified drug substance ("Drug Substance"). On the other hand, several detergents, including Ecosurf EH-9, resulted in undesirable virus-like particles.

### Infectious Titer

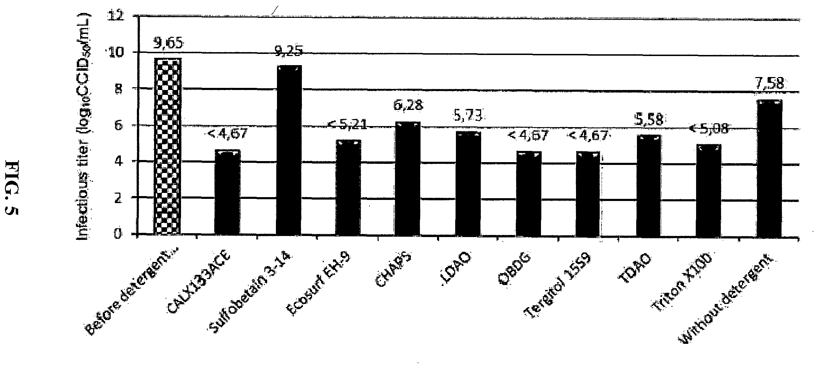

The impact of the detergents on the infectious titer was evaluated by cell culture infectious dose 50% (CCID₅₀) assay. As shown in **FIG. 5****,** except for Sulfobetan 3-14 and CHAPs, all the other alternative detergents tested were able to reduce the infectious titer by more than about 4 log₁₀CCID₅₀/mL under the splitting conditions used in this screening study.

### Example 2. Assessing the Splitting Conditions for Tergitol 15S9

At the end of the screening study described in Example 1, based on all the data collected, Tergitol 15S9 was selected for further evaluation. Several parameters are likely to have an impact on the efficacy of detergents during downstream processes, such as pH, conductivity, concentration, etc. This example describes assessment of the splitting conditions for Tergitol 15S9 in terms of the detergent concentration and conductivity. Two different splitting time periods were also applied to identify any potential impact on the splitting efficiency.

The experiments were designed to vary the detergent concentration and conductivity over 3 levels as shown in **Table 2**. Conductivity was adjusted by adding NaCl. In order to avoid the dilution effects related to the NaCl addition, equal volumes of stock solutions (see **Table 2**) were added for each of the levels studied.

**Table 2. Definition of the levels studied in assessing the splitting conditions for Tergitol 15S9.**

| **Settings** | **Level 1** | **Level 2** | **Level 3** |
|---|---|---|---|
| Conductivity (adjusted by adding NaCl) | 0 mM NaCl | 250 mM NaCl | 500 mM NaCl |
| % detergent (m/v) | 0.1% | 0.55% | 1% |
| Stock solution | 3% sucrose in PBS | 1.92 M NaCl prepared with 3% sucrose in PBS | 3.85 M NaCl prepared with 3% sucrose in PBS |

The splitting studies were performed according to the splitting procedure described in Example 1. The various conditions studied are summarized in **Table 3**. A control containing 0.5% (m/v) Triton X-100 and 0 mM NaCl was used to compare splitting efficiency. Splitting conditions are defined by 2 criteria: (1) a higher SRID titer than that of the Triton X-100 control (about 1000 pg/mL), and (2) same SDS-PAGE profile as the Triton X-100 control, both determined in Samples E2 as described in Example 1.

**Table 3. Summary of the various conditions evaluated in assessing the splitting conditions for Tergitol 15S9.**

| **Conditions** | **1** | **2** | **3** | **4** | **5** | **6** |
|---|---|---|---|---|---|---|
| % detergent (m/v) | 0.55 | 1 | 0.55 | 0.55 | 1 | 0.55 |
| NaCl conc. (mM) | 250 | 0 | 0 | 500 | 500 | 250 |
| Buffer | 3% sucrose in PBS | | | | | |
| Duration | 30 minutes or 90 minutes | | | | | |
| Temperature | 22°C | | | | | |

| **Conditions** | **7** | **8** | **9** | **10** | **11** | **Triton X-100 control** |
|---|---|---|---|---|---|---|
| % detergent (m/v) | 0.1 | 0.55 | 0.1 | 0.1 | 1 | 0.5 |
| [NaCl] (mM) | 250 | 250 | 500 | 0 | 250 | 0 |
| Buffer | 3% sucrose in PBS | | | | | |
| Duration | 30 minutes or 90 minutes | | | | | |
| Temperature | 22°C | | | | | |

The results obtained from both incubation time periods (i.e., 30 and 90 minutes) were similar and thus, only those obtained from the 90-minute incubation time are described here.

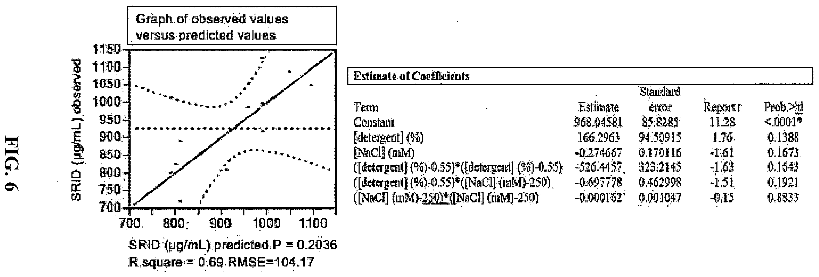

The statistical analysis of the SRID data using the JMP software globally centered the SRID titer around 900 pg/mL, resulting in the generation of a SRID statistical model describing the influential parameters and their optimum (simple trend because the *p* value > 0.05). **FIG. 6** shows this SRID statistical model as well as the estimate of the associated coefficients.

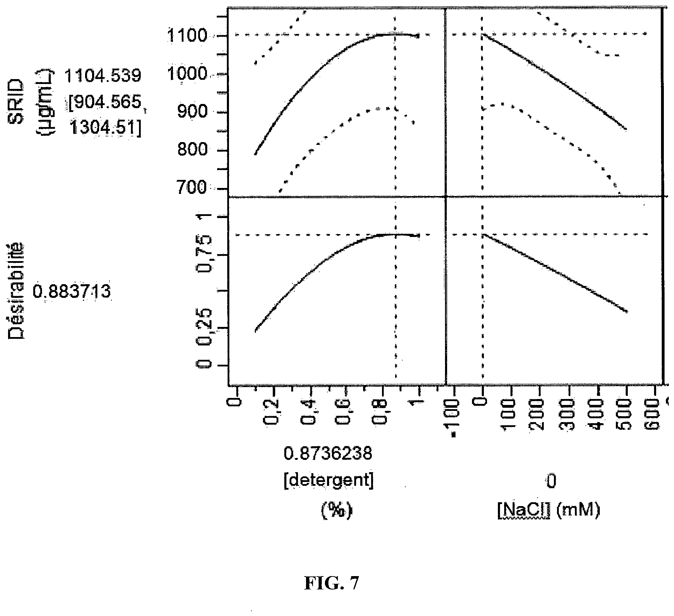

The Prediction Profiler of the JMP software makes it possible to define the conditions required to obtain an enhanced SRID titer regardless of the incubation time (30 minutes vs 90 minutes). The graphic generated by the Prediction Profiler based on the data obtained from the 90-minute incubation time is shown in **FIG. 7****.** Based on these results, the optimal concentration for Tergitol 15S9 is about 0.7% (v/v) (or 0.87% (v/v) specifically based on the data shown in **FIG. 7****)** and the optimal conductivity is 0 mM of NaCl. More generally, a concentration of more than 0.45% (v/v) of Tergitol 15S9 can achieve a SRID titer more than 1000 µg/mL.

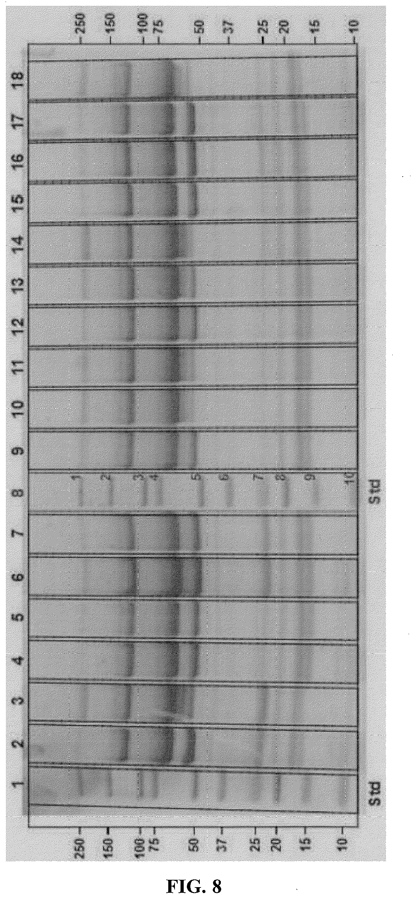

The protein profiles by SDS-PAGE under denaturant/non-reducing conditions from 4 different batches of testing are shown in **FIG. 8****.** The samples loaded onto the SDS-PAGE gel are summarized in **Table 4.** As shown in **FIG. 8****,** the splitting conditions [Tergitol 15S9] = 0.5% / [NaCl] = 0mM (Lanes 5 and 7) generated the protein profiles that are closest to the protein profiles generated by Triton X-100 ([Triton X-100] = 0.5% / [NaCl] = 0mM; Lanes 2 and 6).

**Table 4. Summary of the samples loaded onto the SDS-PAGE gel shown in FIG. 8.**

| **Batch** | **Lane** | **Detergent** | **Conditions** | **Splitting time** |
|---|---|---|---|---|
| 1 | 2 | Triton X-100 | 0.5% - 60 minutes | |
| | 3 | Tergitol 15S9 | 0.7% - 60 minutes | |
| 2 | 4 | Triton X-100 | 0.5% - 60 minutes | |
| | 5 | Tergitol 15S9 | | |
| 3 | 6 | Triton X-100 | 0.5% - 60 minutes | |
| | 7 | Tergitol 15S9 | | |
| 4 | 9 | Tergitol 15S9 | 1* | 90 minutes |
| | 10 | | 2* | |
| | 11 | | 2* | 30 minutes |
| | 12 | | 3* | 90 minutes |
| | 13 | | 4* | |
| | 14 | | 5* | |
| | 15 | | 7* | |
| | 16 | | 9* | |
| | 17 | | 10* | |
| | 18 | | 11* | |

| | | | | |
|---|---|---|---|---|
| *: Condition as described in **Table 3.** | | | | |

Based on this study, it was concluded that a maximum SRID titer can be achieved when Tergitol 15S9 is used at a concentration of 0.7% (v/v) without conductivity adjustment (i.e., 0 mM NaCl) for 30 to 90 minutes of splitting time. However, in order to maintain a post-splitting SRID titer above 1000 µg/mL and a protein profile that is comparable to that of the Triton X-100 control, it was concluded that the following splitting conditions are preferred for the production of drug substance for *in vivo* studies in mice: 0 mM NaCl (i.e., no conductivity adjustment), 0.5% (v/v) Tergitol 15S9, and 60 minutes incubation time. It is expected that similar results and conditions will be obtained for DEVIRON^{™} 13-S9 (Deviron 13S9).

### Example 3. Evaluation of Downstream Process for Production of Drug Substance

This Example describes the evaluation of a downstream process for producing influenza monovalent drug substance using Tergitol 15S9 as the splitting agent in lieu of Triton X-100.

Briefly, the A/H1N1/Michigan/45/15 X-275 (H1N1) virus strain grown in embryonated eggs was used for the evaluation. Various samples were taken at different steps of the downstream process and were analyzed using the analytical methods summarized in **Table 5.** For comparison, a reference batch of drug substance was prepared using Triton X-100 as the splitting agent ("Triton X-100 control"). More specifically, Sample E0 was obtained before splitting, Sample E1 was obtained following addition of 0.5% (v/v) of Tergitol 15S9. and incubation at 22°C for 1 hour under magnetic stirring (225 rpm). Samples E2-E5 were obtained throughout the downstream process including for example, centrifugation, filtration, and formaldehyde inactivation. Sample E6 was collected from the final monovalent drug substance.

**Table 5. Analytical methods for sample analysis.**

| **Test** | **Sample(s)** | **Method** |
|---|---|---|
| HA content | E0, E1, E2, E3, E4, E5, E6 | SRID assay |
| Protein content | E0, E1, E2, E3, E4, E5, E6 | Chemiluminescence assay |
| Infectious titer | E0, E4 | Titration on eggs |
| Inactivation control | E6 | Titration on eggs |
| Protein profile | E0, E1, E2, E3, E4, E5, E6 | SDS-PAGE |
| Particle size | E6 | DLS |
| Morphological characteristics | E0, E1, E2, E6 | TEM |
| NA enzymatic activity | E6 | MUNANA assay |
| Endotoxin content | E6 | LAL assay |
| Sterility | E6 | Membrane filtration |

The SRID titer and protein content were determined using the same methods described in Example 1 and the data showed that the SRID titer in Samples E0 to E6 evolved until reaching a final value of 61% at the drug substance stage. Similarly, the protein content in Samples E0 to E6 evolved until reaching a final value of 28% at the drug substance stage, which was the same order of magnitude observed in the reference batch using Triton X-100 as the splitting agent.

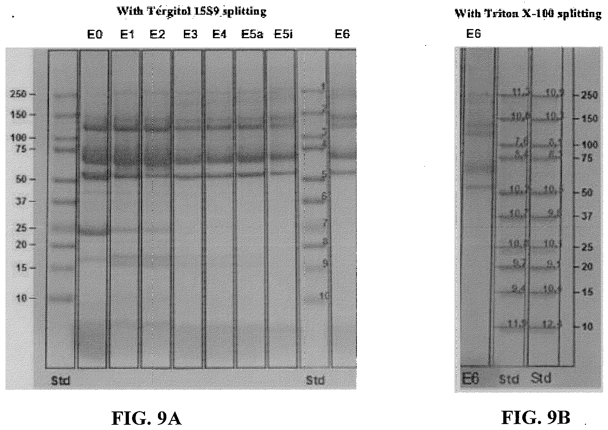

The protein profile analyzed by SDS-PAGE under denatured/non-reducing conditions is shown in **FIG. 9A****,** which demonstrates the evolution of the purity rate during the purification process, with the visible presence of the expected major proteins (i.e., monomer HA (about 70 kDa), dimer HA (about 140 kDa), trimer HA (about 210 kDa), and NA (about 55 kDa)) at the drug substance stage (Sample E6). The protein profile of the drug substance (Sample E6) obtained from the reference batch is shown in **FIG. 9B****.**

The infectious titers of Sample E0 and Sample E4, as measured on eggs, are provided in **Table 6.** Also provided in **Table 6** are the infectious titers of the corresponding samples obtained from the reference batch using Triton X-100 as the splitting agent. As can be seen from **Table 6,** both Tergitol 15S9 and Triton X-100 led to a comparable logarithmic decrease in the infectious titer between the steps from which Samples E0 and E4 were obtained, thus confirming the splitting efficiency of Tergitol 15S9 compared to Triton X-100.

**Table 6. Infectious titers on eggs.**

| | **Tergitol 15S9** | **Triton X-100** |
|---|---|---|
| **Sample E0** | 11.00 log₁₀CCID₅₀/mL | 11.05 log₁₀CCID₅₀/mL |
| **Sample E4** | 4.60 log₁₀CCID₅₀/mL | 4.09 log₁₀CCID₅₀/mL |

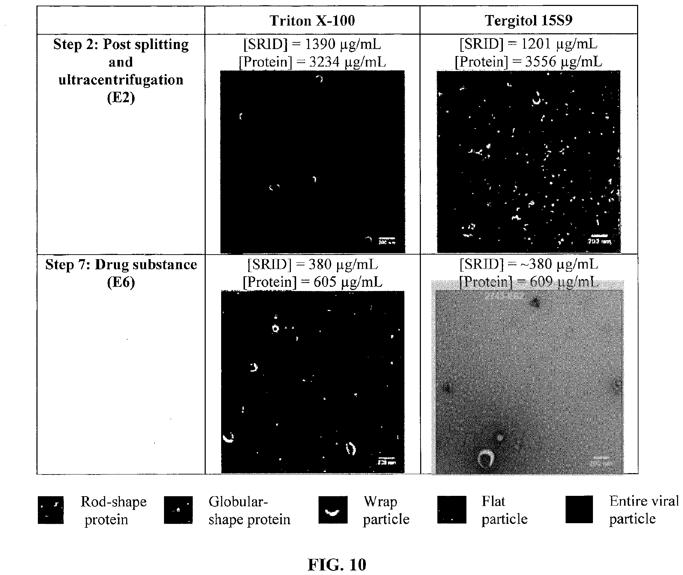

The morphological characteristics of the particles in Sample E2 and Sample E6 analyzed by TEM are shown in **FIG. 10****.** Also provided in **FIG. 10** are the morphological characteristics of the particles in the corresponding samples obtained from the reference batch using Triton X-100 as the splitting agent. As shown in **FIG. 10****,** the production process using Tergitol 15S9 as the splitting agent produced a similar particle profile as the Triton X-100 control, materialized by an absence of intact influenza viral particles and many small structures representing proteins and wrap/flat particles.

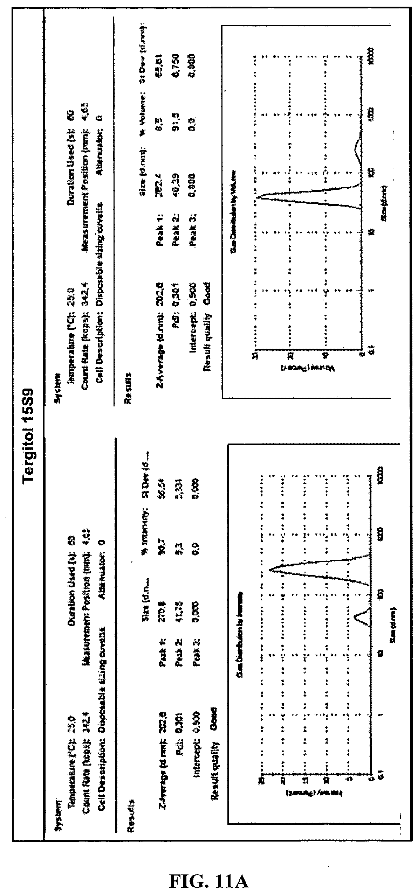

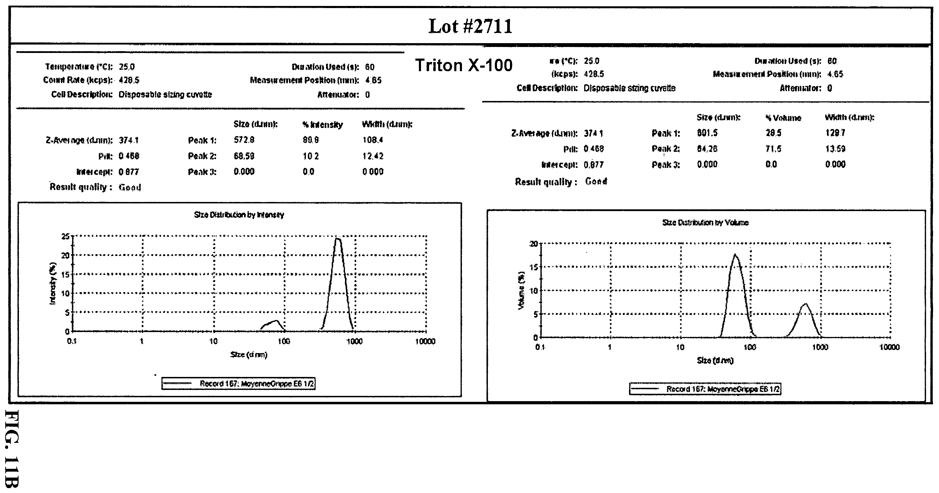

The particle size distribution in the samples collected at the drug substance stage (i.e., Sample E6) was determined using DLS and the profiles (intensity and volume) are shown in **FIG. 11A-11B****.** As shown in **FIG. 11A****,** the drug substance obtained using Tergitol 15S9 as the splitting agent has a comparable profile as the drug substance obtained using Triton X-100 as the splitting agent **(****FIG. 11B****)** with the presence of 2 distinct populations, < 100 nm and > 200 nm respectively.

For quality control, the samples collected at the drug substance stage (i.e., Sample E6) were also tested for inactivation control, NA enzymatic activity, endotoxin content, and sterility. The inactivation control was tested using titration on eggs. The NA enzymatic activity was tested using the MUNANA assay, which is an assay based on the NA enzyme cleaving the 2'-(4-Methylumbelliferyl)-α-D-N-acetylneuraminic acid (MUNANA) substrate to release the fluorescent product 4-methylumbelliferone (4-MU). The endotoxin content was determined using the limulus amoebocyte lysate (LAL) assay, which is an assay for the detection of viable and non-viable Gram-negative bacteria. The sterility was tested using membrane filtration. **Table 7** below summarizes the final characteristics obtained from the samples collected at the drug substance stage (i.e., Sample E6). Overall, this study shows that Tergitol 15S9 produces results comparable to Triton X-100 at the splitting step and has little impact on subsequent downstream process steps in terms of yield, purity and structure.

**Table 7. Characteristics of the drug substance.**

| **Criterion** | | **Tergitol 15S9** | **Triton X-100** |
|---|---|---|---|
| SRID titer | | 351 µg/mL | 375 µg/mL |
| Protein concentration | | 609 µg/mL | 605 g/mL |
| SRID/protein ratio | | ∼ 58% | ∼ 62% |
| SDS-PAGE | | HA + NA majority | HA + NA majority |
| Inactivation control | | OK | OK |
| Sterility test | | OK | OK |
| Endotoxin content | | < 0.5 EU/mL | < 0.5 EU/mL |

| TEM | | | |
|---|---|---|---|
| | % whole viruses/particles | 10% | 0% |
| | wrap/flat ratio | 3.8 | 2.6 |

| NA enzymatic activity | | | |
|---|---|---|---|
| | NA activity | 228 µmol/mL/h | 155 µmol/mL/h |
| | NA concentration by MS* | 0.119 mg/mL | ND** |
| | Specific activity | 1915 µmol/mg/h | ND** |

| | | | |
|---|---|---|---|
| *: Mass spectrometry. **: Not determined. | | | |

### Example 4. Immunogenicity of Recombinant Monovalent Influenza Vaccines

This Example describes the evaluation of monovalent influenza vaccine containing recombinant hemagglutinin (HA) protein of the A/Michigan/45/2015 or B/Phuket/3073/2013 virus strain for the ability to induce an immunogenic response in mice. The recombinant HA protein was expressed in the insect cell line expresSF+^{®} using a baculovirus vector, extracted from the cells with Triton X-100 or Tergitol 15S9, further purified by column chromatography, and then formulated into vaccine for intramuscular injection. Groups of mice were immunized with 0.01 µg, 0.04 µg, 0.64 µg, or 10.0 µg of recombinant HA protein per animal or vehicle alone (PBS). Each group received an identical booster dose 21 days after the initial immunization. Post-immune serum was collected from each animal on days 21 and 35. Serum pools used for analysis were prepared by mixing equal volumes of serum from each animal within a group. - Immunogenicity assessment was performed by hemagglutination inhibition (HAI) assay.

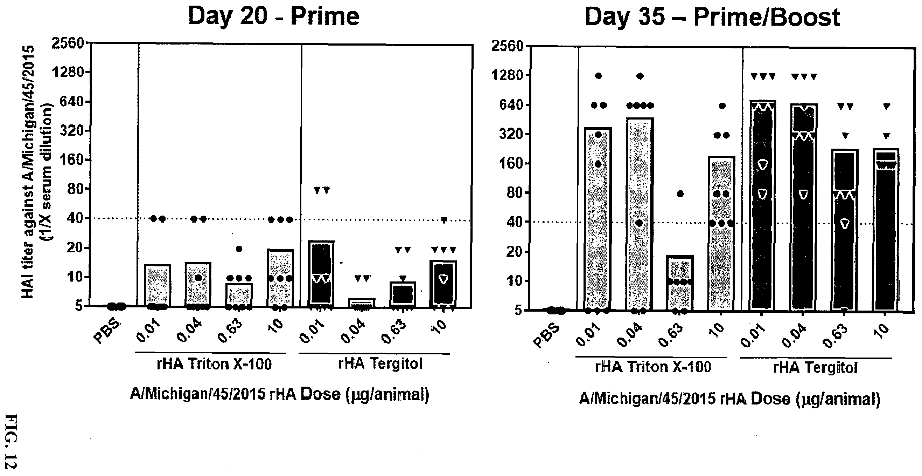

**FIG. 12** sets forth representative serum HAI titer induced against the A/Michigan/45/2015 virus strain at day 20 (prime) and day 35 (prime/boost) (2 ANOVA models for repeated measures (log-10 HAI titers as dependent variable), followed by p value adjustment by Dunnett's multiple comparison test). As shown in **FIG. 12****,** the recombinant HA protein purified using Tergitol 15S9 performs similarly to the recombinant HA protein purified using Triton X-100. There are no statistically significant differences at all doses compared. Moreover, fold-difference in HAI geometric mean titers (GMT) between the groups does not exceed 1.5-fold at 0.04 µg dose.

### Example 5. Immunogenicity of Monovalent Influenza Split Vaccines

This Example describes the evaluation of monovalent influenza split vaccine containing hemagglutinin (HA) protein obtained from the A/Michigan/45/2015 virus strain for the ability to induce an immunogenic response in mice. The HA protein was obtained using Tergitol 15S9 or Triton X-100 as the splitting agent, purified according to a downstream process similar to that described in Example 3, and formulated into vaccine for intramuscular injection. Groups of mice were immunized with 0.002 µg, 0.02 µg, 0.16 µg, 1.25 µg, or 10.0 µg of HA protein per animal or vehicle alone (PBS). Each group received an identical booster dose 21 days after the initial immunization. Post-immune serum was collected from each animal on days 20 and 35. Serum pools used for analysis were prepared by mixing equal volumes of serum from each animal within a group. Immunogenicity assessment was performed by hemagglutination inhibition (HAI) assay.

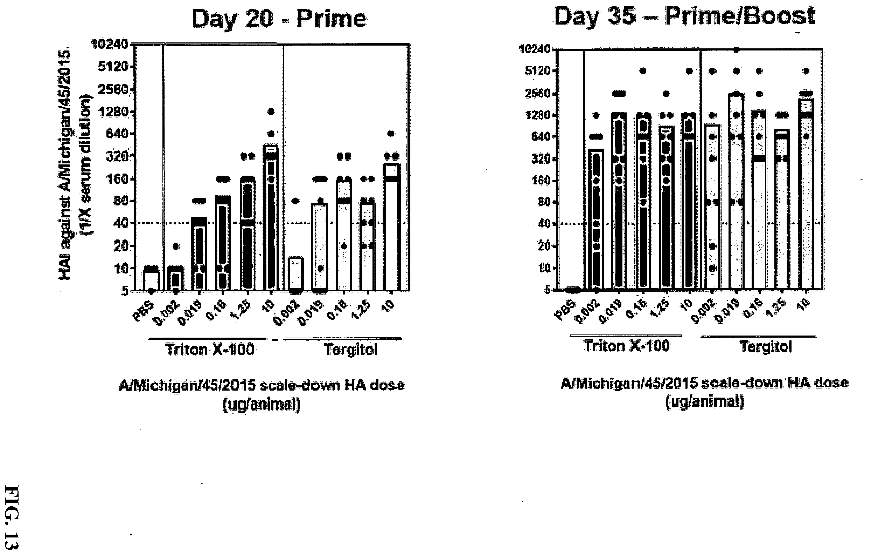

**FIG. 13** sets forth representative serum HAI titer induced against the A/Michigan/45/2015 virus strain at day 20 (prime) and day 35 (prime/boost) (2 ANOVA models for repeated measures (log-10 HAI titers as dependent variable), followed by *p* value adjustment by Dunnett's multiple comparison test). As shown in **FIG. 13****,** the HA protein obtained using Tergitol 15S9 as the splitting agent performs similarly to the HA protein obtained using Triton X-100 as the splitting agent. There are no statistically significant differences at all doses compared.

### Example 6. Evaluation of Deviron 13S9 for Production of Drug Substance

This Example describes the evaluation of using Deviron 13S9 for producing influenza monovalent drug substance. Deviron 13S9 was evaluated in a splitting procedure involving the B/Phuket/3073/2013 virus strain grown in eggs. Specifically, Deviron 13S9 was added to viral samples and incubated under magnetic stirring. Various samples were taken before, during, and after the splitting procedure and were analyzed for their respective HA content, protein profile, morphological characteristics, and residual infectious virus. As shown in the table below, Deviron 13S9 was as effective as Tergitol 15S9 and Triton X-100 as a splitting agent.

**Table 8. Evaluation of Deviron 13S9 for production of drug substance.**

| | Deviron 13S9 Batch #1 | Deviron 13S9 Batch #2 | Tergitol 15S9 | **Triton X-100** |
|---|---|---|---|---|
| Residual infectious virus | No Residual infectious virus | | | |
| HA content in µg/mL (SRID) | 263.46 | 232.3 | 241.24 | 269.97 |
| Morphological characterization (TEM) | Splitting observed | | | |
| Protein profile (SDS-PAGE) | Comparable protein profile | | | |
| Splitting efficiency (ultracentrifugation in sucrose gradient) | Conform* | | TBD | Conform* |

| | | | | |
|---|---|---|---|---|
| *: Max total protein by OD₂₈₀ at 40%sucrose for whole virus and lower than 30%sucrose for split virus. | | | | |

### Example 7. Evaluation of Tergitol 15S9 and Deviron 13S9 for Production of Drug Substance from MDCK Cells

This Example describes the process for producing influenza monovalent drug substance from MDCK cells using Tergitol 15S9 or Deviron 13S9 as the splitting agent.

Tergitol 15S9 and Deviron 13S9 are evaluated in a splitting procedure involving the A/H1N1/Michigan/45/15 X-275 (H1N1) virus strain grown in MDCK cells. Specifically, Tergitol 15S9 or Deviron 13S9 is added to viral samples derived from MDCK cells and incubated under magnetic stirring. Various samples were taken before, during, and after the splitting procedure and are analyzed for their respective HA content, protein content, protein profile, particle size, morphological characteristics, and infectious titer. It is expected that both Tergitol 15S9 and Deviron 13S9 can effectively split viral samples derived from MDCK cells as well as provide stabilizing effects on the resulting immunogenic or vaccine composition.

While the foregoing disclose has been described in some detail by way of illustration and example for purposes of clarity and understanding, it will be clear to one of ordinary skill in the art from a reading of this disclosure that various changes in form and detail can be made without departing from the true scope of the present disclosure and may be practiced within the scope of the appended claims. For example, all constructs, methods, and/or component features, steps, elements, or other aspects thereof can be used in various combinations.

Claims or descriptions that include "or" between one or more members of a group are considered satisfied if one, more than one, or all of the group members are present in, employed in, or otherwise relevant to a given product or process unless indicated to the contrary or otherwise evident from the context. The disclosure includes embodiments in which exactly one member of the group is present in, employed in, or otherwise relevant to a given product or process. The disclosure also includes embodiments in which more than one, or the entire group members are present in, employed in, or otherwise relevant to a given product or process. Furthermore, it is to be understood that the disclosure encompasses all variations, combinations, and permutations in which one or more limitations, elements, clauses, descriptive terms, etc., from one or more of the listed claims is introduced into another claim dependent on the same base claim (or, as relevant, any other claim) unless otherwise indicated or unless it would be evident to one of ordinary skill in the art that a contradiction or inconsistency would arise. Where elements are presented as lists, (e.g., in Markush group or similar format) it is to be understood that each subgroup of the elements is also disclosed, and any element(s) can be removed from the group. In general, where embodiments or aspects of the present disclosure, is/are referred to as comprising particular elements, features, etc., certain embodiments or aspects consist, or consist essentially of, such elements, features, etc. For purposes of simplicity those embodiments have not in every case been specifically set forth in so many words herein. It should also be understood that any embodiment or aspect of the present disclosure can be explicitly excluded from the claims, regardless of whether the specific exclusion is recited in the specification.

All patents, patent applications, websites, other publications or documents, accession numbers and the like cited herein are incorporated by reference in their entirety for all purposes to the same extent as if each individual item were specifically and individually indicated to be so incorporated by reference.

## Claims

1. A method for preparing at least one viral protein in a manufacturing process of a vaccine, the method comprising subjecting a suspension of virions or a suspension of host cells to a detergent comprising a compound having a CAS registry number of CAS 68131-40-8 or CAS 84133-50-6, wherein each virion comprises a lipid membrane that comprises the at least one viral protein, and wherein the host cells have been modified to express the at least one viral protein.

2. The method of claim 1, wherein
the detergent does not comprise a compound having a CAS registry number of CAS 9002-93-1 or CAS 9036-19-5; and/or
the detergent consists of the compound having the CAS registry number of CAS 68131-40-8 or CAS 84133-50-6; and/or
the detergent is added to the suspension of virions or the suspension of host cells in an amount of from 0.01% to 10% by volume, such as from 0.1% to 5% by volume or from 0.5% to 1% by volume, or in an amount of 0.5% by volume; and/or
subjecting the suspension of virions or the suspension of host cells to the detergent releases the at least one viral protein from the virions or host cells, and wherein the method further comprises obtaining a first suspension comprising the at least one viral protein released from the virions or host cells, optionally wherein the method further comprises removing the detergent from the first suspension to obtain a second suspension comprising the at least one viral protein and a trace amount of the detergent, and optionally wherein removing the detergent comprises diafiltration.

3. The method of claim 2, further comprising purifying the at least one viral protein from the second suspension.

4. The method of any one of claims 1-3, further comprising formulating the at least one viral protein into a vaccine, wherein the vaccine contains no more than 250 µg/0.5 mL of the compound having the CAS registry number of CAS 68131-40-8 or CAS 84133-50-6, optionally wherein formulating comprises combining the at least one viral protein with an adjuvant to produce the vaccine; and/or
wherein the method further comprises filling the vaccine into a syringe or a vial; and/or
wherein the vaccine is an influenza vaccine, optionally
wherein the suspension of virions is obtained by cultivating an influenza virus in an embryonated chicken egg or the suspension of virions is obtained by cultivating an influenza virus in a viral propagation cell line, optionally in a Madin-Darby Canine Kidney (MDCK) cell line, or wherein the suspension of host cells has been modified to recombinantly express an influenza hemagglutinin or neuraminidase polypeptide, optionally the suspension of host cells comprises SF9 cells of *Spodoptera frugiperda* or Chinese hamster ovary (CHO) cells, optionally the at least one viral protein is expressed in the SF9 cells using a baculovirus vector.

5. The method of claim 4, wherein the influenza vaccine is a monovalent influenza vaccine or the influenza vaccine is a multivalent influenza vaccine, such as a trivalent, quadrivalent, pentavalent, or octavalent influenza vaccine.

6. The method of claim 5, wherein
the multivalent influenza vaccine comprises at least one viral protein obtained from an influenza A virus and at least one viral protein obtained from an influenza B virus; and/or
the influenza vaccine is a trivalent influenza vaccine comprising a first viral protein obtained from an H1N1 influenza virus strain, a second viral protein obtained from an H3N2 influenza virus strain, and a third viral protein obtained from an influenza virus of a B/Victoria lineage, optionally wherein the method comprises combining and formulating the first, second, and third viral proteins into the trivalent influenza vaccine; or the first, second, and third viral proteins comprise influenza hemagglutinin proteins; and/or each of the first, second, and third viral proteins is an influenza hemagglutinin protein present in an amount between 5 µg to 60 µg per 0.5 mL dose of the vaccine; or
the influenza vaccine is a quadrivalent influenza vaccine comprising a first viral protein obtained from an H1N1 influenza virus strain, a second viral protein obtained from an H3N2 influenza virus strain, a third viral protein obtained from an influenza virus of a B/Victoria lineage, and a fourth viral protein obtained from an influenza virus of a B/Yamagata lineage, optionally wherein
the method comprises combining and formulating the first, second, third, and fourth viral proteins into the quadrivalent influenza vaccine; and/or
the first, second, third, and fourth viral proteins comprise influenza hemagglutinin proteins; and/or the amount of influenza hemagglutinin protein in each of the first, second, third, and fourth viral proteins is between 5 µg to 60 µg per 0.5 mL dose of the vaccine.

7. The method of any one of claims 4-6, wherein
the vaccine further comprises sodium phosphate buffered isotonic sodium chloride and optionally a preservative, wherein, optionally, the preservative is mercury or a mercury derivative, wherein, optionally, the mercury derivative is thimerosal; and/or
the vaccine further comprises formaldehyde at a concentration of no more than 100 µg/0.5 mL.

8. A vaccine manufactured by the method of any one of claims 4-7.

9. An immunogenic composition comprising an inactivated virus or at least one viral protein and a trace amount of a detergent comprising a compound having a CAS registry number of CAS 68131-40-8 or CAS 84133-50-6, optionally wherein
the detergent does not comprise a compound having a CAS registry number of CAS 9002-93-1 or CAS 9036-19-5; and/or
the detergent consists of the compound having the CAS registry number of CAS 68131-40-8 or CAS 84133-50-6; and/or
the inactivated virus is an influenza A virus, optionally an H1N1 influenza virus strain or wherein the influenza A virus is an H3N2 influenza virus strain; and/or
the inactivated virus is an influenza B virus, optionally a B/Victoria lineage or a B/Yamagata lineage; and/or
the at least one viral protein is an influenza hemagglutinin protein and/or an influenza neuraminidase protein; and/or
the at least one viral protein is a recombinant influenza structural protein, optionally wherein the recombinant influenza structural protein is expressed in SF9 cells of *Spodoptera frugiperda* or CHO cells, and optionally wherein the recombinant influenza structural protein is expressed in the SF9 cells using a baculovirus vector, optionally wherein the recombinant influenza structural protein is a recombinant influenza hemagglutinin protein and/or a recombinant influenza neuraminidase protein; and/or
the immunogenic composition comprises at least one viral protein from an influenza A virus and at least one viral protein from an influenza B virus, optionally comprises a first viral protein from an H1N1 influenza virus strain, a second viral protein from an H3N2 influenza virus strain, and a third viral protein from an influenza virus of a B/Victoria lineage, optionally wherein the first, second, and third viral proteins are influenza hemagglutinin proteins and/or influenza neuraminidase proteins, optionally wherein each of the first, second, and third viral proteins is an influenza hemagglutinin protein present in an amount of between 5 µg to 60 µg per 0.5 mL dose of the immunogenic composition; or
the immunogenic composition comprises a first viral protein from an H1N1 influenza virus strain, a second viral protein from an H3N2 influenza virus strain, a third viral protein from an influenza virus of a B/Victoria lineage, and a fourth viral protein from an influenza virus of a B/Yamagata lineage, optionally wherein the first, second, third, and fourth viral proteins are influenza hemagglutinin proteins and/or influenza neuraminidase proteins, optionally wherein each of the first, second, third, and fourth viral proteins is an influenza hemagglutinin protein present in an amount of between 5 µg to 60 µg per 0.5 mL dose of the immunogenic composition; and/or
the immunogenic composition further comprises an adjuvant.

10. A vaccine comprising the immunogenic composition of claim 9 and a pharmaceutically acceptable carrier, optionally wherein
the vaccine comprises at least one influenza virus hemagglutinin protein; and/or
the vaccine comprises no more than 250 µg/0.5 mL of the compound having the CAS registry number of CAS 68131-40-8 or CAS 84133-50-6; and/or
the vaccine further comprises sodium phosphate buffered isotonic sodium chloride and optionally a preservative, wherein, optionally, the preservative is mercury or a mercury derivative, wherein, optionally, the mercury derivative is thimerosal; and/or
the vaccine further comprises formaldehyde at a concentration of no more than 100 µg/0.5 mL; and/or
the vaccine is a live attenuated virus vaccine, an inactivated whole virus vaccine, a virosome vaccine, a split-virion vaccine, a subunit vaccine, or a recombinant vaccine.

11. A vaccine comprising:
5-60 µg/0.5 mL of an influenza hemagglutinin antigen from each of an H1N1 influenza virus strain, an H3N2 influenza virus strain, and an influenza virus of a B/Victoria lineage;
a sodium phosphate-buffered isotonic sodium chloride solution;
no more than 30-100 µg/0.5 mL of formaldehyde;
no more than 250 µg/0.5 mL of a compound having a CAS registry number of CAS 68131-40-8 or CAS 84133-50-6; and
optionally a preservative,
wherein, optionally,
the vaccine does not comprise a compound having a CAS registry number of CAS 9002-93-1 or CAS 9036-19-5; and/or
the preservative comprises thimerosal in an amount of 2 µg/0.5 mL or mercury in an amount of 20 µg/0.5 mL; and/or
the vaccine comprises 15 µg/0.5 mL of the influenza hemagglutinin antigen from each of an H1N1 influenza virus strain, an H3N2 influenza virus strain, and an influenza virus of a B/Victoria lineage, and no more than 30 µg/0.5 mL of formaldehyde, optionally further comprises thimerosal as the preservative in an amount of 2 µg/0.5 mL; or
the vaccine comprises 15 µg/0.5 mL of an influenza hemagglutinin antigen from each of an H1N1 influenza virus strain, an H3N2 influenza virus strain, and an influenza virus of a B/Victoria lineage, and no more than 100 µg/0.5 mL of formaldehyde, and wherein the preservative comprises mercury in an amount of 25 µg/0.5 mL;
or
a vaccine comprising:
5-60 µg/0.5 mL of an influenza hemagglutinin antigen from each of an H1N1 influenza virus strain, an H3N2 influenza virus strain, an influenza virus of a B/Victoria lineage, and an influenza virus of a B/Yamagata lineage;
a sodium phosphate-buffered isotonic sodium chloride solution;
no more than 100 µg/0.5 mL of formaldehyde;
no more than 250 µg/0.5 mL of a compound having a CAS registry number of CAS 68131-40-8 or CAS 84133-50-6; and
optionally a preservative,
wherein, optionally,
the vaccine does not comprise a compound having a CAS registry number of CAS 9002-93-1 or CAS 9036-19-5; and/or
the vaccine comprises no more than 30 µg/0.5 mL of formaldehyde; and/or
the preservative is mercury in an amount of 25 µg/0.5 mL;
or
a vaccine comprising:
5-60 µg/0.5 mL of an influenza hemagglutinin antigen from each of an H1N1 influenza virus strain, an H3N2 influenza virus strain, an influenza virus of a B/Victoria lineage, and an influenza virus of a B/Yamagata lineage;
a buffer solution comprising sodium chloride, potassium chloride, dibasic sodium phosphate dihydrate, and monobasic potassium phosphate;
no more than 30 µg/0.5 mL of formaldehyde; and
no more than 250 µg/0.5 mL of a compound having a CAS registry number of CAS 68131-40-8 or CAS 84133-50-6,
wherein, optionally,
the vaccine does not comprise a compound having a CAS registry number of CAS 9002-93-1 or CAS 9036-19-5; and/or
the vaccine does not comprise an adjuvant or a preservative;
or
a vaccine comprising:
45 µg/0.5 mL of a recombinant influenza hemagglutinin antigen from each of an H1N1 influenza virus strain, an H3N2 influenza virus strain, and an influenza virus of a B/Victoria lineage;
sodium chloride;
monobasic sodium phosphate;
dibasic sodium phosphate;
polysorbate 20; and
no more than 100 µg/0.5 mL of a compound having a CAS registry number of CAS 68131-40-8 or CAS 84133-50-6,
wherein, optionally,
the vaccine does not comprise a compound having a CAS registry number of CAS 9002-93-1 or CAS 9036-19-5; and/or
the vaccine comprises:
4.4 mg/0.5 mL sodium chloride;
0.195 mg/0.5 mL monobasic sodium phosphate; .
1.3 mg/0.5 mL dibasic sodium phosphate; and
27.5 µg/0.5 mL polysorbate 20; or
the vaccine comprises:
4.4 mg/0.5 mL sodium chloride;
0.2 mg/0.5 mL monobasic sodium phosphate;
0.5 mg/0.5 mL dibasic sodium phosphate; and
27.5 µg/0.5 mL polysorbate 20; and/or
the vaccine does not comprise a preservative;
or
a vaccine comprising:
45 µg/0.5 mL of a recombinant influenza hemagglutinin antigen from each of an H1N1 influenza virus strain, an H3N2 influenza virus strain, an influenza virus of a B/Victoria lineage, and an influenza virus of a B/Yamagata lineage;
sodium chloride;
monobasic sodium phosphate;
dibasic sodium phosphate;
polysorbate 20; and
no more than 100 µg/0.5 mL of a compound having a CAS registry number of CAS 68131 - 40-8 or CAS 84133-50-6,
wherein, optionally,
the vaccine does not comprise a compound having a CAS registry number of CAS 9002-93-1 or CAS 9036-19-5; and/or
the vaccine comprises:
4.4 mg/0.5 mL sodium chloride;
0.195 mg/0.5 mL monobasic sodium phosphate;
1.3 mg/0.5 mL dibasic sodium phosphate; and
27.5 µg/0.5 mL polysorbate 20, or
the vaccine comprises:
4.4 mg/0.5 mL sodium chloride;
0.2 mg/0.5 mL monobasic sodium phosphate;
0.5 mg/0.5 mL dibasic sodium phosphate; and
27.5 µg/0.5 mL polysorbate 20; and/or
the vaccine does not comprise a preservative.

12. The vaccine of any one of claims 10-11, wherein
the serum hemagglutinin inhibition (HAI) titer induced by the vaccine is the same or higher than the serum HAI titer induced by a control vaccine that is identical to the vaccine of any one of claims 10-11 except that the trace amount of the detergent comprising the compound having the CAS registry number of CAS 68131-40-8 or CAS 84133-50-6 is replaced with a trace amount of a detergent comprising a compound having a CAS registry number of CAS 9002-93-1 or CAS 9036-19-5;
or
the vaccine induces a serum hemagglutinin inhibition (HAI) titer of at least 40, wherein the HAI titer is measured at 20 days following vaccination; and/or the vaccine induces a serum HAI that increases at least 4-fold as compared to the serum HAI titer prior to vaccination, wherein the HAI titer is measured at 20 days following vaccination.

13. The vaccine of claim 8, the immunogenic composition of claim 9, or the vaccine of any one of claims 10-12 for use in a method of immunizing a subject,
wherein, optionally,
the immunization prevents an influenza virus infection in the subject, decreases the subject's likelihood of getting an influenza virus infection, or reduces the subject's likelihood of getting serious illness from an influenza virus infection; and/or
the immunization raises a protective immune response in the subject; and/or
the subject is a human, optionally the human is 6 months of age or older, less than 18 years of age, at least 6 months of age and less than 18 years of age, at least 18 years of age and less than 65 years of age, at least 6 months of age and less than 5 years of age, at least 5 years of age and less than 65 years of age, at least 60 years of age, or at least 65 years of age; and/or
the vaccine, the immunogenic composition or the vaccine is administered intramuscularly, .intradermally, subcutaneously, intravenously, intranasally, by inhalation, or intraperitoneally.

14. The vaccine of claim 8, the immunogenic composition of claim 9, or the vaccine of any one of claims 10-12 for use in a method of reducing one or more symptoms of an influenza virus infection or caused by an influenza virus infection.

15. The vaccine or the immunogenic composition for use according to any one of claims 13-14, wherein the influenza virus infection is caused by either or both a seasonal and a pandemic influenza virus strain.
